(19) Europäisches Patentamt
European Patent Office
Office européen des brevets

(11) **EP 4 209 227 A1**

(12) **EUROPEAN PATENT APPLICATION**
published in accordance with Art. 153(4) EPC

(43) Date of publication:
**12.07.2023 Bulletin 2023/28**

(51) International Patent Classification (IPC):
*A61K 39/395* (2006.01)    *A61K 45/00* (2006.01)
*A61P 25/20* (2006.01)    *A61P 17/04* (2006.01)

(21) Application number: **20952354.7**

(86) International application number:
**PCT/JP2020/032987**

(22) Date of filing: **01.09.2020**

(87) International publication number:
**WO 2022/049614 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **CHUGAI SEIYAKU KABUSHIKI KAISHA Tokyo 115-8543 (JP)**

(72) Inventors:
• **FUKAZAWA, Naoki**
 **Tokyo 103-8324 (JP)**
• **OKADA, Fumie**
 **Tokyo 103-8324 (JP)**
• **SAITO, Tomohisa**
 **Tokyo 103-8324 (JP)**
• **HIRAHARA, Tetsuya**
 **Tokyo 103-8324 (JP)**
• **HIROKAWA, Keiko**
 **Tokyo 103-8324 (JP)**
• **MIHARA, Ryosuke**
 **Tokyo 103-8324 (JP)**

(74) Representative: **Vossius & Partner Patentanwälte Rechtsanwälte mbB Siebertstraße 3 81675 München (DE)**

Remarks:
The complete document including Reference Table(s) and the Sequence Listing(s) can be downloaded from the EPO website

(54) **PHARMACEUTICAL COMPOSITION FOR PREVENTION AND/OR TREATMENT OF DIALYSIS PRURITUS CONTAINING IL-31 ANTAGONIST AS ACTIVE INGREDIENT**

(57) In a non-limiting embodiment, there is provided a pharmaceutical composition for prevention and/or treatment of uremic pruritus comprising an IL-31 antagonist as an active ingredient.

FIG. 13

EP 4 209 227 A1

**Description**

[Technical Field]

In one non-limiting aspect, the present disclosure relates to, for example, a pharmaceutical composition for prevention and/or treatment of uremic pruritus comprising an IL-31 antagonist as an active ingredient.

[Background Art]

Pruritus in dialysis patients (also called "uremic pruritus" herein), characterized by refractory and systemic itching, is one of the symptoms daily afflicting dialysis patients. It is generally found more often in patients receiving long-term dialysis (NPL 1), but it varies in terms of the area it occurs, frequency, duration, and how badly it affects the lives of patients. Apparent symptoms of the skin are not usually observed even at the area pruritus is occurring (NPL 2). According to the study carried out in Japan in the year 2000, 72.8% of hemodialysis patients experienced pruritus and about a half of them had sleep disturbance (NPL 3). The severity of insomnia has been reported to be higher in patients with severe pruritus (NPL 4). From the study of "Dialysis Outcomes and Practice Patterns Study (DOPPS)" carried out in 12 countries including Japan, it has been reported that about 40% or more patients had pruritus of a moderate or above grading and had worsened sleep quality (NPL 5). It has also been reported that severe pruritus is a risk factor for vital prognosis of dialysis patients (NPL 6). As above, improving pruritus in dialysis patients is an important task for the improvement of the quality of life (QOL), quality of sleep, and furthermore of the vital prognosis of dialysis patients.

[Citation List]

[Non-Patent Literature]

[NPL 1] Nakai S et al., Current situation on chronic dialysis therapy in Japan (as of December 31, 1999); Journal of the Japanese Society for Dialysis Therapy; 2001, 34:1-31
[NPL 2] Danno K., Friends of itchiness, The perfect guide for treating itchiness you want to leave in the dialysis room; Kinpodo; 2008, p.1-16
[NPL 3] Omori K et al., Situation on dialysis dermal pruritus - Search report on 2474 patients at 41 institutions in the Niigata prefecture -; Journal of the Japanese Society for Dialysis Therapy; 2001; 34:1469-77.
[NPL 4] Narita I et al. Etiology and prognostic significance of severe uremic pruritus in chronic hemodialysis patients. Kidney Int. 2006;69:1626-32
[NPL 5] Pisoni RL et al. Pruritus in haemodialysis patients: International results from the Dialysis Outcomes and Practice Patterns Study (DOPPS). Nephrol Dial Transplant. 2006;21:3495-505
[NPL 6] Kimata N et al. Pruritus in hemodialysis patients: Results from the Japanese Dialysis Outcomes and Practice Patterns Study (JDOPPS). Hemodial Int 2014;18:657-67

[Summary of Invention]

[Technical Problem]

The mechanism of uremic pruritus is still not exactly known, but possible involvement of IL-31 has been suggested (Gangemi S, Quartuccio S, Casciaro M, Trapani G, Minciullo PL, Imbalzano E. Interleukin 31 and skin diseases: A systematic review. Allergy Asthma Proc. 2017;38(6):401-8). Regarding IL-31 and pruritus in dialysis patients, it has been reported that maintenance hemodialysis patients with pruritus had higher serum IL-31 concentration than those without pruritus (Ko MJ et al. Interleukin-31 is associated with uremic pruritus in patients receiving hemodialysis. J Am Acad Dermatol. 2014;71:1151-9).

IL-31 (interleukin-31) is a T-cell cytokine. It is known that dermatitis-like symptoms similar to pruritus or atopic dermatitis occur in transgenic mice overexpressing IL-31 (Nat Immunol (2004) 5, 752-760). It has also been found that the receptor to which IL-31 binds is a heterodimer of IL-31RA (interleukin-31 receptor A) and OSMR (oncostatin M receptor) (WO2004/003140), and IL-31 transduces signals into cells through this receptor.

To date, IL-31 neutralizing antibodies and IL-31RA neutralizing antibodies as IL-31 antagonists have been reported (for example, WO2005/079566; WO2006/063864; WO2006/063865; WO2009/071696; WO2006/088855; WO2006/088955; WO2006/088956; WO2007/133816; WO2007/142325; WO2009/072598; WO2006/122079; WO2007/143231; WO2008/028192; WO2009/072604; WO2010/064697).

However, there has been no report of experimental results showing that an IL-31 antagonist is effective in prevention and/or treatment of uremic pruritus.

[Solution to Problem]

In a non-limiting embodiment, the present disclosure relates to the following:

[1] A pharmaceutical composition for prevention and/or treatment of uremic pruritus comprising an IL-31 antagonist as an active ingredient.

[2] The pharmaceutical composition of [1], wherein the IL-31 antagonist is administered to a subject with or potentially with uremic pruritus at 0.1 mg to 1000 mg/body/2 weeks, 0.1 mg to 1000 mg/body/4 weeks, or 0.1 mg to 1000 mg/body/8 weeks, repeatedly at the same dose and the same dosing interval.

[3] The pharmaceutical composition of [2], wherein the IL-31 antagonist is administered at 25 mg to 100 mg/body/4 weeks.

[4] The pharmaceutical composition of [2] or [3], wherein the IL-31 antagonist is administered at 50 to 100 mg/body/4 weeks.

[5] The pharmaceutical composition of any one of [1] to [4], wherein the IL-31 antagonist is administered to a subject with or potentially with uremic pruritus at 0.01 mg to 10 mg/kg/2 weeks, 0.01 mg to 10 mg/kg/4 weeks, or 0.01 mg to 10 mg/kg/8 weeks, repeatedly at the same dose and the same dosing interval.

[6] The pharmaceutical composition of [5], wherein the IL-31 antagonist is administered at 0.2 mg to 2 mg/kg/4 weeks.

[7] The pharmaceutical composition of any one of [1] to [6], wherein the IL-31 antagonist is administered to a subject with or potentially with uremic pruritus having a serum IL-31 concentration equal to or higher than a predetermined value.

[7-2] The pharmaceutical composition of any one of [1] to [7], which is for administration only to a subject that has been determined to be a responder to prevention and/or treatment with an IL-31 antagonist by a method for predicting the response of a subject to prevention and/or treatment with an IL-31 antagonist, the method comprising:

measuring IL-31 concentration in a serum obtained from a subject with or potentially with uremic pruritus; and determining the subject as a responder to prevention and/or treatment with an IL-31 antagonist if the IL-31 concentration is equal to or higher than a predetermined value.

[7-3] The pharmaceutical composition of any one of [1] to [7], which is for administration only to a subject that has been determined to be a responder to prevention and/or treatment with an IL-31 antagonist by a method for predicting the response of a subject to prevention and/or treatment with an IL-31 antagonist, the method comprising: determining a subject with or potentially with uremic pruritus whose serum IL-31 concentration has been measured as a responder to prevention and/or treatment with an IL-31 antagonist if the IL-31 concentration is equal to or higher than a predetermined value.

[8] The pharmaceutical composition of any one of [1] to [7-3], which is for the improvement of sleep disturbance caused by uremic pruritus.

[9] The pharmaceutical composition of [8], wherein the improvement of sleep disturbance is for increasing time from falling asleep to awakening, and/or for decreasing sleep latency (time from going to bed to falling asleep).

[10] The pharmaceutical composition of any one of [1] to [9], wherein the IL-31 antagonist is an antibody that inhibits IL-31 signaling.

[11] The pharmaceutical composition of [10], wherein the antibody does not exhibit cross-reactivity with IL-31RA from any of mouse, rat, and rabbit.

[12] The pharmaceutical composition of [10] or [11], wherein the antibody is an anti-IL-31 neutralizing antibody or an anti-IL-RA neutralizing antibody.

[13] The pharmaceutical composition of [12], wherein the anti-II,-31RA neutralizing antibody is any of:

(1) an anti-IL-31RA antibody comprising an H chain variable region comprising CDR1 as set forth in SEQ ID NO: 1, CDR2 as set forth in SEQ ID NO: 2, and CDR3 as set forth in SEQ ID NO: 3, and an L chain variable region comprising CDR1 as set forth in SEQ ID NO: 4, CDR2 as set forth in SEQ ID NO: 5, and CDR3 as set forth in SEQ ID NO: 6;

(2) an anti-IL-3 1RA antibody comprising an H chain variable region as set forth in SEQ ID NO: 7 and an L chain variable region as set forth in SEQ ID NO: 8; and

(3) an anti-IL-3 1RA antibody comprising an H chain as set forth in SEQ ID NO: 9 and an L chain as set forth in SEQ ID NO: 10.

[14] The pharmaceutical composition of any one of [1] to [13], wherein the uremic pruritus has been treated by a systemic or topical therapy excluding nalfurafine hydrochloride, and the treatment has not been (sufficiently) effective.

[15] The pharmaceutical composition of [14], wherein the systemic therapy is a treatment with an antihistamine agent or an antiallergic agent, and the topical therapy is a treatment with a moisturizing agent or a steroid.

[16] The pharmaceutical composition of any one of [1] to [15], wherein the uremic pruritus is caused by IL-31 signaling.

[17] A method for preventing and/or treating uremic pruritus, comprising administering an IL-31 antagonist to a subject with or potentially with uremic pruritus.

[18] The method of [17], wherein the IL-31 antagonist is administered to the subject with or potentially with uremic pruritus at 0.1 mg to 1000 mg/body/2 weeks, 0.1 mg to 1000 mg/body/4 weeks, or 0.1 mg to 1000 mg/body/8 weeks, repeatedly at the same dose and the same dosing interval.

[19] The method of [17], wherein the IL-31 antagonist is administered to the subject with or potentially with uremic pruritus at 0.01 mg to 10 mg/kg/2 weeks, 0.01 mg to 10 mg/kg/4 weeks, or 0.01 mg to 10 mg/kg/8 weeks, repeatedly at the same dose and the same dosing interval.

[20] The method of any one of [17] to [19], wherein the IL-31 antagonist is administered to a subject with or potentially with uremic pruritus having a serum IL-31 concentration equal to or higher than a predetermined value.

[21] The method of [20], which comprises:

measuring IL-31 concentration in a serum obtained from a subject with or potentially with uremic pruritus; determining the subject as a responder to prevention and/or treatment with an IL-31 antagonist if the IL-31 concentration is equal to or higher than a predetermined value; and administering an IL-31 antagonist to the subject determined to be a responder.

[21-2] The method of [20], which comprises:

determining a subject with or potentially with uremic pruritus whose serum IL-31 concentration has been measured as a responder to prevention and/or treatment with an IL-31 antagonist if the IL-31 concentration is equal to or higher than a predetermined value; and administering the IL-31 antagonist to the subject determined to be a responder.

[22] Use of an IL-31 antagonist in the manufacture of a medicament for prevention and/or treatment of uremic pruritus.

[23] The use of [22], wherein the IL-31 antagonist is administered to a subject with or potentially with uremic pruritus at 0.1 mg to 1000 mg/body/2 weeks, 0.1 mg to 1000 mg/body/4 weeks, or 0.1 mg to 1000 mg/body/8 weeks, repeatedly at the same dose and the same dosing interval.

[24] The use of [22], wherein the IL-31 antagonist is administered to a subject with or potentially with uremic pruritus at 0.01 mg to 10 mg/kg/2 weeks, 0.01 mg to 10 mg/kg/4 weeks, or 0.01 mg to 10 mg/kg/8 weeks, repeatedly at the same dose and the same dosing interval.

[25] The use of any one of [22] to [24], wherein the IL-31 antagonist is administered to a subject with or potentially with uremic pruritus having a serum IL-31 concentration equal to or higher than a predetermined value.

[26] A product comprising (i) a container; (ii) a pharmaceutical composition comprising an IL-31 antagonist as an active ingredient within the container; and (iii) a document instructing that the IL-31 antagonist be administered to a subject with or potentially with uremic pruritus at 0.1 mg to 1000 mg/body/2 weeks, 0.1 mg to 1000 mg/body/4 weeks, or 0.1 mg to 1000 mg/body/8 weeks, repeatedly at the same dose and the same dosing interval; and/or (iv) a document instructing that the IL-31 antagonist be administered to a subject with or potentially with uremic pruritus having a serum IL-31 concentration equal to or higher than a predetermined value.

[27] A product comprising (i) a container; (ii) a pharmaceutical composition comprising an IL-31 antagonist as an active ingredient within the container; and (iii) a document instructing that the IL-31 antagonist be administered to a subject with or potentially with uremic pruritus at 0.01 mg to 10 mg/kg/2 weeks, 0.01 mg to 10 mg/kg/4 weeks, or 0.01 mg to 10 mg/kg/8 weeks, repeatedly at the same dose and the same dosing interval; and/or (iv) a document instructing that the IL-31 antagonist be administered to a subject with or potentially with uremic pruritus having a serum IL-31 concentration equal to or higher than a predetermined value.

[28] A pharmaceutical composition for use in the prevention and/or treatment of uremic pruritus comprising an IL-31 antagonist as an active ingredient, which is further for the improvement of sleep disturbance caused by uremic pruritus.

[29] The pharmaceutical composition of [28], wherein the improvement of sleep disturbance is for increasing time from falling asleep to awakening, and/or for decreasing sleep latency (time from going to bed to falling asleep).

[30] An IL-31 antagonist for use in the prevention and/or treatment of uremic pruritus.

[31] The IL-31 antagonist of [30], which is administered to a subject with or potentially with uremic pruritus at 0.1 mg to 1000 mg/body/1 day to 12 weeks, 0.1 mg to 1000 mg/body/2 weeks, 0.1 mg to 1000 mg/body/4 weeks, or 0.1 mg to 1000 mg/body/8 weeks, repeatedly at the same dose and the same dosing interval.

[32] The IL-31 antagonist of [30], which is administered to a subject with or potentially with uremic pruritus at 0.01 mg to 10 mg/kg/1 day to 12 weeks, 0.01 mg to 10 mg/kg/2 weeks, 0.01 mg to 10 mg/kg/4 weeks, or 0.01 mg to 10 mg/kg/8 weeks, repeatedly at the same dose and the same dosing interval.

[33] The IL-31 antagonist of any one of [30] to [32], which is administered to a subject with or potentially with uremic pruritus having a serum IL-31 concentration equal to or higher than a predetermined value.

[34] A pharmaceutical composition for preventing and/or treating uremic pruritus, comprising an IL-31 antagonist as an active ingredient, wherein the IL-31 antagonist is administered to a subject with or potentially with uremic pruritus having a serum IL-31 concentration equal to or higher than a predetermined value.

[35] The pharmaceutical composition of [28] or [29], wherein the IL-31 antagonist is administered to a subject with or potentially with uremic pruritus at 0.1 mg to 1000 mg/body/2 weeks, 0.1 mg to 1000 mg/body/4 weeks, or 0.1 mg to 1000 mg/body/8 weeks, repeatedly at the same dose and the same dosing interval.

Any combinations of some or all of the one or more elements recited in any of [1] to [35] above are also included in the present disclosure, unless they are technically inconsistent based on common general knowledge in the art, and are contradictory in the context.

[Brief Description of Drawings]

[Fig. 1] Fig. 1 is a graph showing the effects of suppressing pruritus based on the VAS, after the administration of a single subcutaneous dose of CIM331 or placebo to patients with atopic dermatitis (AD).

[Fig. 2] Fig. 2 is a graph showing the effects of improving dermatitis based on the EASI score, after the administration of a single subcutaneous dose of CIM331 or placebo to patients with atopic dermatitis.

[Fig. 3] Fig. 3 is a graph showing the presence or absence of improvement in quality of life (QOL), using sleep efficiency as an index, after the administration of a single subcutaneous dose of CIM331 or placebo to patients with atopic dermatitis.

[Fig. 4] Fig. 4 is a graph showing the amounts of the topical steroid (Locoid) used after the administration of a single subcutaneous dose of CIM331 or placebo to patients with atopic dermatitis.

[Fig. 5] Fig. 5 is a graph showing serum concentration time course of CIM331 after the administration of a single subcutaneous dose of CIM331 to patients with atopic dermatitis.

[Fig. 6] Fig. 6 is a graph showing the frequency of IL-31-induced pruritic behavior after the administration of a single subcutaneous dose of 0.2 mg/kg of CIM331 to cynomolgus monkeys.

[Fig. 7] Fig. 7 is a graph showing the frequency of IL-31-induced pruritic behavior after the administration of a single subcutaneous dose of 1 mg/kg of CIM331 to cynomolgus monkeys.

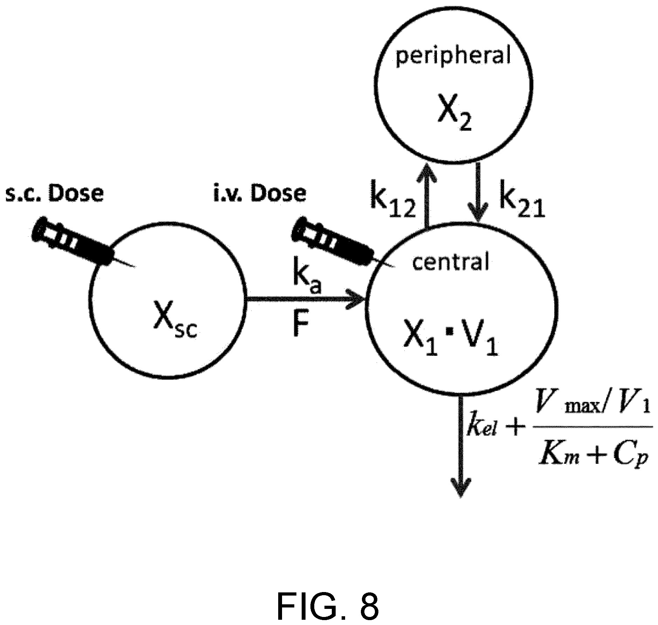

[Fig. 8] Fig. 8 shows a nonlinear analytical model adopting the Michaelis-Menten equation, wherein the symbols designate the following: $X_{sc}$: the amount of drug at the site of subcutaneous administration; $X_1$: the amount of drug in a central compartment; $X_2$: the amount of drug in a peripheral compartment; F: bioavailability; $k_{12}$: the drug transfer rate constant from the central compartment to the peripheral compartment; $k_{21}$: the drug transfer rate constant from the peripheral compartment to the central compartment; $k_a$: the absorption rate constant; $k_{el}$: the non-saturable elimination rate constant; $V_1$: the distribution volume of the central compartment; $V_{max}$: the elimination rate of the antibody when the antibody binds to all the receptors: $K_m$: the antibody concentration for binding to 50% of the entire amount of antigen; and $C_p$: the antibody concentration.

[Fig. 9] Fig. 9 shows predicted changes in the concentration of CIM331 in human serum.

[Fig. 10] Fig. 10 shows graphs each illustrating the relationship between the body weight and exposure in an optimal dosage simulation of CIM331 using a one-compartment model.

[Fig. 11] Fig. 11 shows estimated pruritus VAS at a year after the administration of CIM331 using an indirect turnover model.

[Fig. 12] Fig. 12 is a graph showing the pruritus suppressing effect based on VAS at each evaluation time point after the administration of the placebo, CIM331, or nalfurafine hydrochloride capsule to patients with uremic pruritus.

[Fig. 13] Fig. 13 is a graph showing the percentage of patients in which the pruritus suppressing effect is higher than the predefined level (VAS of less than 30 mm) among the patients with uremic pruritus who received administration of the placebo, CIM331, or nalfurafine hydrochloride capsule.

[Fig. 14] Fig. 14 shows association between pruritus VAS and serum IL-31 levels. The distribution of serum IL-31 concentrations in healthy volunteers (HV) and uremic pruritus (UP) patients is shown in log scale.

[Fig. 15] Fig. 15 shows changes of pruritus VAS from the baseline (BL) in pruritus patients divided into two categories according to the serum IL-31 concentration (cutoff value: 0.86 pg/mL). The changes are shown as the mean $\pm$

standard deviation (SD).

[Description of Embodiments]

Preferred non-limiting aspects of the present disclosure will be hereinafter described.

IL-31 (interleukin-31) is a T-cell cytokine. It is known that IL-31 is involved in pruritus, and in transgenic mice overexpressing IL-31, dermatitis-like symptoms similar to atopic dermatitis occur, and persistent scratching behavior is observed.

The nucleic acid sequence and amino acid sequence of human IL-31 are also known as RefSeq accession number NM_001014336 and RefSeq accession number NP_001014358, respectively.

The receptor for IL-31 is formed of a heterodimer of IL-31 receptor A (IL-3 1RA) and oncostatin M receptor (OSMR) (Nat Immunol (2004) 5, 752-60). IL-31RA, also referred to as NR10, is known to have a plurality of splicing variants (WO 00/075314). Among known splicing variants are NR10.1 (652 amino acids), NR10.2 (252 amino acids), NR10.3 (662 amino acids, also referred to as IL-31RAv4), and IL-31RAv3 (764 amino acids). Examples of preferred Il-31RA include NR10.3 (IL-31RAv4) and IL-31RAv3. The nucleic acid sequence and amino acid sequence of human IL-31RA (IL-31RAv4) are also known as RefSeq accession number NM_001242638 and RefSeq accession number NP_001229567, respectively. The nucleic acid sequence and the amino acid sequence of human IL-31RA (IL-31RAv3) are also known as RefSeq accession number NM_139017 and RefSeq accession number NP_620586, respectively. The nucleic acid sequence and the amino acid sequence of human OSMR are also known as RefSeq accession number NM_003999 and RefSeq accession number NP_003990, respectively.

As used herein, the IL-31 antagonist of the present disclosure, in one aspect, refers to a compound that suppresses or blocks IL-31-induced intracellular signaling. This compound can also be expressed as a compound that inhibits IL-31 signaling. Such a compound may be a naturally occurring compound or an artificially synthesized compound. Moreover, such a compound may be a low-molecular-weight compound or a high-molecular-weight compound such as a protein.

It is known that IL-31 that is present extracellularly triggers intracellular signaling via the IL-31 receptor (heterodimer of IL-31RA and OSMR) present on the cell surface (Nat Immunol (2004) 5, 752-760). The extracellular domain of the IL-31 receptor includes an IL-31-binding domain, and binding of IL-31 thereto causes a change in the conformation of the IL-31 receptor. As a result, intracellular signaling is initiated from the intracellular domain of the IL-31 receptor.

In one method, whether a certain compound inhibits IL-31 signaling can be verified by examining whether the compound inhibits binding of IL-31 to the IL-31 receptor. Examples of methods for making such a determination include an assay using ELISA or flow cytometry and an assay using surface plasmon resonance. With ELISA, for example, whether the compound inhibits the binding of IL-31 to the IL-31 receptor can be evaluated by immobilizing the IL-31 receptor (or IL-31RA) protein onto a plate, preparing a system for detecting the amount of IL-31 protein that binds thereto through the use of a secondary antibody such as an enzyme-labeled anti-IL-31 antibody, and determining whether or not the addition of the compound reduces the amount of detected IL-31 protein.

In an alternative method, whether a certain compound inhibits IL-31 signaling can be verified by examining whether the bioactivity induced by the action of IL-31 on cells is inhibited by the compound. The bioactivity is not particularly limited as long as it can be quantitatively or qualitatively determined using any method, and examples of such bioactivities include cell proliferative activity, protein phosphorylation activity, and gene/protein expression-inducing activity. For example, whether the compound inhibits IL-31 signaling can be evaluated by preparing cells that express the IL-31 receptor on the surface, and whose proliferative activity is induced in response to external IL-31 stimulation, and determining whether or not the addition of the compound reduces the IL-31-induced cell proliferative activity. As such cells, naturally occurring cells inherently expressing the IL-31 receptor may be used, or recombinant cells artificially synthesized to express the IL-31 receptor may be used. A suitable example of recombinant cells includes Ba/F3 cells expressing the IL-31 receptor. As a further alternative, the method described in the document of Dillon et al. (Nat Immunol (2004) 5, 752-760) may be used.

In the present disclosure, the degree of inhibition of IL-31 signaling by the IL-31 antagonist may be, but not limited to, at least 10% or more, preferably 20% or more, 30% or more, 40% or more, 50% or more, 60% or more, 70% or more, and 80% or more, 90% or more, 95% or more, or 98% or more.

In the present disclosure, a preferred embodiment of the compound that inhibits IL-31 signaling includes a protein that inhibits IL-31 signaling. The protein used herein is not particularly limited as long as it has the property of specifically binding to IL-31 or the IL-31 receptor. Examples of preferred proteins include antibodies and antibody-like molecules (Curr Opin Biotechnol (2006) 17, 653-658; Curr Opin Struct Biol (1997) 7, 463-469; and Protein Sci (2006) 15, 14-27). Antibodies include any antibodies such as monoclonal antibodies (e.g., IgG, IgM, IgE, IgA, and IgD), polyclonal antibodies, engineered antibodies (e.g., chimeric antibodies, humanized antibodies, and glycoengineered antibodies (WO 99/54342 and WO 00/61739)), antibody fragments (e.g., Fab, F(ab')2, Fv, and CDR), multi-specific antibodies (e.g., bispecific antibodies), and conjugated antibodies (e.g., antibodies conjugated with polyethylene glycol (PEG), radioactive isotopes, or drugs). On the other hand, examples of antibody-like molecules include DARPin (WO 2002/020565), Affibody (WO 1995/001937), Avimer (WO 2004/044011), and Adnectin (WO 2002/032925). More preferred is an antibody that inhibits IL-31 signaling. Examples of other preferred proteins that inhibit IL-31 signaling include a protein containing the extracellular domain of IL-31RA and a protein containing each extracellular domain of the IL-31 receptor (heterodimer of IL-31RA and OSMR).

In the present disclosure, preferred embodiments of the antibody that inhibits IL-31 signaling include an antibody that inhibits IL-31 signaling by binding to IL-31 (anti-IL-31 neutralizing antibody) and an antibody that inhibits IL-31 signaling by binding to the IL-31 receptor (anti-IL-31 receptor neutralizing antibody). Anti-IL-31 receptor neutralizing antibodies include an antibody that inhibits IL-31 signaling by binding to IL-31RA (anti-IL-31RA neutralizing antibody), an antibody that inhibits IL-31 signaling by binding to OSMR (anti-OSMR neutralizing antibody), and an antibody that inhibits IL-31 signaling by binding to the heterodimer of IL-31RA and OSMR (anti-IL-31RA/OSMR heterodimer neutralizing antibody). Of these anti-IL-31 receptor neutralizing antibodies, preferred is an anti-IL-3 1RA neutralizing antibody or anti-IL-31RA/OSMR heterodimer neutralizing antibody, and more preferred is an anti-IL-31RA neutralizing antibody.

The antibody that inhibits IL-31 signaling of the present disclosure, in one embodiment or another embodiment, preferably does not (substantially) exhibit cross-reactivity with IL-31RA from any of mouse, rat, and rabbit, although it has cross-reactivity with IL-31RA from humans and cynomolgus monkeys.

Methods for preparing antibodies are well known to those skilled in the art, and antibodies can be prepared using the hybridoma method (Nature (1975) 256, 495) or the phage antibody library method (Nature (1991) 352, 624-628, J Mol Biol (1991) 222, 581-597), for example. Using the IL-31 protein or IL-31 receptor protein as an immunogen, a large number of anti-IL-31 antibodies or anti-IL-31 receptor antibodies can be obtained by these methods. Furthermore, screening of these antibodies using any of the above-described methods for detecting the compound that inhibits IL-31 signaling allows an anti-IL-31 neutralizing antibody or an anti-IL-31 receptor neutralizing antibody to be obtained. A protein such as IL-31 or the IL-31 receptor may also be prepared using a genetic engineering technology known to those skilled in the art. Specifically, such a protein can be prepared by inserting a gene encoding a desired protein into an expression vector, introducing the vector into an appropriate host cell, and then purifying the target protein expressed in the host cell or in the culture supernatant of the host cell.

Examples of preferred anti-IL-31 neutralizing antibodies include the anti-IL-31 antibodies described in WO 2006/122079, WO 2008/028192, and WO 2009/071696.

Examples of preferred anti-IL-31RA neutralizing antibodies include, but are not limited to, the anti-IL-31RA (NR10) antibody described in WO 2007/142325, the anti-IL-31RA (NR10) antibody described in WO 2009/072604, and the anti-II,-31RA (NR10) antibody described in WO 2010/064697.

Moreover, examples of other preferred anti-IL-31RA neutralizing antibodies include anti-human IL-31RA (neutralizing) antibodies, specifically including an anti-IL-31RA (neutralizing) antibody that recognizes domain 1 and/or domain 2 of human IL-31RA. As used herein, domain 1 of human IL-31RA designates the region from amino acid at position 53 to amino acid at position 152 (LPAKP to LENIA) in the amino acid sequence as set forth in SEQ ID NO: 11. Domain 2 designates the region from amino acid at position 153 to amino acid at position 259 (KTEPP to EEEAP) in the amino acid sequence as set forth in SEQ ID NO: 11.

Without any limitation, of the anti-IL-3 1RA neutralizing antibodies, more preferred is an anti-IL-31RA antibody comprising an H chain (heavy chain) variable region comprising CDR1 as set forth in SEQ ID NO: 1, CDR2 as set forth in SEQ ID NO: 2, and CDR3 as set forth in SEQ ID NO: 3, and an L chain (light chain) variable region comprising CDR1 as set forth in SEQ ID NO: 4, CDR2 as set forth in SEQ ID NO: 5, and CDR3 as set forth in SEQ ID NO: 6. More preferred is an anti-IL-3 1RA antibody comprising an H chain variable region as set forth in SEQ ID NO: 7 and an L chain variable region as set forth in SEQ ID NO: 8. Particularly preferred is an anti-IL-3 1RA antibody comprising an H chain as set forth in SEQ ID NO: 9 and an L chain as set forth in SEQ ID NO: 10.

Accordingly, in one non-limiting embodiment, a pharmaceutical composition comprising any of:

(1) an anti-IL-31RA antibody comprising an H chain variable region comprising CDR1 as set forth in SEQ ID NO: 1, CDR2 as set forth in SEQ ID NO: 2, and CDR3 as set forth in SEQ ID NO: 3, and an L chain variable region comprising CDR1 as set forth in SEQ ID NO: 4, CDR2 as set forth in SEQ ID NO: 5, and CDR3 as set forth in SEQ ID NO: 6;
(2) an anti-IL-3 1RA antibody comprising an H chain variable region as set forth in SEQ ID NO: 7 and an L chain variable region as set forth in SEQ ID NO: 8;
(3) an anti-IL-3 1RA antibody comprising an H chain as set forth in SEQ ID NO: 9 and an L chain as set forth in SEQ ID NO: 10; or
(4) nemolizumab

as an active ingredient may be administered to a subject (e.g. human patient) with or potentially with uremic pruritus at:

(A) 0.1 mg to 1000 mg/body/2 weeks, 0.1 mg to 1000 mg/body/4 weeks, 0.1 mg to 1000 mg/body/8 weeks, 25 mg to 100 mg/body/4 weeks, 50 mg to 100 mg/body/4 weeks, 5 mg to 75 mg/body/4 weeks, 5 mg to 50 mg/body/4 weeks, 5 mg to 25 mg/body/4 weeks, 5 mg to 20 mg/body/4 weeks, 5 mg/body/4 weeks, 10 mg/body/4 weeks, 15 mg/body/4 weeks, 20 mg/body/4 weeks, 25 mg/body/4 weeks, 30 mg/body/4 weeks (in this case, for example, the initial dose may be 60 mg/body, the interval between the initial dose and the first continued dose may be 4 weeks, and the continued dose may be 30 mg/body/4 weeks), or 60 mg/body/4weeks; or

(B) 0.01 mg to 10 mg/kg/2weeks, 0.01 mg to 10 mg/kg/4 weeks, 0.01 mg to 10 mg/kg/8 weeks, 0.125 to 2.0 mg/kg/4 weeks, 0.125 to 0.5 mg/kg/4 weeks, 0.5 mg to 2.0 mg/kg/4 weeks, or 0.5 mg/kg/4 weeks.

In this case, the serum IL-31 concentration in the subject may be equal to or higher than a predetermined value (for example, 0.15 pg/mL or higher, 0.2 pg/mL or higher, 0.3 pg/mL or higher, 0.4 pg/mL or higher, 0.5 pg/mL or higher, 0.6 pg/mL or higher, 0.7 pg/mL or higher, 0.8 pg/mL or higher, 0.86 pg/mL or higher, 0.9 pg/mL or higher, 1.0 pg/mL or higher, 1.1 pg/mL or higher, 1.25 pg/mL or higher, 1.5 pg/mL or higher, 1.75 pg/mL or higher, 2 pg/mL or higher, 2.25 pg/mL or higher, or 2.5 pg/mL or higher, or higher, as measured using, for example, ultrasensitive enzyme-linked immunosorbent assay (ELISA; SiMoATM, Quanterix, Billerica, MA, USA).

Known methods for defining CDRs include the method according to Kabat et al. (Sequences of Proteins of Immunological Interest, 5th Ed (1991), Bethesda, MD), the method according to Chothia et al. (Science (1986) 233, 755-758), and the method based on antigen-antibody contact regions (J Mol Biol (1996) 262, 732-745). Specifically, each of the methods defines CDRs as follows:

| CDR | Kabat | Chothia | Contact |
|---|---|---|---|
| L1 | L24-L34 | L24-L34 | L30-L36 |
| L2 | L50-L56 | L50-L56 | L46-L55 |
| L3 | L89-L97 | L89-L97 | L89-L96 |
| H1 | H31-H35B | H26-H32/34 | H30-H35B (Kabat numbering) |
| H1 | H31-H35 | H26-H32 | H30-H35 (Chothia numbering) |
| H2 | H50-H65 | H52-H56 | H47-H58 |
| H3 | H95-H102 | H95-H102 | H93-H101 |

An example of a preferred anti-IL-31RA neutralizing antibody of the present disclosure includes an anti-IL-31RA antibody comprising CDR1, CDR2, and CDR3 contained in the H chain variable region as set forth in SEQ ID NO: 7, and CDR1, CDR2, and CDR3 contained in the L chain variable region as set forth in SEQ ID NO: 8, as H chain CDR1, CDR2, and CDR3, and L chain CDR1, CDR2, and CDR3, respectively. The CDRs in such an antibody may be defined in accordance with any of the method according to Kabat et al., the method according to Chothia et al., and the method based on antigen-antibody contact regions, or in accordance with a combination of these methods.

Similarly, preferred as the anti-IL-31RA neutralizing antibody is an anti-IL-31RA antibody that binds to the same epitope as that of the anti-IL-3 1RA antibody defined by the above-described sequences of CDRs of the H chain and L chain, H chain variable region and L chain variable region sequences, and full-length H chain and L chain sequences. An epitope refers to a specific structural unit of an antigen to which an antibody recognizes and binds. When the antigen is a polypeptide, the epitope typically consists of about 6 to 10 amino acids. Epitope identification can be performed using a method known to those skilled in the art, for example, a method of synthesizing peptides by fragmentation of the antigen, a method of introducing site-directed mutagenesis into the antigen (e.g., arginine/glutamic acid scanning, J Biol Chem (1995) 270, 21619-21625, J Biol Chem (2006) 281, 20464-20473), and a method of crystallizing an antigen-antibody complex (Using Antibodies: A Laboratory Manual (1999), Cold Spring Harbor Laboratory Press, New York). In the present disclosure, the recitation "binds to the same epitope" means that the epitopes to which two antibodies bind at least partially overlap each other. The degree of the overlap is, but not limited to, at least 10% or more, preferably 20% or more, 30% or more, 40% or more, 50% or more, 60% or more, 70% or more, and 80% or more, particularly preferably 90% or more, and most preferably 100%.

Similarly, preferred as the anti-IL-31RA neutralizing antibody is an anti-IL-31RA antibody that competes for binding to IL-31RA with the anti-IL-31RA antibody defined by the above-described sequences of CDRs of the H chain and L chain, H chain variable region and L chain variable region sequences, and full-length H chain and L chain sequences. Whether the two antibodies compete with each other can be evaluated by using a competition binding assay utilizing ELISA, for example. A specific method is as follows: One of the two antibodies is pre-labeled with, for example, fluorescence. A system for detecting the binding of the antibody (labeled antibody) to the antigen is prepared. A comparison is made between the case where the other unlabeled antibody (test antibody) coexists and the case where the test antibody does not coexist in the system. If the level of binding of the labeled antibody to the antigen is decreased in the presence of the test antibody, it can be judged that the test antibody and the labeled antibody compete with each other. In the present disclosure, the degree of competition is, but not particularly limited to, at least 10% or more, preferably 20% or more, 30% or more, 40% or more, 50% or more, 60% or more, 70% or more, and 80% or more, and particularly preferably 90% or more, 95% or more, and 98% or more (that is, the level of binding of the other antibody is decreased).

A nucleotide sequence or amino acid sequence encoding the antibody that inhibits IL-31 signaling (e.g., the anti-IL-31 neutralizing antibody or the anti-IL-31RA neutralizing antibody) of the present disclosure can be obtained using a method known to those skilled in the art. Amino acids contained in the amino acid sequence of the antibody described in the present disclosure may undergo a post-translational modification (e.g., a modification involving the conversion of N-terminal glutamine to pyroglutamate by pyroglutamylation is well known to those skilled in the art). Even if the amino acids are thus post-translationally modified, the resulting amino acid sequence is naturally included in the amino acid sequences described in the present disclosure.

Uremic pruritus in the present disclosure is not limited and may preferably be uremic pruritus that results from IL-31 signaling or that is caused by IL-31. Moreover, uremic pruritus may be an uremic pruritus that is responsive to prevention and/or treatment by an IL-31 antagonist. Such uremic pruritus may be uremic pruritus in a patient whose serum IL-31 concentration before starting the IL-31 antagonist administration is equal to or higher than a predetermined value. In this case, the IL-31 antagonist administration is expected to result in exertion of higher pruritus-improving effect. Such a predetermined serum IL-31 concentration that may serve as a baseline at which the exertion of high pruritus-improving effect can be expected may be, for example, 0.86 pg/mL. In one non-limiting embodiment, the present inventors surprisingly demonstrated that a group of uremic pruritus patients who showed significant reduction of itching as a result of IL-31 antagonist administration had higher serum IL-31 concentrations than a group of uremic pruritus patients who did not. This means that serum IL-31 concentration may be used as an index to predict the effectiveness of treatment or prevention of uremic pruritus and/or itching thereof using an IL-31 antagonist (e.g. nemolizumab). In a non-limiting embodiment, the predetermined serum IL-31 concentration may be, for example, 0.15 pg/mL or higher, 0.2 pg/mL or higher, 0.3 pg/mL or higher, 0.4 pg/mL or higher, 0.5 pg/mL or higher, 0.6 pg/mL or higher, 0.7 pg/mL or higher, 0.8 pg/mL or higher, 0.86 pg/mL or higher, 0.9 pg/mL or higher, 1.0 pg/mL or higher, 1.1 pg/mL or higher, 1.25 pg/mL or higher, 1.5 pg/mL or higher, 1.75 pg/mL or higher, 2 pg/mL or higher, 2.25 pg/mL or higher, or 2.5 pg/mL or higher, or higher, as measured using ultrasensitive enzyme-linked immunosorbent assay (ELISA; SiMoA™, Quanterix, Billerica, MA, USA).

Uremic pruritus in the present disclosure is not limited, and may be pruritus for which a systemic therapy (such as antihistamine agents and antiallergic agents) or topical therapy (such as moisturizing agents and steroids), excluding nalfurafine hydrochloride, has been carried out but not sufficiently effective.

Pruritus in dialysis patients is considered to be caused by various factors, such as accumulation of uremic substances, aberrance of calcium and phosphorus metabolisms, secondary hyperparathyroidism, complement activation by the dialysis membrane or effects by heparin, drying of the skin, involvement of pruritic mediators such as amines (histamine, serotonin, etc.) and neuropeptides (substance P, etc.), aberrance of the immune system, and aberrance of endogenous opioids. However, the mechanism of disease development has not been revealed. Therefore, there is no unified guideline for the treatment of pruritus in dialysis patients. Although efforts are made to use a highly biocompatible dialysis membrane and to change dialysis conditions, to modulate calcium and phosphorus concentrations, to treat secondary hyperparathyroidism, to topically apply a moisturizing agent or steroid, to orally administer an antiallergic agent or antihistamine agent, to conduct ultraviolet therapy and so forth, there are many cases where these treatments fail to achieve a successful outcome. In Japan, nalfurafine hydrochloride was launched in 2009 with the indication of "improving pruritus in hemodialysis patients (for use only when sufficient efficacy is not obtained with existing treatments)". However, satisfaction by the treatment is presumed not to be sufficiently high because the degree of pruritus improvement by nalfurafine hydrochloride varies among patients (Shigeki Yamada, et al., "Investigation of the status of uremic pruritus in hemodialysis patients and the efficacy of nalfurafine hydrochloride - Questionnaire administered to 1,936 patients from 17 institutions in Tokai area of Japan -", Journal of the Japanese Society for Dialysis Therapy, 2012; 45:1133-40) and insomnia is observed in 15.8% of the patients as a side effect (Remitch (registered trademark) Capsules 2.5 μg interview form, revised June 2013 (8th edition)). Therefore, there is a need for treatments having enough efficacy and high safety. (1) Nalfurafine hydrochloride oral administration (Brand name: Remitch Capsules)

Usually, for adults, 2.5 μg of nalfurafine hydrochloride is orally administered once a day after an evening meal or before going to bed. The dose may be increased depending on the symptoms but up to 5 μg once a day.

The severity of uremic pruritus (mild, moderate, severe, etc.) can be graded according to grading methods known to those skilled in the art which score the intensity of itching a subject feels, which methods include, for example, Shiratori severity scores, Visual Analogue Scale (VAS), and pruritus Verbal Rating Scale (VRS).

For example, in one embodiment, a subject whose intensity of itching has been measured using VAS and graded as "50 mm or more" may be determined to have uremic pruritus. The subject is not particularly limited as long as the uremic pruritus is pruritus for which a systemic therapy (such as antihistamine agents and antiallergic agents) or topical therapy (such as moisturizing agents and steroids), excluding nalfurafine hydrochloride, has been carried out but not sufficiently effective.

As used herein, the "subject" may preferably be an animal, more preferably a mammal (which may be a mouse, a rat, a rabbit, a dog, a monkey (e.g., a cynomolgus monkey), or the like), and particularly preferably a human, but not limited thereto. The human may be an adult (18 years or older) or a child (0 to younger than 18 years, for example, 6 months to younger than 18 years).

In one aspect, the present disclosure relates to a pharmaceutical composition for prevention and/or treatment of atopic dermatitis (the "pharmaceutical composition for prevention and/or treatment" may also be expressed as "a prophylactic agent and/or a therapeutic agent") comprising an IL-31 antagonist as an active ingredient.

In this case, it may be intended that the IL-31 antagonist be administered at a predetermined dosing interval and a predetermined dose (dosage), repeatedly at the same dose and the same dosing interval, as described in detail below.

In one embodiment, the pharmaceutical composition of the present disclosure may be used for prevention and/or treatment of uremic pruritus.

In a specific embodiment, the pharmaceutical composition of the present disclosure may be used for prevention and/or treatment of uremic pruritus in a patient whose serum IL-31 concentration before starting IL-31 antagonist administration is equal to or higher than a predetermined value. In an exemplary embodiment, the pharmaceutical composition of the present disclosure may be for administration only to a subject that has been determined to be a responder to prevention and/or treatment with an IL-31 antagonist by a method for predicting the response of a subject to prevention and/or treatment with an IL-31 antagonist, the method comprising:

(1) measuring IL-31 concentration in a serum obtained from a subject with or potentially with uremic pruritus; and
(2) determining the subject as a responder to prevention and/or treatment with an IL-31 antagonist if the IL-31 concentration is equal to or higher than a predetermined value.

In this case, the administration of the pharmaceutical composition of the present disclosure is expected to result in exertion of higher pruritus-improving effect. Such a predetermined serum IL-31 concentration that may serve as a baseline at which the exertion of high pruritus-improving effect can be expected may be, for example, 0.86 pg/mL. The concentration of IL-31 in a serum obtained from a patient can be measured by any method known to persons skilled in the art. In a non-limiting preferred embodiment, the predetermined serum IL-31 concentration may be measured using ultrasensitive enzyme-linked immunosorbent assay (ELISA; SiMoATM, Quanterix, Billerica, MA, USA).

In a still further embodiment or another embodiment, the pharmaceutical composition of the present disclosure may be used for improvement of sleep disturbance caused by uremic pruritus. The improvement of sleep disturbance may be characterized by, for example, an increase in the time from falling asleep to awakening, and/or a decrease in sleep latency (the time from going to bed to falling asleep).

In one embodiment of the present disclosure, the prevention and/or treatment of uremic pruritus may refer to, but not limited to, for example, administering a drug or the like to a subject who exhibits uremic pruritus to suppress the symptoms thereof, and/or, for example, administering a drug or the like to a subject who has previously developed uremic pruritus to eliminate the development or reduce the incidence rate of the symptoms thereof. By improving pruritus, it is expected that, for example, the QOL, quality of sleep, or vital prognosis of the dialysis patent is improved.

The subject potentially with uremic pruritus may be a subject who has had uremic pruritus in the past, and may have a risk of recurrence of the symptoms, or may be a subject with suspected uremic pruritus before a doctor or the like makes a diagnosis or determination that the subject has uremic pruritus, but not limited thereto.

In one embodiment, in some cases, the prevention and treatment of uremic pruritus may be interpreted synonymously.

In a single subcutaneous dose study of the IL-31 antagonist for patients with atopic dermatitis in the Examples, an IL-31 antagonist-treated group demonstrated an improvement in sleep efficiency.

As stated above, it is considered that improvement of uremic pruritus is important for the improvement of the QOL, quality of sleep, or vital prognosis of the patient.

Therefore, in another non-limiting aspect, the present disclosure relates to a pharmaceutical composition for prevention and/or treatment of uremic pruritus comprising an IL-31 antagonist as an active ingredient, which is further for improvement of sleep disturbance caused by uremic pruritus. The improvement of sleep disturbance may be characterized by, for example, an increase in the time from falling asleep to awakening, and/or a decrease in sleep latency (the time from going to bed to falling asleep). Alternatively, in a further non-limiting aspect or another non-limiting aspect, the present disclosure relates to a pharmaceutical composition for improving the QOL caused by uremic pruritus. Alternatively, in a further non-limiting aspect or another non-limiting aspect, the present disclosure relates to a pharmaceutical composition for improving the vital prognosis caused by uremic pruritus.

As used herein, "administered repeatedly at the same dose and the same dosing interval" is intended to mean that the dose of the IL-31 antagonist of the present disclosure initially administered to a subject (initial dose) is equal to its maintenance dose subsequently administered (namely, the dose at which the IL-31 antagonist continues to be administered after the administration of the initial dose), and the IL-31 antagonist is administered at equal dosing intervals (intervals between doses). Specifically, for example, the above-described recitation means that the interval between the administration of the initial dose and the administration of the first continued dose, every interval between the administration of the ... n-th (n is an integer of 1 or more) continued dose and the administration of the (n + 1)-th continued dose is equal, and the doses are equal. A person skilled in the art will naturally understand that, for decided dosing intervals (e.g., every 4 weeks in the case where the dosing interval is decided to be every 4 weeks), each dosing interval has a "tolerable range", and the skilled person can decide the tolerable range, as appropriate.

In one embodiment, in the present disclosure, the repeated administration may mean that, for example, the number of continued doses subsequent to the initial dose is 1 to 10000 or more, for example, and more specifically, 1, 2, 3, 4, 5, 6, 7, 8, 9, 10, 11, ... 15, ... 20, ... 25, ... 35, ... 40, ... 50, ... 60, ... 70, ... 80, ... 90, ... 100, ... 500, ... 1000, ... 10000, ... , for example, but not limited thereto.

In one embodiment, it is contemplated that the dosing interval of the pharmaceutical composition or the IL-31 antagonist of the present disclosure is a minimum period of 1 day or longer and a maximum period of 12 weeks or shorter, and may specifically be 1 day, 2 days, 3 days, 4 days, 5 days, 6 days, 1 week, 10 days, 2 weeks, 3 weeks, 4 weeks, 5 weeks, 6 weeks, 7 weeks, 8 weeks, 9 weeks, 10 weeks, 11 weeks, 12 weeks, 1 month, 2 months, or 3 months, for example. The dosing interval may also be expressed differently, and may be specified as once daily or once in 12 weeks, or may be specified as every day or every 12 weeks, for example.

In the present disclosure, the dosage (dose) may be expressed in terms of units other than mg/kg, for example, a fixed dose (mg/body) corresponding to a dose calculated in terms of body weight, or a dose calculated in terms of body surface area (mg/m$^2$).

For example, when it is intended that the IL-31 antagonist of the present disclosure be administered at a fixed dose (mg/body) to a subject with or potentially with uremic pruritus, dosages in mg/kg of the IL-31 antagonist of the present disclosure may be converted to dosages in mg/body, and an appropriate dosage (mg/body) may be selected and administered to the subject. In this case, although the logic for converting mg/kg to mg/body is not limited, a dosage in mg/body may be determined as appropriate, using a logic known to those skilled in the art. One possible example of such a logic is as follows:

Assuming that there are the minimum effective serum concentration and the maximum tolerable (empirical) serum concentration of the IL-31 antagonist in the present disclosure, changing of a dosage in mg/kg into a dosage in mg/body may be considered such that a serum concentration of the IL-31 antagonist is achieved within this range of concentrations, regardless of body weight. The subject may be a subject having a body weight below 100 kg or below 120 kg, for example. A dosage in mg/body for a subject with a high body weight (e.g., a body weight over 100 kg or over 120 kg) may be increased, as required, but not limited thereto. Moreover, if a dosage in mg/body for a child with a low body weight may markedly increase the exposure, a dosage in mg/kg may be considered.

Alternatively, based on the body weight of a typical uremic pruritus patient estimated based on statistics, the dosage per body weight (mg/kg) may be converted into a fixed dose (mg/body) using the following calculation formula: [dosage per body weight] x [estimated body weight] = fixed dose. For example, assuming that the estimated body weight of an adult is, for example, 60 kg, the fixed dose may be calculated to be 30 mg/body from a dosage per body weight of 0.5 mg/kg. Similarly, assuming that the estimated body weight of a child is, for example, 30 kg, the fixed dose may be calculated to be 15 mg/body from a dosage per body weight of 0.5 mg/kg.

In a non-limiting embodiment, for a subject with or potentially with uremic pruritus, for example, a human adult and/or child, the IL-31 antagonist of the present disclosure may be administered at one dosage selected from 0.1 mg to 1000 mg/body, for example, 0.2 mg to 360 mg/body, and preferably, for example, 5 mg to 100 mg/body, 5 mg to 75 mg/body, 5 mg to 50 mg/body, 5 mg to 25 mg/body, 5 mg to 20 mg/body, 10 mg to 100 mg/body, 10 mg to 75 mg/body, 10 mg to 50 mg/body, 10 mg to 40 mg/body, 10 mg to 39.5 mg/body, 10 mg to 39 mg/body, 10 mg to 38.5 mg/body, 10 mg to 38 mg/body, 10 mg to 37.5 mg/body, 15 mg to 100 mg/body, 15 mg to 75 mg/body, 15 mg to 50 mg/body, 15 mg to 40 mg/body, 15 mg to 39.5 mg/body, 15 mg to 39 mg/body, 15 mg to 38.5 mg/body, 15 mg to 38 mg/body, 15 mg to 37.5 mg/body, 17.5 mg to 100 mg/body, 17.5 mg to 75 mg/body, 17.5 mg to 50 mg/body, 17.5 mg to 40 mg/body, 17.5 mg to 39.5 mg/body, 17.5 mg to 39 mg/body, 17.5 mg to 38.5 mg/body, 17.5 mg to 38 mg/body, 17.5 mg to 37.5 mg/body, 20 mg to 100 mg/body, 20 mg to 75 mg/body, 20 mg to 50 mg/body, 20 mg to 40 mg/body, 20 mg to 39.5 mg/body, 20 mg to 39 mg/body, 20 mg to 38.5 mg/body, 20 mg to 38 mg/body, 20 mg to 37.5 mg/body, 22.5 mg to 100 mg/body, 22.5 mg to 75 mg/body, 22.5 mg to 50 mg/body, 22.5 mg to 40 mg/body, 22.5 mg to 39.5 mg/body, 22.5 mg to 39 mg/body, 22.5 mg to 38.5 mg/body, 22.5 mg to 38 mg/body, 22.5 mg to 37.5 mg/body, 25 mg to 500 mg/body, 25 mg to 200 mg/body, 25 mg to 120 mg/body, 25 mg to 110 mg/body, 25 mg to 100 mg/body, 25 mg to 90 mg/body, 25 mg to 80 mg/body, 25 mg to 79 mg/body, 25 mg to 78 mg/body, 25 mg to 77 mg/body, 25 mg to 76 mg/body, 25 mg to 75 mg/body, 25 mg to 74 mg/body, 25 mg to 73 mg/body, 25 mg to 72 mg/body, 25 mg to 71 mg/body, 25 mg to 70 mg/body, 25 mg to 50 mg/body, 30 mg to 50 mg/body, 30 mg to 75 mg/body, 30 mg to 100 mg/body, 30 mg to 150 mg/body, 30 mg to 200 mg/body, 30 mg to 250 mg/body, 30 mg to 300 mg/body, 40 mg to 70 mg/body, 40 mg to 71 mg/body, 40 mg to 72 mg/body, 40 mg to 73 mg/body, 40 mg to 74 mg/body, 40 mg to 75 mg/body, 40 mg to 76 mg/body, 40 mg to 77 mg/body, 40 mg to 78 mg/body, 40 mg to 79 mg/body, 40 mg to 80 mg/body, 40 mg to 90 mg/body, 40 mg to 100 mg/body, 40 mg to 110 mg/body, 40 mg to 120 mg/body, 42.5 mg to 70 mg/body, 42.5 mg to 71 mg/body, 42.5 mg to 72 mg/body, 42.5 mg to 73 mg/body, 42.5 mg to 74 mg/body, 42.5 mg to 75 mg/body, 42.5 mg to 76 mg/body, 42.5 mg to 77 mg/body, 42.5 mg to 78 mg/body, 42.5 mg to 79 mg/body, 42.5 mg to 80 mg/body, 42.5 mg to 90 mg/body, 42.5 mg to 100 mg/body, 42.5 mg to 110 mg/body, 42.5 mg to 120 mg/body, 45 mg to 70 mg/body, 45 mg to 71 mg/body, 45 mg to 72 mg/body, 45 mg to 73 mg/body, 45 mg to 74 mg/body, 45 mg to 75 mg/body, 45 mg to 76 mg/body, 45 mg to 77 mg/body, 45 mg to 78 mg/body, 45 mg to 79 mg/body, 45 mg to 80 mg/body, 45 mg to 90 mg/body, 45 mg to 100 mg/body, 45 mg to 110 mg/body, 45 mg to 120 mg/body, 47.5 mg to 70 mg/body, 47.5 mg to 71 mg/body, 47.5 mg to 72 mg/body, 47.5 mg to 73 mg/body, 47.5 mg to 74 mg/body, 47.5 mg to 75 mg/body, 47.5 mg to 76 mg/body, 47.5 mg to 77 mg/body, 47.5 mg to 78 mg/body, 47.5 mg to 79 mg/body, 47.5 mg to 80 mg/body, 47.5 mg to 90 mg/body, 47.5 mg to 100 mg/body, 47.5 mg to 110 mg/body, 47.5 mg to 120 mg/body, 50 mg to 70 mg/body, 50 mg to 71 mg/body, 50 mg to 72 mg/body, 50 mg to 73 mg/body, 50 mg to 74 mg/body, 50 mg to 75 mg/body, 50 mg to 76 mg/body, 50 mg to 77 mg/body, 50 mg to 78 mg/body, 50 mg to 79 mg/body, 50 mg to 80 mg/body, 50 mg to 90 mg/body, 50 mg to 100 mg/body, 50 mg to 110 mg/body, 50 mg to 120 mg/body, 50 mg to 150 mg/body, 50 mg to 200 mg/body, 50 mg to 250 mg/body, 50 mg to 300 mg/body, 52.5 mg to 70 mg/body, 52.5 mg to 71 mg/body, 52.5 mg to 72 mg/body, 52.5 mg to 73 mg/body, 52.5 mg to 74 mg/body, 52.5 mg to 75 mg/body, 52.5 mg to 76 mg/body, 52.5 mg to 77 mg/body, 52.5 mg to 78 mg/body, 52.5 mg to 79 mg/body, 52.5 mg to 80 mg/body, 52.5 mg to 90 mg/body, 52.5 mg to 100 mg/body, 52.5 mg to 110 mg/body, 52.5 mg to 120 mg/body, 75 mg to 100 mg/body, 75 mg to 150 mg/body, 75 mg to 200 mg/body, 75 mg to 250 mg/body, 75 mg to 300 mg/body, 100 mg to 150 mg/body, 100 mg to 200 mg/body, 100 mg to 250 mg/body, 100 mg to 300 mg/body, 150 mg to 200 mg/body, 150 mg to 250 mg/body, 150 mg to 300 mg/body, 200 mg to 250 mg/body, and 200 mg to 300 mg/body, and at a dosing interval as described above, repeatedly at the same dosage and the same dosing interval. For the sake of avoiding any doubt, it is expressly noted that a person skilled in the art who has read, for example, "50 mg to 200 mg/body", can naturally understand that it directly and unambiguously refers to, for example, specific individual values such as 50 mg/body, 50.5 mg/body, 51 mg/body, 51.5 mg/body, 52 mg/body, 52.5 mg/body, 53 mg/body, 53.5 mg/body, 54 mg/body, 54.5 mg/body, 55 mg/body, 55.5 mg/body, 56 mg/body, 56.5 mg/body, 57 mg/body, 57.5 mg/body, 58 mg/body, 58.5 mg/body, 59 mg/body, 59.5 mg/body, 60 mg/body, 60.5 mg/body, 61 mg/body, 61.5 mg/body, 62 mg/body, 62.5 mg/body, 63 mg/body, 63.5 mg/body, 64 mg/body, 64.5 mg/body, 65 mg/body, 65.5 mg/body, 66 mg/body, 66.5 mg/body, 67 mg/body, 67.5 mg/body, 68 mg/body, 68.5 mg/body, 69 mg/body, 69.5 mg/body, 70 mg/body, 70.5 mg/body, 71 mg/body, 71.5 mg/body, 72 mg/body, 72.5 mg/body, 73 mg/body, 73.5 mg/body, 74 mg/body, 74.5 mg/body, 75 mg/body, 75.5 mg/body, 76 mg/body, 76.5 mg/body, 77 mg/body, 77.5 mg/body, 78 mg/body, 78.5 mg/body, 79 mg/body, 79.5 mg/body, 80 mg/body, 80.5 mg/body, 81 mg/body, 81.5 mg/body, 82 mg/body, 82.5 mg/body, 83 mg/body, 83.5 mg/body, 84 mg/body, 84.5 mg/body, 85 mg/body, 85.5 mg/body, 86 mg/body, 86.5 mg/body, 87 mg/body, 87.5 mg/body, 88 mg/body, 88.5 mg/body, 89 mg/body, 89.5 mg/body, 90 mg/body, 90.5 mg/body, 91 mg/body, 91.5 mg/body, 92 mg/body, 92.5 mg/body, 93 mg/body, 93.5 mg/body, 94 mg/body, 94.5 mg/body, 95 mg/body, 95.5 mg/body, 96 mg/body, 96.5 mg/body, 97 mg/body, 97.5 mg/body, 98 mg/body, 98.5 mg/body, 99 mg/body, 99.5 mg/body, 100 mg/body, 100.5 mg/body, 101 mg/body, 101.5 mg/body, 102 mg/body, 102.5 mg/body, 103 mg/body, 103.5 mg/body, 104 mg/body, 104.5 mg/body, 105 mg/body, 105.5 mg/body, 106 mg/body, 106.5 mg/body, 107 mg/body, 107.5 mg/body, 108 mg/body, 108.5 mg/body, 109 mg/body, 109.5 mg/body, 110 mg/body, 110.5 mg/body, 111 mg/body, 111.5 mg/body, 112 mg/body, 112.5 mg/body, 113 mg/body, 113.5 mg/body, 114 mg/body, 114.5 mg/body, 115 mg/body, 115.5 mg/body, 116 mg/body, 116.5 mg/body, 117 mg/body, 117.5 mg/body, 118 mg/body, 118.5 mg/body, 119 mg/body, 119.5 mg/body, 120 mg/body, 120.5 mg/body, 121 mg/body, 121.5 mg/body, 122 mg/body, 122.5 mg/body, 123 mg/body, 123.5 mg/body, 124 mg/body, 124.5 mg/body, 125 mg/body, 125.5 mg/body, 126 mg/body, 126.5 mg/body, 127 mg/body, 127.5 mg/body, 128 mg/body, 128.5 mg/body, 129 mg/body, 129.5 mg/body, 130 mg/body, 130.5 mg/body, 131 mg/body, 131.5 mg/body, 132 mg/body, 132.5 mg/body, 133 mg/body, 133.5 mg/body, 134 mg/body, 134.5 mg/body, 135 mg/body, 135.5 mg/body, 136 mg/body, 136.5 mg/body, 137 mg/body, 137.5 mg/body, 138 mg/body, 138.5 mg/body, 139 mg/body, 139.5 mg/body, 140 mg/body, 140.5 mg/body, 141 mg/body, 141.5 mg/body, 142 mg/body, 142.5 mg/body, 143 mg/body, 143.5 mg/body, 144 mg/body, 144.5 mg/body, 145 mg/body, 145.5 mg/body, 146 mg/body, 146.5 mg/body, 147 mg/body, 147.5 mg/body, 148 mg/body, 148.5 mg/body, 149 mg/body, 149.5 mg/body, 150 mg/body, 150.5 mg/body, 151 mg/body, 151.5 mg/body, 152 mg/body, 152.5 mg/body, 153 mg/body, 153.5 mg/body, 154 mg/body, 154.5 mg/body, 155 mg/body, 155.5 mg/body, 156 mg/body, 156.5 mg/body, 157 mg/body, 157.5 mg/body, 158 mg/body, 158.5 mg/body, 159 mg/body, 159.5 mg/body, 160 mg/body, 160.5 mg/body, 161 mg/body, 161.5 mg/body, 162 mg/body, 162.5 mg/body, 163 mg/body, 163.5 mg/body, 164 mg/body, 164.5 mg/body, 165 mg/body, 165.5 mg/body, 166 mg/body, 166.5 mg/body, 167 mg/body, 167.5 mg/body, 168 mg/body, 168.5 mg/body, 169 mg/body, 169.5 mg/body, 170 mg/body, 170.5 mg/body, 171 mg/body, 171.5 mg/body, 172 mg/body, 172.5 mg/body, 173 mg/body, 173.5 mg/body, 174 mg/body, 174.5 mg/body, 175 mg/body, 175.5 mg/body, 176 mg/body, 176.5 mg/body, 177 mg/body, 177.5 mg/body, 178 mg/body, 178.5 mg/body, 179 mg/body, 179.5 mg/body, 180 mg/body, 180.5 mg/body, 181 mg/body, 181.5 mg/body, 182 mg/body, 182.5 mg/body, 183 mg/body, 183.5 mg/body, 184 mg/body, 184.5 mg/body, 185 mg/body, 185.5 mg/body, 186 mg/body, 186.5 mg/body, 187 mg/body, 187.5 mg/body, 188 mg/body, 188.5 mg/body, 189 mg/body, 189.5 mg/body, 190 mg/body, 190.5 mg/body, 191 mg/body, 191.5 mg/body, 192 mg/body, 192.5 mg/body, 193 mg/body, 193.5 mg/body, 194 mg/body, 194.5 mg/body, 195 mg/body, 195.5 mg/body, 196 mg/body, 196.5 mg/body, 197 mg/body, 197.5 mg/body, 198 mg/body, 198.5 mg/body, 199 mg/body, 199.5 mg/body, and 200 mg/body.

Alternatively, in another non-limiting embodiment, for a subject with or potentially with uremic pruritus, for example, a human adult or child, the IL-31 antagonist of the present disclosure may be administered at one dosage selected from 0.01 mg to 10 mg/kg, for example, 0.05 mg to 7.5 mg/kg, 0.075 mg to 5 mg/kg, or 0.1 mg to 3 mg/kg, and preferably, for example, 0.1 mg to 0.25 mg/kg, 0.1 mg to 0.3 mg/kg, 0.1 mg to 0.5 mg/kg, 0.1 mg to 0.75 mg/kg, 0.1 mg to 1 mg/kg, 0.1 mg to 1.5 mg/kg, 0.1 mg to 2 mg/kg, 0.1 mg to 3 mg/kg, 0.125 mg to 0.25 mg/kg, 0.125 mg to 0.3 mg/kg, 0.125 mg to 0.5 mg/kg, 0.125 mg to 0.75 mg/kg, 0.125 mg to 1 mg/kg, 0.125 mg to 1.5 mg/kg, 0.125 mg to 2 mg/kg, 0.125 mg to 3 mg/kg, 0.2 mg to 0.3 mg/kg, 0.2 mg to 0.5 mg/kg, 0.2 mg to 0.75 mg/kg, 0.2 mg to 1 mg/kg, 0.2 mg to 1.5 mg/kg, 0.2 mg to 2 mg/kg, 0.2 mg to 3 mg/kg, 0.25 mg to 0.3 mg/kg, 0.25 mg to 0.5 mg/kg, 0.25 mg to 0.75 mg/kg, 0.25 mg to 1 mg/kg, 0.25 mg to 1.5 mg/kg, 0.25 mg to 2 mg/kg, 0.25 mg to 3 mg/kg, 0.3 mg to 0.5 mg/kg, 0.3 mg to 0.75 mg/kg, 0.3 mg to 1 mg/kg, 0.3 mg to 1.5 mg/kg, 0.3 mg to 2 mg/kg, 0.3 mg to 3 mg/kg, 0.5 mg to 0.75 mg/kg, 0.5 mg to 1 mg/kg, 0.5 mg to 1.5 mg/kg, 0.5 mg to 2 mg/kg, 0.5 mg to 3 mg/kg, 0.75 mg to 1 mg/kg, 0.75 mg to 1.5 mg/kg, 0.75 mg to 2 mg/kg, 0.75 mg to 3 mg/kg, 1 mg to 1.5 mg/kg, 1 mg to 2 mg/kg, 1 mg to 3 mg/kg, 1.5 mg to 2 mg/kg, 1.5 mg to 3 mg/kg, 2 mg to 3 mg/kg, 0.15 mg to 2.9 mg/kg, 0.2 mg to 2.8 mg/kg, 0.25 mg to 2.7 mg/kg, 0.3 mg to 2.6 mg/kg, 0.35 mg to 2.5 mg/kg, 0.4 mg to 2.4 mg/kg, 0.425 mg to 2.3 mg/kg, 0.45 mg to 2.2 mg/kg, 0.475 mg to 2.1 mg/kg, 0.5 mg to 2 mg/kg, or 0.5 mg to 1.5 mg/kg and at a dosing interval as described above, repeatedly at the same dose and the same dosing interval. For the sake of avoiding any doubt, it is expressly noted that a person skilled in the art who has read, for example, "0.1 mg to 3 mg/kg", will naturally understand that it directly and unambiguously refers to, for example, specific individual values such as 0.1 mg/kg, 0.11 mg/kg, 0.12 mg/kg, 0.125 mg/kg, 0.13 mg/kg, 0.14 mg/kg, 0.15 mg/kg, 0.16 mg/kg, 0.17 mg/kg, 0.18 mg/kg, 0.19 mg/kg, 0.2 mg/kg, 0.21 mg/kg, 0.22 mg/kg, 0.23 mg/kg, 0.24 mg/kg, 0.25 mg/kg, 0.26 mg/kg, 0.27 mg/kg, 0.28 mg/kg, 0.29 mg/kg, 0.3 mg/kg, 0.31 mg/kg, 0.32 mg/kg, 0.33 mg/kg, 0.34 mg/kg, 0.35 mg/kg, 0.36 mg/kg, 0.37 mg/kg, 0.38 mg/kg, 0.39 mg/kg, 0.4 mg/kg, 0.41 mg/kg, 0.42 mg/kg, 0.43 mg/kg, 0.44 mg/kg, 0.45 mg/kg, 0.46 mg/kg, 0.47 mg/kg, 0.48 mg/kg, 0.49 mg/kg, 0.5 mg/kg, 0.51 mg/kg, 0.52 mg/kg, 0.53 mg/kg, 0.54 mg/kg, 0.55 mg/kg, 0.56 mg/kg, 0.57 mg/kg, 0.58 mg/kg, 0.59 mg/kg, 0.6 mg/kg, 0.61 mg/kg, 0.62 mg/kg, 0.63 mg/kg, 0.64 mg/kg, 0.65 mg/kg, 0.66 mg/kg, 0.67 mg/kg, 0.68 mg/kg, 0.69 mg/kg, 0.7 mg/kg, 0.71 mg/kg, 0.72 mg/kg, 0.73 mg/kg, 0.74 mg/kg, 0.75 mg/kg, 0.76 mg/kg, 0.77 mg/kg, 0.78 mg/kg, 0.79 mg/kg, 0.8 mg/kg, 0.81 mg/kg, 0.82 mg/kg, 0.83 mg/kg, 0.84 mg/kg, 0.85 mg/kg, 0.86 mg/kg, 0.87 mg/kg, 0.88 mg/kg, 0.89 mg/kg, 0.9 mg/kg, 0.91 mg/kg, 0.92 mg/kg, 0.93 mg/kg, 0.94 mg/kg, 0.95 mg/kg, 0.96 mg/kg, 0.97 mg/kg, 0.98 mg/kg, 0.99 mg/kg, 1 mg/kg, 1.01 mg/kg, 1.02 mg/kg, 1.03 mg/kg, 1.04 mg/kg, 1.05 mg/kg, 1.06 mg/kg, 1.07 mg/kg, 1.08 mg/kg, 1.09 mg/kg, 1.1 mg/kg, 1.11 mg/kg, 1.12 mg/kg, 1.13 mg/kg, 1.14 mg/kg, 1.15 mg/kg, 1.16 mg/kg, 1.17 mg/kg, 1.18 mg/kg, 1.19 mg/kg, 1.2 mg/kg, 1.21 mg/kg, 1.22 mg/kg, 1.23 mg/kg, 1.24 mg/kg, 1.25 mg/kg, 1.26 mg/kg, 1.27 mg/kg, 1.28 mg/kg, 1.29 mg/kg, 1.3 mg/kg, 1.31 mg/kg, 1.32 mg/kg, 1.33 mg/kg, 1.34 mg/kg, 1.35 mg/kg, 1.36 mg/kg, 1.37 mg/kg, 1.38 mg/kg, 1.39 mg/kg, 1.4 mg/kg, 1.41 mg/kg, 1.42 mg/kg, 1.43 mg/kg, 1.44 mg/kg, 1.45 mg/kg, 1.46 mg/kg, 1.47 mg/kg, 1.48 mg/kg, 1.49 mg/kg, 1.5 mg/kg, 1.51 mg/kg, 1.52 mg/kg, 1.53 mg/kg, 1.54 mg/kg, 1.55 mg/kg, 1.56 mg/kg, 1.57 mg/kg, 1.58 mg/kg, 1.59 mg/kg, 1.6 mg/kg, 1.61 mg/kg, 1.62 mg/kg, 1.63 mg/kg, 1.64 mg/kg, 1.65 mg/kg, 1.66 mg/kg, 1.67 mg/kg, 1.68 mg/kg, 1.69 mg/kg, 1.7 mg/kg, 1.71 mg/kg, 1.72 mg/kg, 1.73 mg/kg, 1.74 mg/kg, 1.75 mg/kg, 1.76 mg/kg, 1.77 mg/kg, 1.78 mg/kg, 1.79 mg/kg, 1.8 mg/kg, 1.81 mg/kg, 1.82 mg/kg, 1.83 mg/kg, 1.84 mg/kg, 1.85 mg/kg, 1.86 mg/kg, 1.87 mg/kg, 1.88 mg/kg, 1.89 mg/kg, 1.9 mg/kg, 1.91 mg/kg, 1.92 mg/kg, 1.93 mg/kg, 1.94 mg/kg, 1.95 mg/kg, 1.96 mg/kg, 1.97 mg/kg, 1.98 mg/kg, 1.99 mg/kg, 2 mg/kg, 2.01 mg/kg, 2.02 mg/kg, 2.03 mg/kg, 2.04 mg/kg, 2.05 mg/kg, 2.06 mg/kg, 2.07 mg/kg, 2.08 mg/kg, 2.09 mg/kg, 2.1 mg/kg, 2.11 mg/kg, 2.12 mg/kg, 2.13 mg/kg, 2.14 mg/kg, 2.15 mg/kg, 2.16 mg/kg, 2.17 mg/kg, 2.18 mg/kg, 2.19 mg/kg, 2.2 mg/kg, 2.21 mg/kg, 2.22 mg/kg, 2.23 mg/kg, 2.24 mg/kg, 2.25 mg/kg, 2.26 mg/kg, 2.27 mg/kg, 2.28 mg/kg, 2.29 mg/kg, 2.3 mg/kg, 2.31 mg/kg, 2.32 mg/kg, 2.33 mg/kg, 2.34 mg/kg, 2.35 mg/kg, 2.36 mg/kg, 2.37 mg/kg, 2.38 mg/kg, 2.39 mg/kg, 2.4 mg/kg, 2.41 mg/kg, 2.42 mg/kg, 2.43 mg/kg, 2.44 mg/kg, 2.45 mg/kg, 2.46 mg/kg, 2.47 mg/kg, 2.48 mg/kg, 2.49 mg/kg, 2.5 mg/kg, 2.51 mg/kg, 2.52 mg/kg, 2.53 mg/kg, 2.54 mg/kg, 2.55 mg/kg, 2.56 mg/kg, 2.57 mg/kg, 2.58 mg/kg, 2.59 mg/kg, 2.6 mg/kg, 2.61 mg/kg, 2.62 mg/kg, 2.63 mg/kg, 2.64 mg/kg, 2.65 mg/kg, 2.66 mg/kg, 2.67 mg/kg, 2.68 mg/kg, 2.69 mg/kg, 2.7 mg/kg, 2.71 mg/kg, 2.72 mg/kg, 2.73 mg/kg, 2.74 mg/kg, 2.75 mg/kg, 2.76 mg/kg, 2.77 mg/kg, 2.78 mg/kg, 2.79 mg/kg, 2.8 mg/kg, 2.81 mg/kg, 2.82 mg/kg, 2.83 mg/kg, 2.84 mg/kg, 2.85 mg/kg, 2.86 mg/kg, 2.87 mg/kg, 2.88 mg/kg, 2.89 mg/kg, 2.9 mg/kg, 2.91 mg/kg, 2.92 mg/kg, 2.93 mg/kg, 2.94 mg/kg, 2.95 mg/kg, 2.96 mg/kg, 2.97 mg/kg, 2.98 mg/kg, 2.99 mg/kg, and 3 mg/kg.

As described above, in an embodiment where the IL-31 antagonist of the present disclosure is administered at a predetermined dosing interval and a predetermined dose (dosage) repeatedly at the same dose and the same dosing interval, the IL-31 antagonist of the present disclosure may be administered at "0.1 mg to 1000 mg/body/1 day to 12 weeks". Herein, for example, "0.1 mg to 1000 mg/body/1 day to 12 weeks" is contemplated to mean that one dosage is selected from 0.1 mg to 1000 mg as the dosage (e.g., 100 mg/body) of the IL-31 antagonist of the present disclosure, and any one dosing interval is selected from 1 day to 12 weeks as the dosing interval (e.g., 4 weeks) of the IL-31 antagonist of the present disclosure, and the IL-31 antagonist is administered to a subject repeatedly at the same dosage and the same dosing interval. As an example, "100 mg/body/4 weeks" is contemplated to mean that 100 mg/body of the IL-31 antagonist of the present disclosure is administered to a subject every 4 weeks repeatedly at the same dosage and the same dosing interval. In an embodiment where the IL-31 antagonist of the present disclosure is administered at a predetermined dosing interval and a predetermined dose (dosage) repeatedly at the same dose the same dosing interval, the IL-31 antagonist of the present disclosure is preferably administered at 0.1 mg to 1000 mg/body/2 to 8 weeks, and may be administered at, for example, 0.1 mg to 1000 mg/body/2 weeks, 0.1 mg to 1000 mg/body/4 weeks, 0.1 mg to 1000 mg/body/6 weeks, or 0.1 mg to 1000 mg/body/8 weeks, but not limited thereto. Alternatively, the IL-31 antagonist of the present disclosure is more preferably administered at 0.2 mg to 360 mg/body/2 to 8 weeks, and may be administered at, for example, 0.2 mg to 360 mg/body/2 weeks, 0.2 mg to 360 mg/body/4 weeks, 0.2 mg to 360 mg/body/6 weeks, or 0.2 mg to 360 mg/body/8 weeks. Alternatively, as an example, the IL-31 antagonist of the present disclosure is still more preferably administered at 10 mg to 200 mg/body/2 to 8 weeks, and may be administered at, for example, 10 mg to 200 mg/body/2 weeks, 10 mg to 200 mg/body/4 weeks, 10 mg to 200 mg/body/6 weeks, or 10 mg to 200 mg/body/8 weeks. Alternatively, as an example, the IL-31 antagonist of the present disclosure is even more preferably administered at 10 mg to 100 mg/body/2 to 8 weeks, and may be administered at, for example, 10 mg to 100 mg/body/2 weeks, 10 mg to 100 mg/body/4 weeks, 10 mg to 100 mg/body/6 weeks, or 10 mg to 100 mg/body/8 weeks. Alternatively, as an example, the IL-31 antagonist of the present disclosure may be administered at 25 mg to 100 mg/body/4 weeks, 25 mg to 80 mg/body/4 weeks, 25 mg to 75 mg/body/4 weeks, 50 mg to 100 mg/body/4 weeks, 50 mg to 80 mg/body/4 weeks, or 50 mg to 75 mg/body/4 weeks, or at 10 mg to 50 mg/body/2 weeks or 20 mg to 40 mg/body/2 weeks. In a non-limiting embodiment, the IL-31 antagonist of the present disclosure may be administered at, for example, 5 mg/body/4 weeks, 10 mg mg/body/4 weeks, 15 mg/body/4 weeks, 20 mg/body/4 weeks, 25 mg/body/4 weeks, 30 mg/body/4 weeks, 50 mg/body/4 weeks, 50.5 mg/body/4 weeks, 51 mg/body/4 weeks, 51.5 mg/body/4 weeks, 52 mg/body/4 weeks, 52.5 mg/body/4 weeks, 53 mg/body/4 weeks, 53.5 mg/body/4 weeks, 54 mg/body/4 weeks, 54.5 mg/body/4 weeks, 55 mg/body/4 weeks, 55.5 mg/body/4 weeks, 56 mg/body/4 weeks, 56.5 mg/body/4 weeks, 57 mg/body/4 weeks, 57.5 mg/body/4 weeks, 58 mg/body/4 weeks, 58.5 mg/body/4 weeks, 59 mg/body/4 weeks, 59.5 mg/body/4 weeks, 60 mg/body/4 weeks, 60.5 mg/body/4 weeks, 61 mg/body/4 weeks, 61.5 mg/body/4 weeks, 62 mg/body/4 weeks, 62.5 mg/body/4 weeks, 63 mg/body/4 weeks, 63.5 mg/body/4 weeks, 64 mg/body/4 weeks, 64.5 mg/body/4 weeks, 65 mg/body/4 weeks, 65.5 mg/body/4 weeks, 66 mg/body/4 weeks, 66.5 mg/body/4 weeks, 67 mg/body/4 weeks, 67.5 mg/body/4 weeks, 68 mg/body/4 weeks, 68.5 mg/body/4 weeks, 69 mg/body/4 weeks, 69.5 mg/body/4 weeks, 70 mg/body/4 weeks, 70.5 mg/body/4 weeks, 71 mg/body/4 weeks, 71.5 mg/body/4 weeks, 72 mg/body/4 weeks, 72.5 mg/body/4 weeks, 73 mg/body/4 weeks, 73.5 mg/body/4 weeks, 74 mg/body/4 weeks, 74.5 mg/body/4 weeks, 75 mg/body/4 weeks, 75.5 mg/body/4 weeks, 76 mg/body/4 weeks, 76.5 mg/body/4 weeks, 77 mg/body/4 weeks, 77.5 mg/body/4 weeks, 78 mg/body/4 weeks, 78.5 mg/body/4 weeks, 79 mg/body/4 weeks, 79.5 mg/body/4 weeks, 80 mg/body/4 weeks, 80.5 mg/body/4 weeks, 81 mg/body/4 weeks, 81.5 mg/body/4 weeks, 82 mg/body/4 weeks, 82.5 mg/body/4 weeks, 83 mg/body/4 weeks, 83.5 mg/body/4 weeks, 84 mg/body/4 weeks, 84.5 mg/body/4 weeks, 85 mg/body/4 weeks, 85.5 mg/body/4 weeks, 86 mg/body/4 weeks, 86.5 mg/body/4 weeks, 87 mg/body/4 weeks, 87.5 mg/body/4 weeks, 88 mg/body/4 weeks, 88.5 mg/body/4 weeks, 89 mg/body/4 weeks, 89.5 mg/body/4 weeks, 90 mg/body/4 weeks, 90.5 mg/body/4 weeks, 91 mg/body/4 weeks, 91.5 mg/body/4 weeks, 92 mg/body/4 weeks, 92.5 mg/body/4 weeks, 93 mg/body/4 weeks, 93.5 mg/body/4 weeks, 94 mg/body/4 weeks, 94.5 mg/body/4 weeks, 95 mg/body/4 weeks, 95.5 mg/body/4 weeks, 96 mg/body/4 weeks, 96.5 mg/body/4 weeks, 97 mg/body/4 weeks, 97.5 mg/body/4 weeks, 98 mg/body/4 weeks, 98.5 mg/body/4 weeks, 99 mg/body/4 weeks, 99.5 mg/body/4 weeks, 100 mg/body/4 weeks, 100.5 mg/body/4 weeks, 101 mg/body/4 weeks, 101.5 mg/body/4 weeks, 102 mg/body/4 weeks, 102.5 mg/body/4 weeks, 103 mg/body/4 weeks, 103.5 mg/body/4 weeks, 104 mg/body/4 weeks, 104.5 mg/body/4 weeks, 105 mg/body/4 weeks, 105.5 mg/body/4 weeks, 106 mg/body/4 weeks, 106.5 mg/body/4 weeks, 107 mg/body/4 weeks, 107.5 mg/body/4 weeks, 108 mg/body/4 weeks, 108.5 mg/body/4 weeks, 109 mg/body/4 weeks, 109.5 mg/body/4 weeks, 110 mg/body/4 weeks, 110.5 mg/body/4 weeks, 111 mg/body/4 weeks, 111.5 mg/body/4 weeks, 112 mg/body/4 weeks, 112.5 mg/body/4 weeks, 113 mg/body/4 weeks, 113.5 mg/body/4 weeks, 114 mg/body/4 weeks, 114.5 mg/body/4 weeks, 115 mg/body/4 weeks, 115.5 mg/body/4 weeks, 116 mg/body/4 weeks, 116.5 mg/body/4 weeks, 117 mg/body/4 weeks, 117.5 mg/body/4 weeks, 118 mg/body/4 weeks, 118.5 mg/body/4 weeks, 119 mg/body/4 weeks, 119.5 mg/body/4 weeks, 120 mg/body/4 weeks, 120.5 mg/body/4 weeks, 121 mg/body/4 weeks, 121.5 mg/body/4 weeks, 122 mg/body/4 weeks, 122.5 mg/body/4 weeks, 123 mg/body/4 weeks, 123.5 mg/body/4 weeks, 124 mg/body/4 weeks, 124.5 mg/body/4 weeks, 125 mg/body/4 weeks, 125.5 mg/body/4 weeks, 126 mg/body/4 weeks, 126.5 mg/body/4 weeks, 127 mg/body/4 weeks, 127.5 mg/body/4 weeks, 128 mg/body/4 weeks, 128.5 mg/body/4 weeks, 129 mg/body/4 weeks, 129.5 mg/body/4 weeks, 130 mg/body/4 weeks, 130.5 mg/body/4 weeks, 131 mg/body/4 weeks, 131.5 mg/body/4 weeks, 132 mg/body/4 weeks, 132.5 mg/body/4 weeks, 133 mg/body/4 weeks, 133.5 mg/body/4 weeks, 134 mg/body/4 weeks, 134.5 mg/body/4 weeks, 135 mg/body/4 weeks, 135.5 mg/body/4 weeks, 136 mg/body/4 weeks, 136.5 mg/body/4 weeks, 137 mg/body/4 weeks, 137.5 mg/body/4 weeks, 138 mg/body/4 weeks, 138.5 mg/body/4 weeks, 139 mg/body/4 weeks, 139.5 mg/body/4 weeks, 140 mg/body/4 weeks, 140.5 mg/body/4 weeks, 141 mg/body/4 weeks, 141.5 mg/body/4 weeks, 142 mg/body/4 weeks, 142.5 mg/body/4 weeks, 143 mg/body/4 weeks, 143.5 mg/body/4 weeks, 144 mg/body/4 weeks, 144.5 mg/body/4 weeks, 145 mg/body/4 weeks, 145.5 mg/body/4 weeks, 146 mg/body/4 weeks, 146.5 mg/body/4 weeks, 147 mg/body/4 weeks, 147.5 mg/body/4 weeks, 148 mg/body/4 weeks, 148.5 mg/body/4 weeks, 149 mg/body/4 weeks, 149.5 mg/body/4 weeks, 150 mg/body/4 weeks, 150.5 mg/body/4 weeks, 151 mg/body/4 weeks, 151.5 mg/body/4 weeks, 152 mg/body/4 weeks, 152.5 mg/body/4 weeks, 153 mg/body/4 weeks, 153.5 mg/body/4 weeks, 154 mg/body/4 weeks, 154.5 mg/body/4 weeks, 155 mg/body/4 weeks, 155.5 mg/body/4 weeks, 156 mg/body/4 weeks, 156.5 mg/body/4 weeks, 157 mg/body/4 weeks, 157.5 mg/body/4 weeks, 158 mg/body/4 weeks, 158.5 mg/body/4 weeks, 159 mg/body/4 weeks, 159.5 mg/body/4 weeks, 160 mg/body/4 weeks, 160.5 mg/body/4 weeks, 161 mg/body/4 weeks, 161.5 mg/body/4 weeks, 162 mg/body/4 weeks, 162.5 mg/body/4 weeks, 163 mg/body/4 weeks, 163.5 mg/body/4 weeks, 164 mg/body/4 weeks, 164.5 mg/body/4 weeks, 165 mg/body/4 weeks, 165.5 mg/body/4 weeks, 166 mg/body/4 weeks, 166.5 mg/body/4 weeks, 167 mg/body/4 weeks, 167.5 mg/body/4 weeks, 168 mg/body/4 weeks, 168.5 mg/body/4 weeks, 169 mg/body/4 weeks, 169.5 mg/body/4 weeks, 170 mg/body/4 weeks, 170.5 mg/body/4 weeks, 171 mg/body/4 weeks, 171.5 mg/body/4 weeks, 172 mg/body/4 weeks, 172.5 mg/body/4 weeks, 173 mg/body/4 weeks, 173.5 mg/body/4 weeks, 174 mg/body/4 weeks, 174.5 mg/body/4 weeks, 175 mg/body/4 weeks, 175.5 mg/body/4 weeks, 176 mg/body/4 weeks, 176.5 mg/body/4 weeks, 177 mg/body/4 weeks, 177.5 mg/body/4 weeks, 178 mg/body/4 weeks, 178.5 mg/body/4 weeks, 179 mg/body/4 weeks, 179.5 mg/body/4 weeks, 180 mg/body/4 weeks, 180.5 mg/body/4 weeks, 181 mg/body/4 weeks, 181.5 mg/body/4 weeks, 182 mg/body/4 weeks, 182.5 mg/body/4 weeks, 183 mg/body/4 weeks, 183.5 mg/body/4 weeks, 184 mg/body/4 weeks, 184.5 mg/body/4 weeks, 185 mg/body/4 weeks, 185.5 mg/body/4 weeks, 186 mg/body/4 weeks, 186.5 mg/body/4 weeks, 187 mg/body/4 weeks, 187.5 mg/body/4 weeks, 188 mg/body/4 weeks, 188.5 mg/body/4 weeks, 189 mg/body/4 weeks, 189.5 mg/body/4 weeks, 190 mg/body/4 weeks, 190.5 mg/body/4 weeks, 191 mg/body/4 weeks, 191.5 mg/body/4 weeks, 192 mg/body/4 weeks, 192.5 mg/body/4 weeks, 193 mg/body/4 weeks, 193.5 mg/body/4 weeks, 194 mg/body/4 weeks, 194.5 mg/body/4 weeks, 195 mg/body/4 weeks, 195.5 mg/body/4 weeks, 196 mg/body/4 weeks, 196.5 mg/body/4 weeks, 197 mg/body/4 weeks, 197.5 mg/body/4 weeks, 198 mg/body/4 weeks, 198.5 mg/body/4 weeks, 199 mg/body/4 weeks, 199.5 mg/body/4 weeks, or 200 mg/body/4 weeks. Alternatively, in a non-limiting embodiment, the IL-31 antagonist of the present disclosure may be administered at, for example, 50 mg/body/6 weeks, 50.5 mg/body/6 weeks, 51 mg/body/6 weeks, 51.5 mg/body/6 weeks, 52 mg/body/6 weeks, 52.5 mg/body/6 weeks, 53 mg/body/6 weeks, 53.5 mg/body/6 weeks, 54 mg/body/6 weeks, 54.5 mg/body/6 weeks, 55 mg/body/6 weeks, 55.5 mg/body/6 weeks, 56 mg/body/6 weeks, 56.5 mg/body/6 weeks, 57 mg/body/6 weeks, 57.5 mg/body/6 weeks, 58 mg/body/6 weeks, 58.5 mg/body/6 weeks, 59 mg/body/6 weeks, 59.5 mg/body/6 weeks, 60 mg/body/6 weeks, 60.5 mg/body/6 weeks, 61 mg/body/6 weeks, 61.5 mg/body/6 weeks, 62 mg/body/6 weeks, 62.5 mg/body/6 weeks, 63 mg/body/6 weeks, 63.5 mg/body/6 weeks, 64 mg/body/6 weeks, 64.5 mg/body/6 weeks, 65 mg/body/6 weeks, 65.5 mg/body/6 weeks, 66 mg/body/6 weeks, 66.5 mg/body/6 weeks, 67 mg/body/6 weeks, 67.5 mg/body/6 weeks, 68 mg/body/6 weeks, 68.5 mg/body/6 weeks, 69 mg/body/6 weeks, 69.5 mg/body/6 weeks, 70 mg/body/6 weeks, 70.5 mg/body/6 weeks, 71 mg/body/6 weeks, 71.5 mg/body/6 weeks, 72 mg/body/6 weeks, 72.5 mg/body/6 weeks, 73 mg/body/6 weeks, 73.5 mg/body/6 weeks, 74 mg/body/6 weeks, 74.5 mg/body/6 weeks, 75 mg/body/6 weeks, 75.5 mg/body/6 weeks, 76 mg/body/6 weeks, 76.5 mg/body/6 weeks, 77 mg/body/6 weeks, 77.5 mg/body/6 weeks, 78 mg/body/6 weeks, 78.5 mg/body/6 weeks, 79 mg/body/6 weeks, 79.5 mg/body/6 weeks, 80 mg/body/6 weeks, 80.5 mg/body/6 weeks, 81 mg/body/6 weeks, 81.5 mg/body/6 weeks, 82 mg/body/6 weeks, 82.5 mg/body/6 weeks, 83 mg/body/6 weeks, 83.5 mg/body/6 weeks, 84 mg/body/6 weeks, 84.5 mg/body/6 weeks, 85 mg/body/6 weeks, 85.5 mg/body/6 weeks, 86 mg/body/6 weeks, 86.5 mg/body/6 weeks, 87 mg/body/6 weeks, 87.5 mg/body/6 weeks, 88 mg/body/6 weeks, 88.5 mg/body/6 weeks, 89 mg/body/6 weeks, 89.5 mg/body/6 weeks, 90 mg/body/6 weeks, 90.5 mg/body/6 weeks, 91 mg/body/6 weeks, 91.5 mg/body/6 weeks, 92 mg/body/6 weeks, 92.5 mg/body/6 weeks, 93 mg/body/6 weeks, 93.5 mg/body/6 weeks, 94 mg/body/6 weeks, 94.5 mg/body/6 weeks, 95 mg/body/6 weeks, 95.5 mg/body/6 weeks, 96 mg/body/6 weeks, 96.5 mg/body/6 weeks, 97 mg/body/6 weeks, 97.5 mg/body/6 weeks, 98 mg/body/6 weeks, 98.5 mg/body/6 weeks, 99 mg/body/6 weeks, 99.5 mg/body/6 weeks, 100 mg/body/6 weeks, 100.5 mg/body/6 weeks, 101 mg/body/6 weeks, 101.5 mg/body/6 weeks, 102 mg/body/6 weeks, 102.5 mg/body/6 weeks, 103 mg/body/6 weeks, 103.5 mg/body/6 weeks, 104 mg/body/6 weeks, 104.5 mg/body/6 weeks, 105 mg/body/6 weeks, 105.5 mg/body/6 weeks, 106 mg/body/6 weeks, 106.5 mg/body/6 weeks, 107 mg/body/6 weeks, 107.5 mg/body/6 weeks, 108 mg/body/6 weeks, 108.5 mg/body/6 weeks, 109 mg/body/6 weeks, 109.5 mg/body/6 weeks, 110 mg/body/6 weeks, 110.5 mg/body/6 weeks, 111 mg/body/6 weeks, 111.5 mg/body/6 weeks, 112 mg/body/6 weeks, 112.5 mg/body/6 weeks, 113 mg/body/6 weeks, 113.5 mg/body/6 weeks, 114 mg/body/6 weeks, 114.5 mg/body/6 weeks, 115 mg/body/6 weeks, 115.5 mg/body/6 weeks, 116 mg/body/6 weeks, 116.5 mg/body/6 weeks, 117 mg/body/6 weeks, 117.5 mg/body/6 weeks, 118 mg/body/6 weeks, 118.5 mg/body/6 weeks, 119 mg/body/6 weeks, 119.5 mg/body/6 weeks, 120 mg/body/6 weeks, 120.5 mg/body/6 weeks, 121 mg/body/6 weeks, 121.5 mg/body/6 weeks, 122 mg/body/6 weeks, 122.5 mg/body/6 weeks, 123 mg/body/6 weeks, 123.5 mg/body/6 weeks, 124 mg/body/6 weeks, 124.5 mg/body/6 weeks, 125 mg/body/6 weeks, 125.5 mg/body/6 weeks, 126 mg/body/6 weeks, 126.5 mg/body/6 weeks, 127 mg/body/6 weeks, 127.5 mg/body/6 weeks, 128 mg/body/6 weeks, 128.5 mg/body/6 weeks, 129 mg/body/6 weeks, 129.5 mg/body/6 weeks, 130 mg/body/6 weeks, 130.5 mg/body/6 weeks, 131 mg/body/6 weeks, 131.5 mg/body/6 weeks, 132 mg/body/6 weeks, 132.5 mg/body/6 weeks, 133 mg/body/6 weeks, 133.5 mg/body/6 weeks, 134 mg/body/6 weeks, 134.5 mg/body/6 weeks, 135 mg/body/6 weeks, 135.5 mg/body/6 weeks, 136 mg/body/6 weeks, 136.5 mg/body/6 weeks, 137 mg/body/6 weeks, 137.5 mg/body/6 weeks, 138 mg/body/6 weeks, 138.5 mg/body/6 weeks, 139 mg/body/6 weeks, 139.5 mg/body/6 weeks, 140 mg/body/6 weeks, 140.5 mg/body/6 weeks, 141 mg/body/6 weeks, 141.5 mg/body/6 weeks, 142 mg/body/6 weeks, 142.5 mg/body/6 weeks, 143 mg/body/6 weeks, 143.5 mg/body/6 weeks, 144 mg/body/6 weeks, 144.5 mg/body/6 weeks, 145 mg/body/6 weeks, 145.5 mg/body/6 weeks, 146 mg/body/6 weeks, 146.5 mg/body/6 weeks, 147 mg/body/6 weeks, 147.5 mg/body/6 weeks, 148 mg/body/6 weeks, 148.5 mg/body/6 weeks, 149 mg/body/6 weeks, 149.5 mg/body/6 weeks, 150 mg/body/6 weeks, 150.5 mg/body/6 weeks, 151 mg/body/6 weeks, 151.5 mg/body/6 weeks, 152 mg/body/6 weeks, 152.5 mg/body/6 weeks, 153 mg/body/6 weeks, 153.5 mg/body/6 weeks, 154 mg/body/6 weeks, 154.5 mg/body/6 weeks, 155 mg/body/6 weeks, 155.5 mg/body/6 weeks, 156 mg/body/6 weeks, 156.5 mg/body/6 weeks, 157 mg/body/6 weeks, 157.5 mg/body/6 weeks, 158 mg/body/6 weeks, 158.5 mg/body/6 weeks, 159 mg/body/6 weeks, 159.5 mg/body/6 weeks, 160 mg/body/6 weeks, 160.5 mg/body/6 weeks, 161 mg/body/6 weeks, 161.5 mg/body/6 weeks, 162 mg/body/6 weeks, 162.5 mg/body/6 weeks, 163 mg/body/6 weeks, 163.5 mg/body/6 weeks, 164 mg/body/6 weeks, 164.5 mg/body/6 weeks, 165 mg/body/6 weeks, 165.5 mg/body/6 weeks, 166 mg/body/6 weeks, 166.5 mg/body/6 weeks, 167 mg/body/6 weeks, 167.5 mg/body/6 weeks, 168 mg/body/6 weeks, 168.5 mg/body/6 weeks, 169 mg/body/6 weeks, 169.5 mg/body/6 weeks, 170 mg/body/6 weeks, 170.5 mg/body/6 weeks, 171 mg/body/6 weeks, 171.5 mg/body/6 weeks, 172 mg/body/6 weeks, 172.5 mg/body/6 weeks, 173 mg/body/6 weeks, 173.5 mg/body/6 weeks, 174 mg/body/6 weeks, 174.5 mg/body/6 weeks, 175 mg/body/6 weeks, 175.5 mg/body/6 weeks, 176 mg/body/6 weeks, 176.5 mg/body/6 weeks, 177 mg/body/6 weeks, 177.5 mg/body/6 weeks, 178 mg/body/6 weeks, 178.5 mg/body/6 weeks, 179 mg/body/6 weeks, 179.5 mg/body/6 weeks, 180 mg/body/6 weeks, 180.5 mg/body/6 weeks, 181 mg/body/6 weeks, 181.5 mg/body/6 weeks, 182 mg/body/6 weeks, 182.5 mg/body/6 weeks, 183 mg/body/6 weeks, 183.5 mg/body/6 weeks, 184 mg/body/6 weeks, 184.5 mg/body/6 weeks, 185 mg/body/6 weeks, 185.5 mg/body/6 weeks, 186 mg/body/6 weeks, 186.5 mg/body/6 weeks, 187 mg/body/6 weeks, 187.5 mg/body/6 weeks, 188 mg/body/6 weeks, 188.5 mg/body/6 weeks, 189 mg/body/6 weeks, 189.5 mg/body/6 weeks, 190 mg/body/6 weeks, 190.5 mg/body/6 weeks, 191 mg/body/6 weeks, 191.5 mg/body/6 weeks, 192 mg/body/6 weeks, 192.5 mg/body/6 weeks, 193 mg/body/6 weeks, 193.5 mg/body/6 weeks, 194 mg/body/6 weeks, 194.5 mg/body/6 weeks, 195 mg/body/6 weeks, 195.5 mg/body/6 weeks, 196 mg/body/6 weeks, 196.5 mg/body/6 weeks, 197 mg/body/6 weeks, 197.5 mg/body/6 weeks, 198 mg/body/6 weeks, 198.5 mg/body/6 weeks, 199 mg/body/6 weeks, 199.5 mg/body/6 weeks, or 200 mg/body/6 weeks. Alternatively, in a non-limiting embodiment, the IL-31 antagonist of the present disclosure may be administered at, for example, 50 mg/body/8 weeks, 50.5 mg/body/8 weeks, 51 mg/body/8 weeks, 51.5 mg/body/8 weeks, 52 mg/body/8 weeks, 52.5 mg/body/8 weeks, 53 mg/body/8 weeks, 53.5 mg/body/8 weeks, 54 mg/body/8 weeks, 54.5 mg/body/8 weeks, 55 mg/body/8 weeks, 55.5 mg/body/8 weeks, 56 mg/body/8 weeks, 56.5 mg/body/8 weeks, 57 mg/body/8 weeks, 57.5 mg/body/8 weeks, 58 mg/body/8 weeks, 58.5 mg/body/8 weeks, 59 mg/body/8 weeks, 59.5 mg/body/8 weeks, 60 mg/body/8 weeks, 60.5 mg/body/8 weeks, 61 mg/body/8 weeks, 61.5 mg/body/8 weeks, 62 mg/body/8 weeks, 62.5 mg/body/8 weeks, 63 mg/body/8 weeks, 63.5 mg/body/8 weeks, 64 mg/body/8 weeks, 64.5 mg/body/8 weeks, 65 mg/body/8 weeks, 65.5 mg/body/8 weeks, 66 mg/body/8 weeks, 66.5 mg/body/8 weeks, 67 mg/body/8 weeks, 67.5 mg/body/8 weeks, 68 mg/body/8 weeks, 68.5 mg/body/8 weeks, 69 mg/body/8 weeks, 69.5 mg/body/8 weeks, 70 mg/body/8 weeks, 70.5 mg/body/8 weeks, 71 mg/body/8 weeks, 71.5 mg/body/8 weeks, 72 mg/body/8 weeks, 72.5 mg/body/8 weeks, 73 mg/body/8 weeks, 73.5 mg/body/8 weeks, 74 mg/body/8 weeks, 74.5 mg/body/8 weeks, 75 mg/body/8 weeks, 75.5 mg/body/8 weeks, 76 mg/body/8 weeks, 76.5 mg/body/8 weeks, 77 mg/body/8 weeks, 77.5 mg/body/8 weeks, 78 mg/body/8 weeks, 78.5 mg/body/8 weeks, 79 mg/body/8 weeks, 79.5 mg/body/8 weeks, 80 mg/body/8 weeks, 80.5 mg/body/8 weeks, 81 mg/body/8 weeks, 81.5 mg/body/8 weeks, 82 mg/body/8 weeks, 82.5 mg/body/8 weeks, 83 mg/body/8 weeks, 83.5 mg/body/8 weeks, 84 mg/body/8 weeks, 84.5 mg/body/8 weeks, 85 mg/body/8 weeks, 85.5 mg/body/8 weeks, 86 mg/body/8 weeks, 86.5 mg/body/8 weeks, 87 mg/body/8 weeks, 87.5 mg/body/8 weeks, 88 mg/body/8 weeks, 88.5 mg/body/8 weeks, 89 mg/body/8 weeks, 89.5 mg/body/8 weeks, 90 mg/body/8 weeks, 90.5 mg/body/8 weeks, 91 mg/body/8 weeks, 91.5 mg/body/8 weeks, 92 mg/body/8 weeks, 92.5 mg/body/8 weeks, 93 mg/body/8 weeks, 93.5 mg/body/8 weeks, 94 mg/body/8 weeks, 94.5 mg/body/8 weeks, 95 mg/body/8 weeks, 95.5 mg/body/8 weeks, 96 mg/body/8 weeks, 96.5 mg/body/8 weeks, 97 mg/body/8 weeks, 97.5 mg/body/8 weeks, 98 mg/body/8 weeks, 98.5 mg/body/8 weeks, 99 mg/body/8 weeks, 99.5 mg/body/8 weeks, 100 mg/body/8 weeks, 100.5 mg/body/8 weeks, 101 mg/body/8 weeks, 101.5 mg/body/8 weeks, 102 mg/body/8 weeks, 102.5 mg/body/8 weeks, 103 mg/body/8 weeks, 103.5 mg/body/8 weeks, 104 mg/body/8 weeks, 104.5 mg/body/8 weeks, 105 mg/body/8 weeks, 105.5 mg/body/8 weeks, 106 mg/body/8 weeks, 106.5 mg/body/8 weeks, 107 mg/body/8 weeks, 107.5 mg/body/8 weeks, 108 mg/body/8 weeks, 108.5 mg/body/8 weeks, 109 mg/body/8 weeks, 109.5 mg/body/8 weeks, 110 mg/body/8 weeks, 110.5 mg/body/8 weeks, 111 mg/body/8 weeks, 111.5 mg/body/8 weeks, 112 mg/body/8 weeks, 112.5 mg/body/8 weeks, 113 mg/body/8 weeks, 113.5 mg/body/8 weeks, 114 mg/body/8 weeks, 114.5 mg/body/8 weeks, 115 mg/body/8 weeks, 115.5 mg/body/8 weeks, 116 mg/body/8 weeks, 116.5 mg/body/8 weeks, 117 mg/body/8 weeks, 117.5 mg/body/8 weeks, 118 mg/body/8 weeks, 118.5 mg/body/8 weeks, 119 mg/body/8 weeks, 119.5 mg/body/8 weeks, 120 mg/body/8 weeks, 120.5 mg/body/8 weeks, 121 mg/body/8 weeks, 121.5 mg/body/8 weeks, 122 mg/body/8 weeks, 122.5 mg/body/8 weeks, 123 mg/body/8 weeks, 123.5 mg/body/8 weeks, 124 mg/body/8 weeks, 124.5 mg/body/8 weeks, 125 mg/body/8 weeks, 125.5 mg/body/8 weeks, 126 mg/body/8 weeks, 126.5 mg/body/8 weeks, 127 mg/body/8 weeks, 127.5 mg/body/8 weeks, 128 mg/body/8 weeks, 128.5 mg/body/8 weeks, 129 mg/body/8 weeks, 129.5 mg/body/8 weeks, 130 mg/body/8 weeks, 130.5 mg/body/8 weeks, 131 mg/body/8 weeks, 131.5 mg/body/8 weeks, 132 mg/body/8 weeks, 132.5 mg/body/8 weeks, 133 mg/body/8 weeks, 133.5 mg/body/8 weeks, 134 mg/body/8 weeks, 134.5 mg/body/8 weeks, 135 mg/body/8 weeks, 135.5 mg/body/8 weeks, 136 mg/body/8 weeks, 136.5 mg/body/8 weeks, 137 mg/body/8 weeks, 137.5 mg/body/8 weeks, 138 mg/body/8 weeks, 138.5 mg/body/8 weeks, 139 mg/body/8 weeks, 139.5 mg/body/8 weeks, 140 mg/body/8 weeks, 140.5 mg/body/8 weeks, 141 mg/body/8 weeks, 141.5 mg/body/8 weeks, 142 mg/body/8 weeks, 142.5 mg/body/8 weeks, 143 mg/body/8 weeks, 143.5 mg/body/8 weeks, 144 mg/body/8 weeks, 144.5 mg/body/8 weeks, 145 mg/body/8 weeks, 145.5 mg/body/8 weeks, 146 mg/body/8 weeks, 146.5 mg/body/8 weeks, 147 mg/body/8 weeks, 147.5 mg/body/8 weeks, 148 mg/body/8 weeks, 148.5 mg/body/8 weeks, 149 mg/body/8 weeks, 149.5 mg/body/8 weeks, 150 mg/body/8 weeks, 150.5 mg/body/8 weeks, 151 mg/body/8 weeks, 151.5 mg/body/8 weeks, 152 mg/body/8 weeks, 152.5 mg/body/8 weeks, 153 mg/body/8 weeks, 153.5 mg/body/8 weeks, 154 mg/body/8 weeks, 154.5 mg/body/8 weeks, 155 mg/body/8 weeks, 155.5 mg/body/8 weeks, 156 mg/body/8 weeks, 156.5 mg/body/8 weeks, 157 mg/body/8 weeks, 157.5 mg/body/8 weeks, 158 mg/body/8 weeks, 158.5 mg/body/8 weeks, 159 mg/body/8 weeks, 159.5 mg/body/8 weeks, 160 mg/body/8 weeks, 160.5 mg/body/8 weeks, 161 mg/body/8 weeks, 161.5 mg/body/8 weeks, 162 mg/body/8 weeks, 162.5 mg/body/8 weeks, 163 mg/body/8 weeks, 163.5 mg/body/8 weeks, 164 mg/body/8 weeks, 164.5 mg/body/8 weeks, 165 mg/body/8 weeks, 165.5 mg/body/8 weeks, 166 mg/body/8 weeks, 166.5 mg/body/8 weeks, 167 mg/body/8 weeks, 167.5 mg/body/8 weeks, 168 mg/body/8 weeks, 168.5 mg/body/8 weeks, 169 mg/body/8 weeks, 169.5 mg/body/8 weeks, 170 mg/body/8 weeks, 170.5 mg/body/8 weeks, 171 mg/body/8 weeks, 171.5 mg/body/8 weeks, 172 mg/body/8 weeks, 172.5 mg/body/8 weeks, 173 mg/body/8 weeks, 173.5 mg/body/8 weeks, 174 mg/body/8 weeks, 174.5 mg/body/8 weeks, 175 mg/body/8 weeks, 175.5 mg/body/8 weeks, 176 mg/body/8 weeks, 176.5 mg/body/8 weeks, 177 mg/body/8 weeks, 177.5 mg/body/8 weeks, 178 mg/body/8 weeks, 178.5 mg/body/8 weeks, 179 mg/body/8 weeks, 179.5 mg/body/8 weeks, 180 mg/body/8 weeks, 180.5 mg/body/8 weeks, 181 mg/body/8 weeks, 181.5 mg/body/8 weeks, 182 mg/body/8 weeks, 182.5 mg/body/8 weeks, 183 mg/body/8 weeks, 183.5 mg/body/8 weeks, 184 mg/body/8 weeks, 184.5 mg/body/8 weeks, 185 mg/body/8 weeks, 185.5 mg/body/8 weeks, 186 mg/body/8 weeks, 186.5 mg/body/8 weeks, 187 mg/body/8 weeks, 187.5 mg/body/8 weeks, 188 mg/body/8 weeks, 188.5 mg/body/8 weeks, 189 mg/body/8 weeks, 189.5 mg/body/8 weeks, 190 mg/body/8 weeks, 190.5 mg/body/8 weeks, 191 mg/body/8 weeks, 191.5 mg/body/8 weeks, 192 mg/body/8 weeks, 192.5 mg/body/8 weeks, 193 mg/body/8 weeks, 193.5 mg/body/8 weeks, 194 mg/body/8 weeks, 194.5 mg/body/8 weeks, 195 mg/body/8 weeks, 195.5 mg/body/8 weeks, 196 mg/body/8 weeks, 196.5 mg/body/8 weeks, 197 mg/body/8 weeks, 197.5 mg/body/8 weeks, 198 mg/body/8 weeks, 198.5 mg/body/8 weeks, 199 mg/body/8 weeks, 199.5 mg/body/8 weeks, or 200 mg/body/8 weeks. Alternatively, in a non-limiting embodiment, the IL-31 antagonist of the present disclosure may be administered at, for example, 50 mg/body/10 weeks, 50.5 mg/body/10 weeks, 51 mg/body/10 weeks, 51.5 mg/body/10 weeks, 52 mg/body/10 weeks, 52.5 mg/body/10 weeks, 53 mg/body/10 weeks, 53.5 mg/body/10 weeks, 54 mg/body/10 weeks, 54.5 mg/body/10 weeks, 55 mg/body/10 weeks, 55.5 mg/body/10 weeks, 56 mg/body/10 weeks, 56.5 mg/body/10 weeks, 57 mg/body/10 weeks, 57.5 mg/body/10 weeks, 58 mg/body/10 weeks, 58.5 mg/body/10 weeks, 59 mg/body/10 weeks, 59.5 mg/body/10 weeks, 60 mg/body/10 weeks, 60.5 mg/body/10 weeks, 61 mg/body/10 weeks, 61.5 mg/body/10 weeks, 62 mg/body/10 weeks, 62.5 mg/body/10 weeks, 63 mg/body/10 weeks, 63.5 mg/body/10 weeks, 64 mg/body/10 weeks, 64.5 mg/body/10 weeks, 65 mg/body/10 weeks, 65.5 mg/body/10 weeks, 66 mg/body/10 weeks, 66.5 mg/body/10 weeks, 67 mg/body/10 weeks, 67.5 mg/body/10 weeks, 68 mg/body/10 weeks, 68.5 mg/body/10 weeks, 69 mg/body/10 weeks, 69.5 mg/body/10 weeks, 70 mg/body/10 weeks, 70.5 mg/body/10 weeks, 71 mg/body/10 weeks, 71.5 mg/body/10 weeks, 72 mg/body/10 weeks, 72.5 mg/body/10 weeks, 73 mg/body/10 weeks, 73.5 mg/body/10 weeks, 74 mg/body/10 weeks, 74.5 mg/body/10 weeks, 75 mg/body/10 weeks, 75.5 mg/body/10 weeks, 76 mg/body/10 weeks, 76.5 mg/body/10 weeks, 77 mg/body/10 weeks, 77.5 mg/body/10 weeks, 78 mg/body/10 weeks, 78.5 mg/body/10 weeks, 79 mg/body/10 weeks, 79.5 mg/body/10 weeks, 80 mg/body/10 weeks, 80.5 mg/body/10 weeks, 81 mg/body/10 weeks, 81.5 mg/body/10 weeks, 82 mg/body/10 weeks, 82.5 mg/body/10 weeks, 83 mg/body/10 weeks, 83.5 mg/body/10 weeks, 84 mg/body/10 weeks, 84.5 mg/body/10 weeks, 85 mg/body/10 weeks, 85.5 mg/body/10 weeks, 86 mg/body/10 weeks, 86.5 mg/body/10 weeks, 87 mg/body/10 weeks, 87.5 mg/body/10 weeks, 88 mg/body/10 weeks, 88.5 mg/body/10 weeks, 89 mg/body/10 weeks, 89.5 mg/body/10 weeks, 90 mg/body/10 weeks, 90.5 mg/body/10 weeks, 91 mg/body/10 weeks, 91.5 mg/body/10 weeks, 92 mg/body/10 weeks, 92.5 mg/body/10 weeks, 93 mg/body/10 weeks, 93.5 mg/body/10 weeks, 94 mg/body/10 weeks, 94.5 mg/body/10 weeks, 95 mg/body/10 weeks, 95.5 mg/body/10 weeks, 96 mg/body/10 weeks, 96.5 mg/body/10 weeks, 97 mg/body/10 weeks, 97.5 mg/body/10 weeks, 98 mg/body/10 weeks, 98.5 mg/body/10 weeks, 99 mg/body/10 weeks, 99.5 mg/body/10 weeks, 100 mg/body/10 weeks, 100.5 mg/body/10 weeks, 101 mg/body/10 weeks, 101.5 mg/body/10 weeks, 102 mg/body/10 weeks, 102.5 mg/body/10 weeks, 103 mg/body/10 weeks, 103.5 mg/body/10 weeks, 104 mg/body/10 weeks, 104.5 mg/body/10 weeks, 105 mg/body/10 weeks, 105.5 mg/body/10 weeks, 106 mg/body/10 weeks, 106.5 mg/body/10 weeks, 107 mg/body/10 weeks, 107.5 mg/body/10 weeks, 108 mg/body/10 weeks, 108.5 mg/body/10 weeks, 109 mg/body/10 weeks, 109.5 mg/body/10 weeks, 110 mg/body/10 weeks, 110.5 mg/body/10 weeks, 111 mg/body/10 weeks, 111.5 mg/body/10 weeks, 112 mg/body/10 weeks, 112.5 mg/body/10 weeks, 113 mg/body/10 weeks, 113.5 mg/body/10 weeks, 114 mg/body/10 weeks, 114.5 mg/body/10 weeks, 115 mg/body/10 weeks, 115.5 mg/body/10 weeks, 116 mg/body/10 weeks, 116.5 mg/body/10 weeks, 117 mg/body/10 weeks, 117.5 mg/body/10 weeks, 118 mg/body/10 weeks, 118.5 mg/body/10 weeks, 119 mg/body/10 weeks, 119.5 mg/body/10 weeks, 120 mg/body/10 weeks, 120.5 mg/body/10 weeks, 121 mg/body/10 weeks, 121.5 mg/body/10 weeks, 122 mg/body/10 weeks, 122.5 mg/body/10 weeks, 123 mg/body/10 weeks, 123.5 mg/body/10 weeks, 124 mg/body/10 weeks, 124.5 mg/body/10 weeks, 125 mg/body/10 weeks, 125.5 mg/body/10 weeks, 126 mg/body/10 weeks, 126.5 mg/body/10 weeks, 127 mg/body/10 weeks, 127.5 mg/body/10 weeks, 128 mg/body/10 weeks, 128.5 mg/body/10 weeks, 129 mg/body/10 weeks, 129.5 mg/body/10 weeks, 130 mg/body/10 weeks, 130.5 mg/body/10 weeks, 131 mg/body/10 weeks, 131.5 mg/body/10 weeks, 132 mg/body/10 weeks, 132.5 mg/body/10 weeks, 133 mg/body/10 weeks, 133.5 mg/body/10 weeks, 134 mg/body/10 weeks, 134.5 mg/body/10 weeks, 135 mg/body/10 weeks, 135.5 mg/body/10 weeks, 136 mg/body/10 weeks, 136.5 mg/body/10 weeks, 137 mg/body/10 weeks, 137.5 mg/body/10 weeks, 138 mg/body/10 weeks, 138.5 mg/body/10 weeks, 139 mg/body/10 weeks, 139.5 mg/body/10 weeks, 140 mg/body/10 weeks, 140.5 mg/body/10 weeks, 141 mg/body/10 weeks, 141.5 mg/body/10 weeks, 142 mg/body/10 weeks, 142.5 mg/body/10 weeks, 143 mg/body/10 weeks, 143.5 mg/body/10 weeks, 144 mg/body/10 weeks, 144.5 mg/body/10 weeks, 145 mg/body/10 weeks, 145.5 mg/body/10 weeks, 146 mg/body/10 weeks, 146.5 mg/body/10 weeks, 147 mg/body/10 weeks, 147.5 mg/body/10 weeks, 148 mg/body/10 weeks, 148.5 mg/body/10 weeks, 149 mg/body/10 weeks, 149.5 mg/body/10 weeks, 150 mg/body/10 weeks, 150.5 mg/body/10 weeks, 151 mg/body/10 weeks, 151.5 mg/body/10 weeks, 152 mg/body/10 weeks, 152.5 mg/body/10 weeks, 153 mg/body/10 weeks, 153.5 mg/body/10 weeks, 154 mg/body/10 weeks, 154.5 mg/body/10 weeks, 155 mg/body/10 weeks, 155.5 mg/body/10 weeks, 156 mg/body/10 weeks, 156.5 mg/body/10 weeks, 157 mg/body/10 weeks, 157.5 mg/body/10 weeks, 158 mg/body/10 weeks, 158.5 mg/body/10 weeks, 159 mg/body/10 weeks, 159.5 mg/body/10 weeks, 160 mg/body/10 weeks, 160.5 mg/body/10 weeks, 161 mg/body/10 weeks, 161.5 mg/body/10 weeks, 162 mg/body/10 weeks, 162.5 mg/body/10 weeks, 163 mg/body/10 weeks, 163.5 mg/body/10 weeks, 164 mg/body/10 weeks, 164.5 mg/body/10 weeks, 165 mg/body/10 weeks, 165.5 mg/body/10 weeks, 166 mg/body/10 weeks, 166.5 mg/body/10 weeks, 167 mg/body/10 weeks, 167.5 mg/body/10 weeks, 168 mg/body/10 weeks, 168.5 mg/body/10 weeks, 169 mg/body/10 weeks, 169.5 mg/body/10 weeks, 170 mg/body/10 weeks, 170.5 mg/body/10 weeks, 171 mg/body/10 weeks, 171.5 mg/body/10 weeks, 172 mg/body/10 weeks, 172.5 mg/body/10 weeks, 173 mg/body/10 weeks, 173.5 mg/body/10 weeks, 174 mg/body/10 weeks, 174.5 mg/body/10 weeks, 175 mg/body/10 weeks, 175.5 mg/body/10 weeks, 176 mg/body/10 weeks, 176.5 mg/body/10 weeks, 177 mg/body/10 weeks, 177.5 mg/body/10 weeks, 178 mg/body/10 weeks, 178.5 mg/body/10 weeks, 179 mg/body/10 weeks, 179.5 mg/body/10 weeks, 180 mg/body/10 weeks, 180.5 mg/body/10 weeks, 181 mg/body/10 weeks, 181.5 mg/body/10 weeks, 182 mg/body/10 weeks, 182.5 mg/body/10 weeks, 183 mg/body/10 weeks, 183.5 mg/body/10 weeks, 184 mg/body/10 weeks, 184.5 mg/body/10 weeks, 185 mg/body/10 weeks, 185.5 mg/body/10 weeks, 186 mg/body/10 weeks, 186.5 mg/body/10 weeks, 187 mg/body/10 weeks, 187.5 mg/body/10 weeks, 188 mg/body/10 weeks, 188.5 mg/body/10 weeks, 189 mg/body/10 weeks, 189.5 mg/body/10 weeks, 190 mg/body/10 weeks, 190.5 mg/body/10 weeks, 191 mg/body/10 weeks, 191.5 mg/body/10 weeks, 192 mg/body/10 weeks, 192.5 mg/body/10 weeks, 193 mg/body/10 weeks, 193.5 mg/body/10 weeks, 194 mg/body/10 weeks, 194.5 mg/body/10 weeks, 195 mg/body/10 weeks, 195.5 mg/body/10 weeks, 196 mg/body/10 weeks, 196.5 mg/body/10 weeks, 197 mg/body/10 weeks, 197.5 mg/body/10 weeks, 198 mg/body/10 weeks, 198.5 mg/body/10 weeks, 199 mg/body/10 weeks, 199.5 mg/body/10 weeks, or 200 mg/body/10 weeks. Alternatively, in a non-limiting embodiment, the IL-31 antagonist of the present disclosure may be administered at, for example, 50 mg/body/12 weeks, 50.5 mg/body/12 weeks, 51 mg/body/12 weeks, 51.5 mg/body/12 weeks, 52 mg/body/12 weeks, 52.5 mg/body/12 weeks, 53 mg/body/12 weeks, 53.5 mg/body/12 weeks, 54 mg/body/12 weeks, 54.5 mg/body/12 weeks, 55 mg/body/12 weeks, 55.5 mg/body/12 weeks, 56 mg/body/12 weeks, 56.5 mg/body/12 weeks, 57 mg/body/12 weeks, 57.5 mg/body/12 weeks, 58 mg/body/12 weeks, 58.5 mg/body/12 weeks, 59 mg/body/12 weeks, 59.5 mg/body/12 weeks, 60 mg/body/12 weeks, 60.5 mg/body/12 weeks, 61 mg/body/12 weeks, 61.5 mg/body/12 weeks, 62 mg/body/12 weeks, 62.5 mg/body/12 weeks, 63 mg/body/12 weeks, 63.5 mg/body/12 weeks, 64 mg/body/12 weeks, 64.5 mg/body/12 weeks, 65 mg/body/12 weeks, 65.5 mg/body/12 weeks, 66 mg/body/12 weeks, 66.5 mg/body/12 weeks, 67 mg/body/12 weeks, 67.5 mg/body/12 weeks, 68 mg/body/12 weeks, 68.5 mg/body/12 weeks, 69 mg/body/12 weeks, 69.5 mg/body/12 weeks, 70 mg/body/12 weeks, 70.5 mg/body/12 weeks, 71 mg/body/12 weeks, 71.5 mg/body/12 weeks, 72 mg/body/12 weeks, 72.5 mg/body/12 weeks, 73 mg/body/12 weeks, 73.5 mg/body/12 weeks, 74 mg/body/12 weeks, 74.5 mg/body/12 weeks, 75 mg/body/12 weeks, 75.5 mg/body/12 weeks, 76 mg/body/12 weeks, 76.5 mg/body/12 weeks, 77 mg/body/12 weeks, 77.5 mg/body/12 weeks, 78 mg/body/12 weeks, 78.5 mg/body/12 weeks, 79 mg/body/12 weeks, 79.5 mg/body/12 weeks, 80 mg/body/12 weeks, 80.5 mg/body/12 weeks, 81 mg/body/12 weeks, 81.5 mg/body/12 weeks, 82 mg/body/12 weeks, 82.5 mg/body/12 weeks, 83 mg/body/12 weeks, 83.5 mg/body/12 weeks, 84 mg/body/12 weeks, 84.5 mg/body/12 weeks, 85 mg/body/12 weeks, 85.5 mg/body/12 weeks, 86 mg/body/12 weeks, 86.5 mg/body/12 weeks, 87 mg/body/12 weeks, 87.5 mg/body/12 weeks, 88 mg/body/12 weeks, 88.5 mg/body/12 weeks, 89 mg/body/12 weeks, 89.5 mg/body/12 weeks, 90 mg/body/12 weeks, 90.5 mg/body/12 weeks, 91 mg/body/12 weeks, 91.5 mg/body/12 weeks, 92 mg/body/12 weeks, 92.5 mg/body/12 weeks, 93 mg/body/12 weeks, 93.5 mg/body/12 weeks, 94 mg/body/12 weeks, 94.5 mg/body/12 weeks, 95 mg/body/12 weeks, 95.5 mg/body/12 weeks, 96 mg/body/12 weeks, 96.5 mg/body/12 weeks, 97 mg/body/12 weeks, 97.5 mg/body/12 weeks, 98 mg/body/12 weeks, 98.5 mg/body/12 weeks, 99 mg/body/12 weeks, 99.5 mg/body/12 weeks, 100 mg/body/12 weeks, 100.5 mg/body/12 weeks, 101 mg/body/12 weeks, 101.5 mg/body/12 weeks, 102 mg/body/12 weeks, 102.5 mg/body/12 weeks, 103 mg/body/12 weeks, 103.5 mg/body/12 weeks, 104 mg/body/12 weeks, 104.5 mg/body/12 weeks, 105 mg/body/12 weeks, 105.5 mg/body/12 weeks, 106 mg/body/12 weeks, 106.5 mg/body/12 weeks, 107 mg/body/12 weeks, 107.5 mg/body/12 weeks, 108 mg/body/12 weeks, 108.5 mg/body/12 weeks, 109 mg/body/12 weeks, 109.5 mg/body/12 weeks, 110 mg/body/12 weeks, 110.5 mg/body/12 weeks, 111 mg/body/12 weeks, 111.5 mg/body/12 weeks, 112 mg/body/12 weeks, 112.5 mg/body/12 weeks, 113 mg/body/12 weeks, 113.5 mg/body/12 weeks, 114 mg/body/12 weeks, 114.5 mg/body/12 weeks, 115 mg/body/12 weeks, 115.5 mg/body/12 weeks, 116 mg/body/12 weeks, 116.5 mg/body/12 weeks, 117 mg/body/12 weeks, 117.5 mg/body/12 weeks, 118 mg/body/12 weeks, 118.5 mg/body/12 weeks, 119 mg/body/12 weeks, 119.5 mg/body/12 weeks, 120 mg/body/12 weeks, 120.5 mg/body/12 weeks, 121 mg/body/12 weeks, 121.5 mg/body/12 weeks, 122 mg/body/12 weeks, 122.5 mg/body/12 weeks, 123 mg/body/12 weeks, 123.5 mg/body/12 weeks, 124 mg/body/12 weeks, 124.5 mg/body/12 weeks, 125 mg/body/12 weeks, 125.5 mg/body/12 weeks, 126 mg/body/12 weeks, 126.5 mg/body/12 weeks, 127 mg/body/12 weeks, 127.5 mg/body/12 weeks, 128 mg/body/12 weeks, 128.5 mg/body/12 weeks, 129 mg/body/12 weeks, 129.5 mg/body/12 weeks, 130 mg/body/12 weeks, 130.5 mg/body/12 weeks, 131 mg/body/12 weeks, 131.5 mg/body/12 weeks, 132 mg/body/12 weeks, 132.5 mg/body/12 weeks, 133 mg/body/12 weeks, 133.5 mg/body/12 weeks, 134 mg/body/12 weeks, 134.5 mg/body/12 weeks, 135 mg/body/12 weeks, 135.5 mg/body/12 weeks, 136 mg/body/12 weeks, 136.5 mg/body/12 weeks, 137 mg/body/12 weeks, 137.5 mg/body/12 weeks, 138 mg/body/12 weeks, 138.5 mg/body/12 weeks, 139 mg/body/12 weeks, 139.5 mg/body/12 weeks, 140 mg/body/12 weeks, 140.5 mg/body/12 weeks, 141 mg/body/12 weeks, 141.5 mg/body/12 weeks, 142 mg/body/12 weeks, 142.5 mg/body/12 weeks, 143 mg/body/12 weeks, 143.5 mg/body/12 weeks, 144 mg/body/12 weeks, 144.5 mg/body/12 weeks, 145 mg/body/12 weeks, 145.5 mg/body/12 weeks, 146 mg/body/12 weeks, 146.5 mg/body/12 weeks, 147 mg/body/12 weeks, 147.5 mg/body/12 weeks, 148 mg/body/12 weeks, 148.5 mg/body/12 weeks, 149 mg/body/12 weeks, 149.5 mg/body/12 weeks, 150 mg/body/12 weeks, 150.5 mg/body/12 weeks, 151 mg/body/12 weeks, 151.5 mg/body/12 weeks, 152 mg/body/12 weeks, 152.5 mg/body/12 weeks, 153 mg/body/12 weeks, 153.5 mg/body/12 weeks, 154 mg/body/12 weeks, 154.5 mg/body/12 weeks, 155 mg/body/12 weeks, 155.5 mg/body/12 weeks, 156 mg/body/12 weeks, 156.5 mg/body/12 weeks, 157 mg/body/12 weeks, 157.5 mg/body/12 weeks, 158 mg/body/12 weeks, 158.5 mg/body/12 weeks, 159 mg/body/12 weeks, 159.5 mg/body/12 weeks, 160 mg/body/12 weeks, 160.5 mg/body/12 weeks, 161 mg/body/12 weeks, 161.5 mg/body/12 weeks, 162 mg/body/12 weeks, 162.5 mg/body/12 weeks, 163 mg/body/12 weeks, 163.5 mg/body/12 weeks, 164 mg/body/12 weeks, 164.5 mg/body/12 weeks, 165 mg/body/12 weeks, 165.5 mg/body/12 weeks, 166 mg/body/12 weeks, 166.5 mg/body/12 weeks, 167 mg/body/12 weeks, 167.5 mg/body/12 weeks, 168 mg/body/12 weeks, 168.5 mg/body/12 weeks, 169 mg/body/12 weeks, 169.5 mg/body/12 weeks, 170 mg/body/12 weeks, 170.5 mg/body/12 weeks, 171 mg/body/12 weeks, 171.5 mg/body/12 weeks, 172 mg/body/12 weeks, 172.5 mg/body/12 weeks, 173 mg/body/12 weeks, 173.5 mg/body/12 weeks, 174 mg/body/12 weeks, 174.5 mg/body/12 weeks, 175 mg/body/12 weeks, 175.5 mg/body/12 weeks, 176 mg/body/12 weeks, 176.5 mg/body/12 weeks, 177 mg/body/12 weeks, 177.5 mg/body/12 weeks, 178 mg/body/12 weeks, 178.5 mg/body/12 weeks, 179 mg/body/12 weeks, 179.5 mg/body/12 weeks, 180 mg/body/12 weeks, 180.5 mg/body/12 weeks, 181 mg/body/12 weeks, 181.5 mg/body/12 weeks, 182 mg/body/12 weeks, 182.5 mg/body/12 weeks, 183 mg/body/12 weeks, 183.5 mg/body/12 weeks, 184 mg/body/12 weeks, 184.5 mg/body/12 weeks, 185 mg/body/12 weeks, 185.5 mg/body/12 weeks, 186 mg/body/12 weeks, 186.5 mg/body/12 weeks, 187 mg/body/12 weeks, 187.5 mg/body/12 weeks, 188 mg/body/12 weeks, 188.5 mg/body/12 weeks, 189 mg/body/12 weeks, 189.5 mg/body/12 weeks, 190 mg/body/12 weeks, 190.5 mg/body/12 weeks, 191 mg/body/12 weeks, 191.5 mg/body/12 weeks, 192 mg/body/12 weeks, 192.5 mg/body/12 weeks, 193 mg/body/12 weeks, 193.5 mg/body/12 weeks, 194 mg/body/12 weeks, 194.5 mg/body/12 weeks, 195 mg/body/12 weeks, 195.5 mg/body/12 weeks, 196 mg/body/12 weeks, 196.5 mg/body/12 weeks, 197 mg/body/12 weeks, 197.5 mg/body/12 weeks, 198 mg/body/12 weeks, 198.5 mg/body/12 weeks, 199 mg/body/12 weeks, 199.5 mg/body/12 weeks, or 200 mg/body/12 weeks.

In a non-limiting embodiment, the IL-31 antagonist of the present disclosure may be administered at 25 mg to 100 mg/body/4 weeks, 50 mg to 100 mg/body/4 weeks, or 50 mg to 75 mg/body/4 weeks. In another non-limiting embodiment, the IL-31 antagonist of the present disclosure may be administered at 10 mg to 50 mg/body/2 weeks or 20 mg to 40 mg/body/2 weeks.

Alternatively, in an embodiment where the IL-31 antagonist of the present disclosure is administered at a predetermined dosing interval and a predetermined dose (dosage) repeatedly at the same dose and the same dosing interval, the administration of a dose of the IL-31 antagonist of the present disclosure first administered to a subject (initial dose) may be followed by administration of a continued dose (i.e. a dose that continues to be administered after administration of the initial dose). In one non-limiting embodiment, the initial dose may be twice the continued dose. For example, the initial dose may be 60 mg/body, and the dosing interval between the initial dose and the first continued dose may be 4 weeks, and the continued dose may be 30 mg/body/4 weeks.

Alternatively, in an embodiment where the IL-31 antagonist of the present disclosure is administered at a predetermined dosing interval and a predetermined dose (dosage) and the administration is repeated at the same dose and the same dosing interval, the IL-31 antagonist of the present disclosure may be administered at "0.01 mg to 10 mg/kg/1 day to 12 weeks". Herein, for example, "0.01 mg to 10 mg/kg/1 day to 12 weeks" is contemplated to mean that

EP 4 209 227 A1

one dosage is selected from 0.01 mg to 10 mg as the dosage of the IL-31 antagonist of the present disclosure, and any one dosing interval is selected from 1 day to 12 weeks as the dosing interval (e.g., 4 weeks) of the IL-31 antagonist of the present disclosure, and the IL-31 antagonist is repeatedly administered to a subject at the same dose and the same dosing interval. In an embodiment where the IL-31 antagonist of the present disclosure is administered at a predetermined dosing interval and a predetermined dose (dosage) and the administration is repeated at the same dose and the same dosing interval, the IL-31 antagonist of the present disclosure is preferably administered at 0.01 mg to 10 mg/kg/2 to 8 weeks, and may be administered at, for example, 0.01 mg to 10 mg/kg/2 weeks, 0.01 mg to 10 mg/kg/4 weeks, 0.01 mg to 10 mg/kg/6 weeks, or 0.01 mg to 10 mg/kg/8 weeks, but not limited thereto. Alternatively, the IL-31 antagonist of the present disclosure is more preferably administered at 0.1 mg to 3 mg/body/2 to 8 weeks, and may be administered at, for example, 0.1 mg to 3 mg/kg/2 weeks, 0.1 mg to 3 mg/kg/4 weeks, 0.1 mg to 3 mg/kg/6 weeks, or 0.1 mg to 3 mg/kg/8 weeks. Alternatively, as an example, the IL-31 antagonist of the present disclosure is more preferably administered at 0.2 mg to 2 mg/body/2 to 8 weeks, and may be administered at, for example, 0.2 mg to 2 mg/kg/2 weeks, 0.2 mg to 2 mg/kg/4 weeks, 0.2 mg to 2 mg/kg/6 weeks, or 0.2 mg to 2 mg/kg/8 weeks. Alternatively, as an example, the IL-31 antagonist of the present disclosure is more preferably administered at 0.5 mg to 1.5 mg/body/4 to 8 weeks, and may be administered at, for example, 0.5 mg to 1.5 mg/kg/4 weeks, 0.5 mg to 1.5 mg/kg/6 weeks, or 0.5 mg to 1.5 mg/kg/8 weeks. In a non-limiting embodiment, the IL-31 antagonist of the present disclosure may be administered at, for example, 0.1 mg/kg/4 weeks, 0.11 mg/kg/4 weeks, 0.12 mg/kg/4 weeks, 0.125 mg/kg/4 weeks, 0.13 mg/kg/4 weeks, 0.14 mg/kg/4 weeks, 0.15 mg/kg/4 weeks, 0.16 mg/kg/4 weeks, 0.17 mg/kg/4 weeks, 0.18 mg/kg/4 weeks, 0.19 mg/kg/4 weeks, 0.2 mg/kg/4 weeks, 0.21 mg/kg/4 weeks, 0.22 mg/kg/4 weeks, 0.23 mg/kg/4 weeks, 0.24 mg/kg/4 weeks, 0.25 mg/kg/4 weeks, 0.26 mg/kg/4 weeks, 0.27 mg/kg/4 weeks, 0.28 mg/kg/4 weeks, 0.29 mg/kg/4 weeks, 0.3 mg/kg/4 weeks, 0.31 mg/kg/4 weeks, 0.32 mg/kg/4 weeks, 0.33 mg/kg/4 weeks, 0.34 mg/kg/4 weeks, 0.35 mg/kg/4 weeks, 0.36 mg/kg/4 weeks, 0.37 mg/kg/4 weeks, 0.38 mg/kg/4 weeks, 0.39 mg/kg/4 weeks, 0.4 mg/kg/4 weeks, 0.41 mg/kg/4 weeks, 0.42 mg/kg/4 weeks, 0.43 mg/kg/4 weeks, 0.44 mg/kg/4 weeks, 0.45 mg/kg/4 weeks, 0.46 mg/kg/4 weeks, 0.47 mg/kg/4 weeks, 0.48 mg/kg/4 weeks, 0.49 mg/kg/4 weeks, 0.5 mg/kg/4 weeks, 0.51 mg/kg/4 weeks, 0.52 mg/kg/4 weeks, 0.53 mg/kg/4 weeks, 0.54 mg/kg/4 weeks, 0.55 mg/kg/4 weeks, 0.56 mg/kg/4 weeks, 0.57 mg/kg/4 weeks, 0.58 mg/kg/4 weeks, 0.59 mg/kg/4 weeks, 0.6 mg/kg/4 weeks, 0.61 mg/kg/4 weeks, 0.62 mg/kg/4 weeks, 0.63 mg/kg/4 weeks, 0.64 mg/kg/4 weeks, 0.65 mg/kg/4 weeks, 0.66 mg/kg/4 weeks, 0.67 mg/kg/4 weeks, 0.68 mg/kg/4 weeks, 0.69 mg/kg/4 weeks, 0.7 mg/kg/4 weeks, 0.71 mg/kg/4 weeks, 0.72 mg/kg/4 weeks, 0.73 mg/kg/4 weeks, 0.74 mg/kg/4 weeks, 0.75 mg/kg/4 weeks, 0.76 mg/kg/4 weeks, 0.77 mg/kg/4 weeks, 0.78 mg/kg/4 weeks, 0.79 mg/kg/4 weeks, 0.8 mg/kg/4 weeks, 0.81 mg/kg/4 weeks, 0.82 mg/kg/4 weeks, 0.83 mg/kg/4 weeks, 0.84 mg/kg/4 weeks, 0.85 mg/kg/4 weeks, 0.86 mg/kg/4 weeks, 0.87 mg/kg/4 weeks, 0.88 mg/kg/4 weeks, 0.89 mg/kg/4 weeks, 0.9 mg/kg/4 weeks, 0.91 mg/kg/4 weeks, 0.92 mg/kg/4 weeks, 0.93 mg/kg/4 weeks, 0.94 mg/kg/4 weeks, 0.95 mg/kg/4 weeks, 0.96 mg/kg/4 weeks, 0.97 mg/kg/4 weeks, 0.98 mg/kg/4 weeks, 0.99 mg/kg/4 weeks, 1 mg/kg/4 weeks, 1.01 mg/kg/4 weeks, 1.02 mg/kg/4 weeks, 1.03 mg/kg/4 weeks, 1.04 mg/kg/4 weeks, 1.05 mg/kg/4 weeks, 1.06 mg/kg/4 weeks, 1.07 mg/kg/4 weeks, 1.08 mg/kg/4 weeks, 1.09 mg/kg/4 weeks, 1.1 mg/kg/4 weeks, 1.11 mg/kg/4 weeks, 1.12 mg/kg/4 weeks, 1.13 mg/kg/4 weeks, 1.14 mg/kg/4 weeks, 1.15 mg/kg/4 weeks, 1.16 mg/kg/4 weeks, 1.17 mg/kg/4 weeks, 1.18 mg/kg/4 weeks, 1.19 mg/kg/4 weeks, 1.2 mg/kg/4 weeks, 1.21 mg/kg/4 weeks, 1.22 mg/kg/4 weeks, 1.23 mg/kg/4 weeks, 1.24 mg/kg/4 weeks, 1.25 mg/kg/4 weeks, 1.26 mg/kg/4 weeks, 1.27 mg/kg/4 weeks, 1.28 mg/kg/4 weeks, 1.29 mg/kg/4 weeks, 1.3 mg/kg/4 weeks, 1.31 mg/kg/4 weeks, 1.32 mg/kg/4 weeks, 1.33 mg/kg/4 weeks, 1.34 mg/kg/4 weeks, 1.35 mg/kg/4 weeks, 1.36 mg/kg/4 weeks, 1.37 mg/kg/4 weeks, 1.38 mg/kg/4 weeks, 1.39 mg/kg/4 weeks, 1.4 mg/kg/4 weeks, 1.41 mg/kg/4 weeks, 1.42 mg/kg/4 weeks, 1.43 mg/kg/4 weeks, 1.44 mg/kg/4 weeks, 1.45 mg/kg/4 weeks, 1.46 mg/kg/4 weeks, 1.47 mg/kg/4 weeks, 1.48 mg/kg/4 weeks, 1.49 mg/kg/4 weeks, 1.5 mg/kg/4 weeks, 1.51 mg/kg/4 weeks, 1.52 mg/kg/4 weeks, 1.53 mg/kg/4 weeks, 1.54 mg/kg/4 weeks, 1.55 mg/kg/4 weeks, 1.56 mg/kg/4 weeks, 1.57 mg/kg/4 weeks, 1.58 mg/kg/4 weeks, 1.59 mg/kg/4 weeks, 1.6 mg/kg/4 weeks, 1.61 mg/kg/4 weeks, 1.62 mg/kg/4 weeks, 1.63 mg/kg/4 weeks, 1.64 mg/kg/4 weeks, 1.65 mg/kg/4 weeks, 1.66 mg/kg/4 weeks, 1.67 mg/kg/4 weeks, 1.68 mg/kg/4 weeks, 1.69 mg/kg/4 weeks, 1.7 mg/kg/4 weeks, 1.71 mg/kg/4 weeks, 1.72 mg/kg/4 weeks, 1.73 mg/kg/4 weeks, 1.74 mg/kg/4 weeks, 1.75 mg/kg/4 weeks, 1.76 mg/kg/4 weeks, 1.77 mg/kg/4 weeks, 1.78 mg/kg/4 weeks, 1.79 mg/kg/4 weeks, 1.8 mg/kg/4 weeks, 1.81 mg/kg/4 weeks, 1.82 mg/kg/4 weeks, 1.83 mg/kg/4 weeks, 1.84 mg/kg/4 weeks, 1.85 mg/kg/4 weeks, 1.86 mg/kg/4 weeks, 1.87 mg/kg/4 weeks, 1.88 mg/kg/4 weeks, 1.89 mg/kg/4 weeks, 1.9 mg/kg/4 weeks, 1.91 mg/kg/4 weeks, 1.92 mg/kg/4 weeks, 1.93 mg/kg/4 weeks, 1.94 mg/kg/4 weeks, 1.95 mg/kg/4 weeks, 1.96 mg/kg/4 weeks, 1.97 mg/kg/4 weeks, 1.98 mg/kg/4 weeks, 1.99 mg/kg/4 weeks, 2 mg/kg/4 weeks, 2.01 mg/kg/4 weeks, 2.02 mg/kg/4 weeks, 2.03 mg/kg/4 weeks, 2.04 mg/kg/4 weeks, 2.05 mg/kg/4 weeks, 2.06 mg/kg/4 weeks, 2.07 mg/kg/4 weeks, 2.08 mg/kg/4 weeks, 2.09 mg/kg/4 weeks, 2.1 mg/kg/4 weeks, 2.11 mg/kg/4 weeks, 2.12 mg/kg/4 weeks, 2.13 mg/kg/4 weeks, 2.14 mg/kg/4 weeks, 2.15 mg/kg/4 weeks, 2.16 mg/kg/4 weeks, 2.17 mg/kg/4 weeks, 2.18 mg/kg/4 weeks, 2.19 mg/kg/4 weeks, 2.2 mg/kg/4 weeks, 2.21 mg/kg/4 weeks, 2.22 mg/kg/4 weeks, 2.23 mg/kg/4 weeks, 2.24 mg/kg/4 weeks, 2.25 mg/kg/4 weeks, 2.26 mg/kg/4 weeks, 2.27 mg/kg/4 weeks, 2.28 mg/kg/4 weeks, 2.29 mg/kg/4 weeks, 2.3 mg/kg/4 weeks, 2.31 mg/kg/4 weeks, 2.32 mg/kg/4 weeks, 2.33 mg/kg/4 weeks, 2.34 mg/kg/4 weeks, 2.35 mg/kg/4 weeks, 2.36 mg/kg/4 weeks, 2.37 mg/kg/4 weeks, 2.38 mg/kg/4 weeks, 2.39 mg/kg/4 weeks, 2.4 mg/kg/4 weeks, 2.41 mg/kg/4 weeks, 2.42 mg/kg/4 weeks, 2.43 mg/kg/4 weeks, 2.44 mg/kg/4 weeks, 2.45 mg/kg/4 weeks, 2.46 mg/kg/4 weeks, 2.47 mg/kg/4 weeks, 2.48 mg/kg/4 weeks, 2.49 mg/kg/4 weeks, 2.5 mg/kg/4 weeks, 2.51 mg/kg/4 weeks, 2.52 mg/kg/4 weeks, 2.53 mg/kg/4 weeks, 2.54 mg/kg/4 weeks, 2.55 mg/kg/4 weeks, 2.56 mg/kg/4 weeks, 2.57 mg/kg/4 weeks, 2.58 mg/kg/4 weeks, 2.59 mg/kg/4 weeks, 2.6 mg/kg/4 weeks, 2.61 mg/kg/4 weeks, 2.62 mg/kg/4 weeks, 2.63 mg/kg/4 weeks, 2.64 mg/kg/4 weeks, 2.65 mg/kg/4 weeks, 2.66 mg/kg/4 weeks, 2.67 mg/kg/4 weeks, 2.68 mg/kg/4 weeks, 2.69 mg/kg/4 weeks, 2.7 mg/kg/4 weeks, 2.71 mg/kg/4 weeks, 2.72 mg/kg/4 weeks, 2.73 mg/kg/4 weeks, 2.74 mg/kg/4 weeks, 2.75 mg/kg/4 weeks, 2.76 mg/kg/4 weeks, 2.77 mg/kg/4 weeks, 2.78 mg/kg/4 weeks, 2.79 mg/kg/4 weeks, 2.8 mg/kg/4 weeks, 2.81 mg/kg/4 weeks, 2.82 mg/kg/4 weeks, 2.83 mg/kg/4 weeks, 2.84 mg/kg/4 weeks, 2.85 mg/kg/4 weeks, 2.86 mg/kg/4 weeks, 2.87 mg/kg/4 weeks, 2.88 mg/kg/4 weeks, 2.89 mg/kg/4 weeks, 2.9 mg/kg/4 weeks, 2.91 mg/kg/4 weeks, 2.92 mg/kg/4 weeks, 2.93 mg/kg/4 weeks, 2.94 mg/kg/4 weeks, 2.95 mg/kg/4 weeks, 2.96 mg/kg/4 weeks, 2.97 mg/kg/4 weeks, 2.98 mg/kg/4 weeks, 2.99 mg/kg/4 weeks, or 3 mg/kg/4 weeks. Alternatively, in a non-limiting embodiment, the IL-31 antagonist of the present disclosure may be administered at, for example, 0.1 mg/kg/6 weeks, 0.11 mg/kg/6 weeks, 0.12 mg/kg/6 weeks, 0.125 mg/kg/6 weeks, 0.13 mg/kg/6 weeks, 0.14 mg/kg/6 weeks, 0.15 mg/kg/6 weeks, 0.16 mg/kg/6 weeks, 0.17 mg/kg/6 weeks, 0.18 mg/kg/6 weeks, 0.19 mg/kg/6 weeks, 0.2 mg/kg/6 weeks, 0.21 mg/kg/6 weeks, 0.22 mg/kg/6 weeks, 0.23 mg/kg/6 weeks, 0.24 mg/kg/6 weeks, 0.25 mg/kg/6 weeks, 0.26 mg/kg/6 weeks, 0.27 mg/kg/6 weeks, 0.28 mg/kg/6 weeks, 0.29 mg/kg/6 weeks, 0.3 mg/kg/6 weeks, 0.31 mg/kg/6 weeks, 0.32 mg/kg/6 weeks, 0.33 mg/kg/6 weeks, 0.34 mg/kg/6 weeks, 0.35 mg/kg/6 weeks, 0.36 mg/kg/6 weeks, 0.37 mg/kg/6 weeks, 0.38 mg/kg/6 weeks, 0.39 mg/kg/6 weeks, 0.4 mg/kg/6 weeks, 0.41 mg/kg/6 weeks, 0.42 mg/kg/6 weeks, 0.43 mg/kg/6 weeks, 0.44 mg/kg/6 weeks, 0.45 mg/kg/6 weeks, 0.46 mg/kg/6 weeks, 0.47 mg/kg/6 weeks, 0.48 mg/kg/6 weeks, 0.49 mg/kg/6 weeks, 0.5 mg/kg/6 weeks, 0.51 mg/kg/6 weeks, 0.52 mg/kg/6 weeks, 0.53 mg/kg/6 weeks, 0.54 mg/kg/6 weeks, 0.55 mg/kg/6 weeks, 0.56 mg/kg/6 weeks, 0.57 mg/kg/6 weeks, 0.58 mg/kg/6 weeks, 0.59 mg/kg/6 weeks, 0.6 mg/kg/6 weeks, 0.61 mg/kg/6 weeks, 0.62 mg/kg/6 weeks, 0.63 mg/kg/6 weeks, 0.64 mg/kg/6 weeks, 0.65 mg/kg/6 weeks, 0.66 mg/kg/6 weeks, 0.67 mg/kg/6 weeks, 0.68 mg/kg/6 weeks, 0.69 mg/kg/6 weeks, 0.7 mg/kg/6 weeks, 0.71 mg/kg/6 weeks, 0.72 mg/kg/6 weeks, 0.73 mg/kg/6 weeks, 0.74 mg/kg/6 weeks, 0.75 mg/kg/6 weeks, 0.76 mg/kg/6 weeks, 0.77 mg/kg/6 weeks, 0.78 mg/kg/6 weeks, 0.79 mg/kg/6 weeks, 0.8 mg/kg/6 weeks, 0.81 mg/kg/6 weeks, 0.82 mg/kg/6 weeks, 0.83 mg/kg/6 weeks, 0.84 mg/kg/6 weeks, 0.85 mg/kg/6 weeks, 0.86 mg/kg/6 weeks, 0.87 mg/kg/6 weeks, 0.88 mg/kg/6 weeks, 0.89 mg/kg/6 weeks, 0.9 mg/kg/6 weeks, 0.91 mg/kg/6 weeks, 0.92 mg/kg/6 weeks, 0.93 mg/kg/6 weeks, 0.94 mg/kg/6 weeks, 0.95 mg/kg/6 weeks, 0.96 mg/kg/6 weeks, 0.97 mg/kg/6 weeks, 0.98 mg/kg/6 weeks, 0.99 mg/kg/6 weeks, 1 mg/kg/6 weeks, 1.01 mg/kg/6 weeks, 1.02 mg/kg/6 weeks, 1.03 mg/kg/6 weeks, 1.04 mg/kg/6 weeks, 1.05 mg/kg/6 weeks, 1.06 mg/kg/6 weeks, 1.07 mg/kg/6 weeks, 1.08 mg/kg/6 weeks, 1.09 mg/kg/6 weeks, 1.1 mg/kg/6 weeks, 1.11 mg/kg/6 weeks, 1.12 mg/kg/6 weeks, 1.13 mg/kg/6 weeks, 1.14 mg/kg/6 weeks, 1.15 mg/kg/6 weeks, 1.16 mg/kg/6 weeks, 1.17 mg/kg/6 weeks, 1.18 mg/kg/6 weeks, 1.19 mg/kg/6 weeks, 1.2 mg/kg/6 weeks, 1.21 mg/kg/6 weeks, 1.22 mg/kg/6 weeks, 1.23 mg/kg/6 weeks, 1.24 mg/kg/6 weeks, 1.25 mg/kg/6 weeks, 1.26 mg/kg/6 weeks, 1.27 mg/kg/6 weeks, 1.28 mg/kg/6 weeks, 1.29 mg/kg/6 weeks, 1.3 mg/kg/6 weeks, 1.31 mg/kg/6 weeks, 1.32 mg/kg/6 weeks, 1.33 mg/kg/6 weeks, 1.34 mg/kg/6 weeks, 1.35 mg/kg/6 weeks, 1.36 mg/kg/6 weeks, 1.37 mg/kg/6 weeks, 1.38 mg/kg/6 weeks, 1.39 mg/kg/6 weeks, 1.4 mg/kg/6 weeks, 1.41 mg/kg/6 weeks, 1.42 mg/kg/6 weeks, 1.43 mg/kg/6 weeks, 1.44 mg/kg/6 weeks, 1.45 mg/kg/6 weeks, 1.46 mg/kg/6 weeks, 1.47 mg/kg/6 weeks, 1.48 mg/kg/6 weeks, 1.49 mg/kg/6 weeks, 1.5 mg/kg/6 weeks, 1.51 mg/kg/6 weeks, 1.52 mg/kg/6 weeks, 1.53 mg/kg/6 weeks, 1.54 mg/kg/6 weeks, 1.55 mg/kg/6 weeks, 1.56 mg/kg/6 weeks, 1.57 mg/kg/6 weeks, 1.58 mg/kg/6 weeks, 1.59 mg/kg/6 weeks, 1.6 mg/kg/6 weeks, 1.61 mg/kg/6 weeks, 1.62 mg/kg/6 weeks, 1.63 mg/kg/6 weeks, 1.64 mg/kg/6 weeks, 1.65 mg/kg/6 weeks, 1.66 mg/kg/6 weeks, 1.67 mg/kg/6 weeks, 1.68 mg/kg/6 weeks, 1.69 mg/kg/6 weeks, 1.7 mg/kg/6 weeks, 1.71 mg/kg/6 weeks, 1.72 mg/kg/6 weeks, 1.73 mg/kg/6 weeks, 1.74 mg/kg/6 weeks, 1.75 mg/kg/6 weeks, 1.76 mg/kg/6 weeks, 1.77 mg/kg/6 weeks, 1.78 mg/kg/6 weeks, 1.79 mg/kg/6 weeks, 1.8 mg/kg/6 weeks, 1.81 mg/kg/6 weeks, 1.82 mg/kg/6 weeks, 1.83 mg/kg/6 weeks, 1.84 mg/kg/6 weeks, 1.85 mg/kg/6 weeks, 1.86 mg/kg/6 weeks, 1.87 mg/kg/6 weeks, 1.88 mg/kg/6 weeks, 1.89 mg/kg/6 weeks, 1.9 mg/kg/6 weeks, 1.91 mg/kg/6 weeks, 1.92 mg/kg/6 weeks, 1.93 mg/kg/6 weeks, 1.94 mg/kg/6 weeks, 1.95 mg/kg/6 weeks, 1.96 mg/kg/6 weeks, 1.97 mg/kg/6 weeks, 1.98 mg/kg/6 weeks, 1.99 mg/kg/6 weeks, 2 mg/kg/6 weeks, 2.01 mg/kg/6 weeks, 2.02 mg/kg/6 weeks, 2.03 mg/kg/6 weeks, 2.04 mg/kg/6 weeks, 2.05 mg/kg/6 weeks, 2.06 mg/kg/6 weeks, 2.07 mg/kg/6 weeks, 2.08 mg/kg/6 weeks, 2.09 mg/kg/6 weeks, 2.1 mg/kg/6 weeks, 2.11 mg/kg/6 weeks, 2.12 mg/kg/6 weeks, 2.13 mg/kg/6 weeks, 2.14 mg/kg/6 weeks, 2.15 mg/kg/6 weeks, 2.16 mg/kg/6 weeks, 2.17 mg/kg/6 weeks, 2.18 mg/kg/6 weeks, 2.19 mg/kg/6 weeks, 2.2 mg/kg/6 weeks, 2.21 mg/kg/6 weeks, 2.22 mg/kg/6 weeks, 2.23 mg/kg/6 weeks, 2.24 mg/kg/6 weeks, 2.25 mg/kg/6 weeks, 2.26 mg/kg/6 weeks, 2.27 mg/kg/6 weeks, 2.28 mg/kg/6 weeks, 2.29 mg/kg/6 weeks, 2.3 mg/kg/6 weeks, 2.31 mg/kg/6 weeks, 2.32 mg/kg/6 weeks, 2.33 mg/kg/6 weeks, 2.34 mg/kg/6 weeks, 2.35 mg/kg/6 weeks, 2.36 mg/kg/6 weeks, 2.37 mg/kg/6 weeks, 2.38 mg/kg/6 weeks, 2.39 mg/kg/6 weeks, 2.4 mg/kg/6 weeks, 2.41 mg/kg/6 weeks, 2.42 mg/kg/6 weeks, 2.43 mg/kg/6 weeks, 2.44 mg/kg/6 weeks, 2.45 mg/kg/6 weeks, 2.46 mg/kg/6 weeks, 2.47 mg/kg/6 weeks, 2.48 mg/kg/6 weeks, 2.49 mg/kg/6 weeks, 2.5 mg/kg/6 weeks, 2.51 mg/kg/6 weeks, 2.52 mg/kg/6 weeks, 2.53 mg/kg/6 weeks, 2.54 mg/kg/6 weeks, 2.55 mg/kg/6 weeks, 2.56 mg/kg/6 weeks, 2.57 mg/kg/6 weeks, 2.58 mg/kg/6 weeks, 2.59 mg/kg/6 weeks, 2.6 mg/kg/6 weeks, 2.61 mg/kg/6 weeks, 2.62 mg/kg/6 weeks, 2.63 mg/kg/6 weeks, 2.64 mg/kg/6 weeks, 2.65 mg/kg/6 weeks, 2.66 mg/kg/6 weeks, 2.67 mg/kg/6 weeks, 2.68 mg/kg/6 weeks, 2.69 mg/kg/6 weeks, 2.7 mg/kg/6 weeks, 2.71 mg/kg/6 weeks, 2.72 mg/kg/6 weeks, 2.73 mg/kg/6 weeks, 2.74 mg/kg/6 weeks, 2.75 mg/kg/6 weeks, 2.76 mg/kg/6 weeks, 2.77 mg/kg/6 weeks, 2.78 mg/kg/6 weeks, 2.79 mg/kg/6 weeks, 2.8 mg/kg/6 weeks, 2.81 mg/kg/6 weeks, 2.82 mg/kg/6 weeks, 2.83 mg/kg/6 weeks, 2.84 mg/kg/6 weeks, 2.85 mg/kg/6 weeks, 2.86 mg/kg/6 weeks, 2.87 mg/kg/6 weeks, 2.88 mg/kg/6 weeks, 2.89 mg/kg/6 weeks, 2.9 mg/kg/6 weeks, 2.91 mg/kg/6 weeks, 2.92 mg/kg/6 weeks, 2.93 mg/kg/6 weeks, 2.94 mg/kg/6 weeks, 2.95 mg/kg/6 weeks, 2.96 mg/kg/6 weeks, 2.97 mg/kg/6 weeks, 2.98 mg/kg/6 weeks, 2.99 mg/kg/6 weeks, or 3 mg/kg/6 weeks. Alternatively, in a non-limiting embodiment, the IL-31 antagonist of the present disclosure may be administered at, for example, 0.1 mg/kg/8 weeks, 0.11 mg/kg/8 weeks, 0.12 mg/kg/8 weeks, 0.125 mg/kg/8 weeks, 0.13 mg/kg/8 weeks, 0.14 mg/kg/8 weeks, 0.15 mg/kg/8 weeks, 0.16 mg/kg/8 weeks, 0.17 mg/kg/8 weeks, 0.18 mg/kg/8 weeks, 0.19 mg/kg/8 weeks, 0.2 mg/kg/8 weeks, 0.21 mg/kg/8 weeks, 0.22 mg/kg/8 weeks, 0.23 mg/kg/8 weeks, 0.24 mg/kg/8 weeks, 0.25 mg/kg/8 weeks, 0.26 mg/kg/8 weeks, 0.27 mg/kg/8 weeks, 0.28 mg/kg/8 weeks, 0.29 mg/kg/8 weeks, 0.3 mg/kg/8 weeks, 0.31 mg/kg/8 weeks, 0.32 mg/kg/8 weeks, 0.33 mg/kg/8 weeks, 0.34 mg/kg/8 weeks, 0.35 mg/kg/8 weeks, 0.36 mg/kg/8 weeks, 0.37 mg/kg/8 weeks, 0.38 mg/kg/8 weeks, 0.39 mg/kg/8 weeks, 0.4 mg/kg/8 weeks, 0.41 mg/kg/8 weeks, 0.42 mg/kg/8 weeks, 0.43 mg/kg/8 weeks, 0.44 mg/kg/8 weeks, 0.45 mg/kg/8 weeks, 0.46 mg/kg/8 weeks, 0.47 mg/kg/8 weeks, 0.48 mg/kg/8 weeks, 0.49 mg/kg/8 weeks, 0.5 mg/kg/8 weeks, 0.51 mg/kg/8 weeks, 0.52 mg/kg/8 weeks, 0.53 mg/kg/8 weeks, 0.54 mg/kg/8 weeks, 0.55 mg/kg/8 weeks, 0.56 mg/kg/8 weeks, 0.57 mg/kg/8 weeks, 0.58 mg/kg/8 weeks, 0.59 mg/kg/8 weeks, 0.6 mg/kg/8 weeks, 0.61 mg/kg/8 weeks, 0.62 mg/kg/8 weeks, 0.63 mg/kg/8 weeks, 0.64 mg/kg/8 weeks, 0.65 mg/kg/8 weeks, 0.66 mg/kg/8 weeks, 0.67 mg/kg/8 weeks, 0.68 mg/kg/8 weeks, 0.69 mg/kg/8 weeks, 0.7 mg/kg/8 weeks, 0.71 mg/kg/8 weeks, 0.72 mg/kg/8 weeks, 0.73 mg/kg/8 weeks, 0.74 mg/kg/8 weeks, 0.75 mg/kg/8 weeks, 0.76 mg/kg/8 weeks, 0.77 mg/kg/8 weeks, 0.78 mg/kg/8 weeks, 0.79 mg/kg/8 weeks, 0.8 mg/kg/8 weeks, 0.81 mg/kg/8 weeks, 0.82 mg/kg/8 weeks, 0.83 mg/kg/8 weeks, 0.84 mg/kg/8 weeks, 0.85 mg/kg/8 weeks, 0.86 mg/kg/8 weeks, 0.87 mg/kg/8 weeks, 0.88 mg/kg/8 weeks, 0.89 mg/kg/8 weeks, 0.9 mg/kg/8 weeks, 0.91 mg/kg/8 weeks, 0.92 mg/kg/8 weeks, 0.93 mg/kg/8 weeks, 0.94 mg/kg/8 weeks, 0.95 mg/kg/8 weeks, 0.96 mg/kg/8 weeks, 0.97 mg/kg/8 weeks, 0.98 mg/kg/8 weeks, 0.99 mg/kg/8 weeks, 1 mg/kg/8 weeks, 1.01 mg/kg/8 weeks, 1.02 mg/kg/8 weeks, 1.03 mg/kg/8 weeks, 1.04 mg/kg/8 weeks, 1.05 mg/kg/8 weeks, 1.06 mg/kg/8 weeks, 1.07 mg/kg/8 weeks, 1.08 mg/kg/8 weeks, 1.09 mg/kg/8 weeks, 1.1 mg/kg/8 weeks, 1.11 mg/kg/8 weeks, 1.12 mg/kg/8 weeks, 1.13 mg/kg/8 weeks, 1.14 mg/kg/8 weeks, 1.15 mg/kg/8 weeks, 1.16 mg/kg/8 weeks, 1.17 mg/kg/8 weeks, 1.18 mg/kg/8 weeks, 1.19 mg/kg/8 weeks, 1.2 mg/kg/8 weeks, 1.21 mg/kg/8 weeks, 1.22 mg/kg/8 weeks, 1.23 mg/kg/8 weeks, 1.24 mg/kg/8 weeks, 1.25 mg/kg/8 weeks, 1.26 mg/kg/8 weeks, 1.27 mg/kg/8 weeks, 1.28 mg/kg/8 weeks, 1.29 mg/kg/8 weeks, 1.3 mg/kg/8 weeks, 1.31 mg/kg/8 weeks, 1.32 mg/kg/8 weeks, 1.33 mg/kg/8 weeks, 1.34 mg/kg/8 weeks, 1.35 mg/kg/8 weeks, 1.36 mg/kg/8 weeks, 1.37 mg/kg/8 weeks, 1.38 mg/kg/8 weeks, 1.39 mg/kg/8 weeks, 1.4 mg/kg/8 weeks, 1.41 mg/kg/8 weeks, 1.42 mg/kg/8 weeks, 1.43 mg/kg/8 weeks, 1.44 mg/kg/8 weeks, 1.45 mg/kg/8 weeks, 1.46 mg/kg/8 weeks, 1.47 mg/kg/8 weeks, 1.48 mg/kg/8 weeks, 1.49 mg/kg/8 weeks, 1.5 mg/kg/8 weeks, 1.51 mg/kg/8 weeks, 1.52 mg/kg/8 weeks, 1.53 mg/kg/8 weeks, 1.54 mg/kg/8 weeks, 1.55 mg/kg/8 weeks, 1.56 mg/kg/8 weeks, 1.57 mg/kg/8 weeks, 1.58 mg/kg/8 weeks, 1.59 mg/kg/8 weeks, 1.6 mg/kg/8 weeks, 1.61 mg/kg/8 weeks, 1.62 mg/kg/8 weeks, 1.63 mg/kg/8 weeks, 1.64 mg/kg/8 weeks, 1.65 mg/kg/8 weeks, 1.66 mg/kg/8 weeks, 1.67 mg/kg/8 weeks, 1.68 mg/kg/8 weeks, 1.69 mg/kg/8 weeks, 1.7 mg/kg/8 weeks, 1.71 mg/kg/8 weeks, 1.72 mg/kg/8 weeks, 1.73 mg/kg/8 weeks, 1.74 mg/kg/8 weeks, 1.75 mg/kg/8 weeks, 1.76 mg/kg/8 weeks, 1.77 mg/kg/8 weeks, 1.78 mg/kg/8 weeks, 1.79 mg/kg/8 weeks, 1.8 mg/kg/8 weeks, 1.81 mg/kg/8 weeks, 1.82 mg/kg/8 weeks, 1.83 mg/kg/8 weeks, 1.84 mg/kg/8 weeks, 1.85 mg/kg/8 weeks, 1.86 mg/kg/8 weeks, 1.87 mg/kg/8 weeks, 1.88 mg/kg/8 weeks, 1.89 mg/kg/8 weeks, 1.9 mg/kg/8 weeks, 1.91 mg/kg/8 weeks, 1.92 mg/kg/8 weeks, 1.93 mg/kg/8 weeks, 1.94 mg/kg/8 weeks, 1.95 mg/kg/8 weeks, 1.96 mg/kg/8 weeks, 1.97 mg/kg/8 weeks, 1.98 mg/kg/8 weeks, 1.99 mg/kg/8 weeks, 2 mg/kg/8 weeks, 2.01 mg/kg/8 weeks, 2.02 mg/kg/8 weeks, 2.03 mg/kg/8 weeks, 2.04 mg/kg/8 weeks, 2.05 mg/kg/8 weeks, 2.06 mg/kg/8 weeks, 2.07 mg/kg/8 weeks, 2.08 mg/kg/8 weeks, 2.09 mg/kg/8 weeks, 2.1 mg/kg/8 weeks, 2.11 mg/kg/8 weeks, 2.12 mg/kg/8 weeks, 2.13 mg/kg/8 weeks, 2.14 mg/kg/8 weeks, 2.15 mg/kg/8 weeks, 2.16 mg/kg/8 weeks, 2.17 mg/kg/8 weeks, 2.18 mg/kg/8 weeks, 2.19 mg/kg/8 weeks, 2.2 mg/kg/8 weeks, 2.21 mg/kg/8 weeks, 2.22 mg/kg/8 weeks, 2.23 mg/kg/8 weeks, 2.24 mg/kg/8 weeks, 2.25 mg/kg/8 weeks, 2.26 mg/kg/8 weeks, 2.27 mg/kg/8 weeks, 2.28 mg/kg/8 weeks, 2.29 mg/kg/8 weeks, 2.3 mg/kg/8 weeks, 2.31 mg/kg/8 weeks, 2.32 mg/kg/8 weeks, 2.33 mg/kg/8 weeks, 2.34 mg/kg/8 weeks, 2.35 mg/kg/8 weeks, 2.36 mg/kg/8 weeks, 2.37 mg/kg/8 weeks, 2.38 mg/kg/8 weeks, 2.39 mg/kg/8 weeks, 2.4 mg/kg/8 weeks, 2.41 mg/kg/8 weeks, 2.42 mg/kg/8 weeks, 2.43 mg/kg/8 weeks, 2.44 mg/kg/8 weeks, 2.45 mg/kg/8 weeks, 2.46 mg/kg/8 weeks, 2.47 mg/kg/8 weeks, 2.48 mg/kg/8 weeks, 2.49 mg/kg/8 weeks, 2.5 mg/kg/8 weeks, 2.51 mg/kg/8 weeks, 2.52 mg/kg/8 weeks, 2.53 mg/kg/8 weeks, 2.54 mg/kg/8 weeks, 2.55 mg/kg/8 weeks, 2.56 mg/kg/8 weeks, 2.57 mg/kg/8 weeks, 2.58 mg/kg/8 weeks, 2.59 mg/kg/8 weeks, 2.6 mg/kg/8 weeks, 2.61 mg/kg/8 weeks, 2.62 mg/kg/8 weeks, 2.63 mg/kg/8 weeks, 2.64 mg/kg/8 weeks, 2.65 mg/kg/8 weeks, 2.66 mg/kg/8 weeks, 2.67 mg/kg/8 weeks, 2.68 mg/kg/8 weeks, 2.69 mg/kg/8 weeks, 2.7 mg/kg/8 weeks, 2.71 mg/kg/8 weeks, 2.72 mg/kg/8 weeks, 2.73 mg/kg/8 weeks, 2.74 mg/kg/8 weeks, 2.75 mg/kg/8 weeks, 2.76 mg/kg/8 weeks, 2.77 mg/kg/8 weeks, 2.78 mg/kg/8 weeks, 2.79 mg/kg/8 weeks, 2.8 mg/kg/8 weeks, 2.81 mg/kg/8 weeks, 2.82 mg/kg/8 weeks, 2.83 mg/kg/8 weeks, 2.84 mg/kg/8 weeks, 2.85 mg/kg/8 weeks, 2.86 mg/kg/8 weeks, 2.87 mg/kg/8 weeks, 2.88 mg/kg/8 weeks, 2.89 mg/kg/8 weeks, 2.9 mg/kg/8 weeks, 2.91 mg/kg/8 weeks, 2.92 mg/kg/8 weeks, 2.93 mg/kg/8 weeks, 2.94 mg/kg/8 weeks, 2.95 mg/kg/8 weeks, 2.96 mg/kg/8 weeks, 2.97 mg/kg/8 weeks, 2.98 mg/kg/8 weeks, 2.99 mg/kg/8 weeks, or 3 mg/kg/8 weeks. Alternatively, in a non-limiting embodiment, the IL-31 antagonist of the present disclosure may be administered at, for example, 0.1 mg/kg/10 weeks, 0.11 mg/kg/10 weeks, 0.12 mg/kg/10 weeks, 0.125 mg/kg/10 weeks, 0.13 mg/kg/10 weeks, 0.14 mg/kg/10 weeks, 0.15 mg/kg/10 weeks, 0.16 mg/kg/10 weeks, 0.17 mg/kg/10 weeks, 0.18 mg/kg/10 weeks, 0.19 mg/kg/10 weeks, 0.2 mg/kg/10 weeks, 0.21 mg/kg/10 weeks, 0.22 mg/kg/10 weeks, 0.23 mg/kg/10 weeks, 0.24 mg/kg/10 weeks, 0.25 mg/kg/10 weeks, 0.26 mg/kg/10 weeks, 0.27 mg/kg/10 weeks, 0.28 mg/kg/10 weeks, 0.29 mg/kg/10 weeks, 0.3 mg/kg/10 weeks, 0.31 mg/kg/10 weeks, 0.32 mg/kg/10 weeks, 0.33 mg/kg/10 weeks, 0.34 mg/kg/10 weeks, 0.35 mg/kg/10 weeks, 0.36 mg/kg/10 weeks, 0.37 mg/kg/10 weeks, 0.38 mg/kg/10 weeks, 0.39 mg/kg/10 weeks, 0.4 mg/kg/10 weeks, 0.41 mg/kg/10 weeks, 0.42 mg/kg/10 weeks, 0.43 mg/kg/10 weeks, 0.44 mg/kg/10 weeks, 0.45 mg/kg/10 weeks, 0.46 mg/kg/10 weeks, 0.47 mg/kg/10 weeks, 0.48 mg/kg/10 weeks, 0.49 mg/kg/10 weeks, 0.5 mg/kg/10 weeks, 0.51 mg/kg/10 weeks, 0.52 mg/kg/10 weeks, 0.53 mg/kg/10 weeks, 0.54 mg/kg/10 weeks, 0.55 mg/kg/10 weeks, 0.56 mg/kg/10 weeks, 0.57 mg/kg/10 weeks, 0.58 mg/kg/10 weeks, 0.59 mg/kg/10 weeks, 0.6 mg/kg/10 weeks, 0.61 mg/kg/10 weeks, 0.62 mg/kg/10 weeks, 0.63 mg/kg/10 weeks, 0.64 mg/kg/10 weeks, 0.65 mg/kg/10 weeks, 0.66 mg/kg/10 weeks, 0.67 mg/kg/10 weeks, 0.68 mg/kg/10 weeks, 0.69 mg/kg/10 weeks, 0.7 mg/kg/10 weeks, 0.71 mg/kg/10 weeks, 0.72 mg/kg/10 weeks, 0.73 mg/kg/10 weeks, 0.74 mg/kg/10 weeks, 0.75 mg/kg/10 weeks, 0.76 mg/kg/10 weeks, 0.77 mg/kg/10 weeks, 0.78 mg/kg/10 weeks, 0.79 mg/kg/10 weeks, 0.8 mg/kg/10 weeks, 0.81 mg/kg/10 weeks, 0.82 mg/kg/10 weeks, 0.83 mg/kg/10 weeks, 0.84 mg/kg/10 weeks, 0.85 mg/kg/10 weeks, 0.86 mg/kg/10 weeks, 0.87 mg/kg/10 weeks, 0.88 mg/kg/10 weeks, 0.89 mg/kg/10 weeks, 0.9 mg/kg/10 weeks, 0.91 mg/kg/10 weeks, 0.92 mg/kg/10 weeks, 0.93 mg/kg/10 weeks, 0.94 mg/kg/10 weeks, 0.95 mg/kg/10 weeks, 0.96 mg/kg/10 weeks, 0.97 mg/kg/10 weeks, 0.98 mg/kg/10 weeks, 0.99 mg/kg/10 weeks, 1 mg/kg/10 weeks, 1.01 mg/kg/10 weeks, 1.02 mg/kg/10 weeks, 1.03 mg/kg/10 weeks, 1.04 mg/kg/10 weeks, 1.05 mg/kg/10 weeks, 1.06 mg/kg/10 weeks, 1.07 mg/kg/10 weeks, 1.08 mg/kg/10 weeks, 1.09 mg/kg/10 weeks, 1.1 mg/kg/10 weeks, 1.11 mg/kg/10 weeks, 1.12 mg/kg/10 weeks, 1.13 mg/kg/10 weeks, 1.14 mg/kg/10 weeks, 1.15 mg/kg/10 weeks, 1.16 mg/kg/10 weeks, 1.17 mg/kg/10 weeks, 1.18 mg/kg/10 weeks, 1.19 mg/kg/10 weeks, 1.2 mg/kg/10 weeks, 1.21 mg/kg/10 weeks, 1.22 mg/kg/10 weeks, 1.23 mg/kg/10 weeks, 1.24 mg/kg/10 weeks, 1.25 mg/kg/10 weeks, 1.26 mg/kg/10 weeks, 1.27 mg/kg/10 weeks, 1.28 mg/kg/10 weeks, 1.29 mg/kg/10 weeks, 1.3 mg/kg/10 weeks, 1.31 mg/kg/10 weeks, 1.32 mg/kg/10 weeks, 1.33 mg/kg/10 weeks, 1.34 mg/kg/10 weeks, 1.35 mg/kg/10 weeks, 1.36 mg/kg/10 weeks, 1.37 mg/kg/10 weeks, 1.38 mg/kg/10 weeks, 1.39 mg/kg/10 weeks, 1.4 mg/kg/10 weeks, 1.41 mg/kg/10 weeks, 1.42 mg/kg/10 weeks, 1.43 mg/kg/10 weeks, 1.44 mg/kg/10 weeks, 1.45 mg/kg/10 weeks, 1.46 mg/kg/10 weeks, 1.47 mg/kg/10 weeks, 1.48 mg/kg/10 weeks, 1.49 mg/kg/10 weeks, 1.5 mg/kg/10 weeks, 1.51 mg/kg/10 weeks, 1.52 mg/kg/10 weeks, 1.53 mg/kg/10 weeks, 1.54 mg/kg/10 weeks, 1.55 mg/kg/10 weeks, 1.56 mg/kg/10 weeks, 1.57 mg/kg/10 weeks, 1.58 mg/kg/10 weeks, 1.59 mg/kg/10 weeks, 1.6 mg/kg/10 weeks, 1.61 mg/kg/10 weeks, 1.62 mg/kg/10 weeks, 1.63 mg/kg/10 weeks, 1.64 mg/kg/10 weeks, 1.65 mg/kg/10 weeks, 1.66 mg/kg/10 weeks, 1.67 mg/kg/10 weeks, 1.68 mg/kg/10 weeks, 1.69 mg/kg/10 weeks, 1.7 mg/kg/10 weeks, 1.71 mg/kg/10 weeks, 1.72 mg/kg/10 weeks, 1.73 mg/kg/10 weeks, 1.74 mg/kg/10 weeks, 1.75 mg/kg/10 weeks, 1.76 mg/kg/10 weeks, 1.77 mg/kg/10 weeks, 1.78 mg/kg/10 weeks, 1.79 mg/kg/10 weeks, 1.8 mg/kg/10 weeks, 1.81 mg/kg/10 weeks, 1.82 mg/kg/10 weeks, 1.83 mg/kg/10 weeks, 1.84 mg/kg/10 weeks, 1.85 mg/kg/10 weeks, 1.86 mg/kg/10 weeks, 1.87 mg/kg/10 weeks, 1.88 mg/kg/10 weeks, 1.89 mg/kg/10 weeks, 1.9 mg/kg/10 weeks, 1.91 mg/kg/10 weeks, 1.92 mg/kg/10 weeks, 1.93 mg/kg/10 weeks, 1.94 mg/kg/10 weeks, 1.95 mg/kg/10 weeks, 1.96 mg/kg/10 weeks, 1.97 mg/kg/10 weeks, 1.98 mg/kg/10 weeks, 1.99 mg/kg/10 weeks, 2 mg/kg/10 weeks, 2.01 mg/kg/10 weeks, 2.02 mg/kg/10 weeks, 2.03 mg/kg/10 weeks, 2.04 mg/kg/10 weeks, 2.05 mg/kg/10 weeks, 2.06 mg/kg/10 weeks, 2.07 mg/kg/10 weeks, 2.08 mg/kg/10 weeks, 2.09 mg/kg/10 weeks, 2.1 mg/kg/10 weeks, 2.11 mg/kg/10 weeks, 2.12 mg/kg/10 weeks, 2.13 mg/kg/10 weeks, 2.14 mg/kg/10 weeks, 2.15 mg/kg/10 weeks, 2.16 mg/kg/10 weeks, 2.17 mg/kg/10 weeks, 2.18 mg/kg/10 weeks, 2.19 mg/kg/10 weeks, 2.2 mg/kg/10 weeks, 2.21 mg/kg/10 weeks, 2.22 mg/kg/10 weeks, 2.23 mg/kg/10 weeks, 2.24 mg/kg/10 weeks, 2.25 mg/kg/10 weeks, 2.26 mg/kg/10 weeks, 2.27 mg/kg/10 weeks, 2.28 mg/kg/10 weeks, 2.29 mg/kg/10 weeks, 2.3 mg/kg/10 weeks, 2.31 mg/kg/10 weeks, 2.32 mg/kg/10 weeks, 2.33 mg/kg/10 weeks, 2.34 mg/kg/10 weeks, 2.35 mg/kg/10 weeks, 2.36 mg/kg/10 weeks, 2.37 mg/kg/10 weeks, 2.38 mg/kg/10 weeks, 2.39 mg/kg/10 weeks, 2.4 mg/kg/10 weeks, 2.41 mg/kg/10 weeks, 2.42 mg/kg/10 weeks, 2.43 mg/kg/10 weeks, 2.44 mg/kg/10 weeks, 2.45 mg/kg/10 weeks, 2.46 mg/kg/10 weeks, 2.47 mg/kg/10 weeks, 2.48 mg/kg/10 weeks, 2.49 mg/kg/10 weeks, 2.5 mg/kg/10 weeks, 2.51 mg/kg/10 weeks, 2.52 mg/kg/10 weeks, 2.53 mg/kg/10 weeks, 2.54 mg/kg/10 weeks, 2.55 mg/kg/10 weeks, 2.56 mg/kg/10 weeks, 2.57 mg/kg/10 weeks, 2.58 mg/kg/10 weeks, 2.59 mg/kg/10 weeks, 2.6 mg/kg/10 weeks, 2.61 mg/kg/10 weeks, 2.62 mg/kg/10 weeks, 2.63 mg/kg/10 weeks, 2.64 mg/kg/10 weeks, 2.65 mg/kg/10 weeks, 2.66 mg/kg/10 weeks, 2.67 mg/kg/10 weeks, 2.68 mg/kg/10 weeks, 2.69 mg/kg/10 weeks, 2.7 mg/kg/10 weeks, 2.71 mg/kg/10 weeks, 2.72 mg/kg/10 weeks, 2.73 mg/kg/10 weeks, 2.74 mg/kg/10 weeks, 2.75 mg/kg/10 weeks, 2.76 mg/kg/10 weeks, 2.77 mg/kg/10 weeks, 2.78 mg/kg/10 weeks, 2.79 mg/kg/10 weeks, 2.8 mg/kg/10 weeks, 2.81 mg/kg/10 weeks, 2.82 mg/kg/10 weeks, 2.83 mg/kg/10 weeks, 2.84 mg/kg/10 weeks, 2.85 mg/kg/10 weeks, 2.86 mg/kg/10 weeks, 2.87 mg/kg/10 weeks, 2.88 mg/kg/10 weeks, 2.89 mg/kg/10 weeks, 2.9 mg/kg/10 weeks, 2.91 mg/kg/10 weeks, 2.92 mg/kg/10 weeks, 2.93 mg/kg/10 weeks, 2.94 mg/kg/10 weeks, 2.95 mg/kg/10 weeks, 2.96 mg/kg/10 weeks, 2.97 mg/kg/10 weeks, 2.98 mg/kg/10 weeks, 2.99 mg/kg/10 weeks, or 3 mg/kg/10 weeks. Alternatively, in a non-limiting embodiment, the IL-31 antagonist of the present disclosure may be administered at, for example, 0.1 mg/kg/12 weeks, 0.11 mg/kg/12 weeks, 0.12 mg/kg/12 weeks, 0.125 mg/kg/12 weeks, 0.13 mg/kg/12 weeks, 0.14 mg/kg/12 weeks, 0.15 mg/kg/12 weeks, 0.16 mg/kg/12 weeks, 0.17 mg/kg/12 weeks, 0.18 mg/kg/12 weeks, 0.19 mg/kg/12 weeks, 0.2 mg/kg/12 weeks, 0.21 mg/kg/12 weeks, 0.22 mg/kg/12 weeks, 0.23 mg/kg/12 weeks, 0.24 mg/kg/12 weeks, 0.25 mg/kg/12 weeks, 0.26 mg/kg/12 weeks, 0.27 mg/kg/12 weeks, 0.28 mg/kg/12 weeks, 0.29 mg/kg/12 weeks, 0.3 mg/kg/12 weeks, 0.31 mg/kg/12 weeks, 0.32 mg/kg/12 weeks, 0.33 mg/kg/12 weeks, 0.34 mg/kg/12 weeks, 0.35 mg/kg/12 weeks, 0.36 mg/kg/12 weeks, 0.37 mg/kg/12 weeks, 0.38 mg/kg/12 weeks, 0.39 mg/kg/12 weeks, 0.4 mg/kg/12 weeks, 0.41 mg/kg/12 weeks, 0.42 mg/kg/12 weeks, 0.43 mg/kg/12 weeks, 0.44 mg/kg/12 weeks, 0.45 mg/kg/12 weeks, 0.46 mg/kg/12 weeks, 0.47 mg/kg/12 weeks, 0.48 mg/kg/12 weeks, 0.49 mg/kg/12 weeks, 0.5 mg/kg/12 weeks, 0.51 mg/kg/12 weeks, 0.52 mg/kg/12 weeks, 0.53 mg/kg/12 weeks, 0.54 mg/kg/12 weeks, 0.55 mg/kg/12 weeks, 0.56 mg/kg/12 weeks, 0.57 mg/kg/12 weeks, 0.58 mg/kg/12 weeks, 0.59 mg/kg/12 weeks, 0.6 mg/kg/12 weeks, 0.61 mg/kg/12 weeks, 0.62 mg/kg/12 weeks, 0.63 mg/kg/12 weeks, 0.64 mg/kg/12 weeks, 0.65 mg/kg/12 weeks, 0.66 mg/kg/12 weeks, 0.67 mg/kg/12 weeks, 0.68 mg/kg/12 weeks, 0.69 mg/kg/12 weeks, 0.7 mg/kg/12 weeks, 0.71 mg/kg/12 weeks, 0.72 mg/kg/12 weeks, 0.73 mg/kg/12 weeks, 0.74 mg/kg/12 weeks, 0.75 mg/kg/12 weeks, 0.76 mg/kg/12 weeks, 0.77 mg/kg/12 weeks, 0.78 mg/kg/12 weeks, 0.79 mg/kg/12 weeks, 0.8 mg/kg/12 weeks, 0.81 mg/kg/12 weeks, 0.82 mg/kg/12 weeks, 0.83 mg/kg/12 weeks, 0.84 mg/kg/12 weeks, 0.85 mg/kg/12 weeks, 0.86 mg/kg/12 weeks, 0.87 mg/kg/12 weeks, 0.88 mg/kg/12 weeks, 0.89 mg/kg/12 weeks, 0.9 mg/kg/12 weeks, 0.91 mg/kg/12 weeks, 0.92 mg/kg/12 weeks, 0.93 mg/kg/12 weeks, 0.94 mg/kg/12 weeks, 0.95 mg/kg/12 weeks, 0.96 mg/kg/12 weeks, 0.97 mg/kg/12 weeks, 0.98 mg/kg/12 weeks, 0.99 mg/kg/12 weeks, 1 mg/kg/12 weeks, 1.01 mg/kg/12 weeks, 1.02 mg/kg/12 weeks, 1.03 mg/kg/12 weeks, 1.04 mg/kg/12 weeks, 1.05 mg/kg/12 weeks, 1.06 mg/kg/12 weeks, 1.07 mg/kg/12 weeks, 1.08 mg/kg/12 weeks, 1.09 mg/kg/12 weeks, 1.1 mg/kg/12 weeks, 1.11 mg/kg/12 weeks, 1.12 mg/kg/12 weeks, 1.13 mg/kg/12 weeks, 1.14 mg/kg/12 weeks, 1.15 mg/kg/12 weeks, 1.16 mg/kg/12 weeks, 1.17 mg/kg/12 weeks, 1.18 mg/kg/12 weeks, 1.19 mg/kg/12 weeks, 1.2 mg/kg/12 weeks, 1.21 mg/kg/12 weeks, 1.22 mg/kg/12 weeks, 1.23 mg/kg/12 weeks, 1.24 mg/kg/12 weeks, 1.25 mg/kg/12 weeks, 1.26 mg/kg/12 weeks, 1.27 mg/kg/12 weeks, 1.28 mg/kg/12 weeks, 1.29 mg/kg/12 weeks, 1.3 mg/kg/12 weeks, 1.31 mg/kg/12 weeks, 1.32 mg/kg/12 weeks, 1.33 mg/kg/12 weeks, 1.34 mg/kg/12 weeks, 1.35 mg/kg/12 weeks, 1.36 mg/kg/12 weeks, 1.37 mg/kg/12 weeks, 1.38 mg/kg/12 weeks, 1.39 mg/kg/12 weeks, 1.4 mg/kg/12 weeks, 1.41 mg/kg/12 weeks, 1.42 mg/kg/12 weeks, 1.43 mg/kg/12 weeks, 1.44 mg/kg/12 weeks, 1.45 mg/kg/12 weeks, 1.46 mg/kg/12 weeks, 1.47 mg/kg/12 weeks, 1.48 mg/kg/12 weeks, 1.49 mg/kg/12 weeks, 1.5 mg/kg/12 weeks, 1.51 mg/kg/12 weeks, 1.52 mg/kg/12 weeks, 1.53 mg/kg/12 weeks, 1.54 mg/kg/12 weeks, 1.55 mg/kg/12 weeks, 1.56 mg/kg/12 weeks, 1.57 mg/kg/12 weeks, 1.58 mg/kg/12 weeks, 1.59 mg/kg/12 weeks, 1.6 mg/kg/12 weeks, 1.61 mg/kg/12 weeks, 1.62 mg/kg/12 weeks, 1.63 mg/kg/12 weeks, 1.64 mg/kg/12 weeks, 1.65 mg/kg/12 weeks, 1.66 mg/kg/12 weeks, 1.67 mg/kg/12 weeks, 1.68 mg/kg/12 weeks, 1.69 mg/kg/12 weeks, 1.7 mg/kg/12 weeks, 1.71 mg/kg/12 weeks, 1.72 mg/kg/12 weeks, 1.73 mg/kg/12 weeks, 1.74 mg/kg/12 weeks, 1.75 mg/kg/12 weeks, 1.76 mg/kg/12 weeks, 1.77 mg/kg/12 weeks, 1.78 mg/kg/12 weeks, 1.79 mg/kg/12 weeks, 1.8 mg/kg/12 weeks, 1.81 mg/kg/12 weeks, 1.82 mg/kg/12 weeks, 1.83 mg/kg/12 weeks, 1.84 mg/kg/12 weeks, 1.85 mg/kg/12 weeks, 1.86 mg/kg/12 weeks, 1.87 mg/kg/12 weeks, 1.88 mg/kg/12 weeks, 1.89 mg/kg/12 weeks, 1.9 mg/kg/12 weeks, 1.91 mg/kg/12 weeks, 1.92 mg/kg/12 weeks, 1.93 mg/kg/12 weeks, 1.94 mg/kg/12 weeks, 1.95 mg/kg/12 weeks, 1.96 mg/kg/12 weeks, 1.97 mg/kg/12 weeks, 1.98 mg/kg/12 weeks, 1.99 mg/kg/12 weeks, 2 mg/kg/12 weeks, 2.01 mg/kg/12 weeks, 2.02 mg/kg/12 weeks, 2.03 mg/kg/12 weeks, 2.04 mg/kg/12 weeks, 2.05 mg/kg/12 weeks, 2.06 mg/kg/12 weeks, 2.07 mg/kg/12 weeks, 2.08 mg/kg/12 weeks, 2.09 mg/kg/12 weeks, 2.1 mg/kg/12 weeks, 2.11 mg/kg/12 weeks, 2.12 mg/kg/12 weeks, 2.13 mg/kg/12 weeks, 2.14 mg/kg/12 weeks, 2.15 mg/kg/12 weeks, 2.16 mg/kg/12 weeks, 2.17 mg/kg/12 weeks, 2.18 mg/kg/12 weeks, 2.19 mg/kg/12 weeks, 2.2 mg/kg/12 weeks, 2.21 mg/kg/12 weeks, 2.22 mg/kg/12 weeks, 2.23 mg/kg/12 weeks, 2.24 mg/kg/12 weeks, 2.25 mg/kg/12 weeks, 2.26 mg/kg/12 weeks, 2.27 mg/kg/12 weeks, 2.28 mg/kg/12 weeks, 2.29 mg/kg/12 weeks, 2.3 mg/kg/12 weeks, 2.31 mg/kg/12 weeks, 2.32 mg/kg/12 weeks, 2.33 mg/kg/12 weeks, 2.34 mg/kg/12 weeks, 2.35 mg/kg/12 weeks, 2.36 mg/kg/12 weeks, 2.37 mg/kg/12 weeks, 2.38 mg/kg/12 weeks, 2.39 mg/kg/12 weeks, 2.4 mg/kg/12 weeks, 2.41 mg/kg/12 weeks, 2.42 mg/kg/12 weeks, 2.43 mg/kg/12 weeks, 2.44 mg/kg/12 weeks, 2.45 mg/kg/12 weeks, 2.46 mg/kg/12 weeks, 2.47 mg/kg/12 weeks, 2.48 mg/kg/12 weeks, 2.49 mg/kg/12 weeks, 2.5 mg/kg/12 weeks, 2.51 mg/kg/12 weeks, 2.52 mg/kg/12 weeks, 2.53 mg/kg/12 weeks, 2.54 mg/kg/12 weeks, 2.55 mg/kg/12 weeks, 2.56 mg/kg/12 weeks, 2.57 mg/kg/12 weeks, 2.58 mg/kg/12 weeks, 2.59 mg/kg/12 weeks, 2.6 mg/kg/12 weeks, 2.61 mg/kg/12 weeks, 2.62 mg/kg/12 weeks, 2.63 mg/kg/12 weeks, 2.64 mg/kg/12 weeks, 2.65 mg/kg/12 weeks, 2.66 mg/kg/12 weeks, 2.67 mg/kg/12 weeks, 2.68 mg/kg/12 weeks, 2.69 mg/kg/12 weeks, 2.7 mg/kg/12 weeks, 2.71 mg/kg/12 weeks, 2.72 mg/kg/12 weeks, 2.73 mg/kg/12 weeks, 2.74 mg/kg/12 weeks, 2.75 mg/kg/12 weeks, 2.76 mg/kg/12 weeks, 2.77 mg/kg/12 weeks, 2.78 mg/kg/12 weeks, 2.79 mg/kg/12 weeks, 2.8 mg/kg/12 weeks, 2.81 mg/kg/12 weeks, 2.82 mg/kg/12 weeks, 2.83 mg/kg/12 weeks, 2.84 mg/kg/12 weeks, 2.85 mg/kg/12 weeks, 2.86 mg/kg/12 weeks, 2.87 mg/kg/12 weeks, 2.88 mg/kg/12 weeks, 2.89 mg/kg/12 weeks, 2.9 mg/kg/12 weeks, 2.91 mg/kg/12 weeks, 2.92 mg/kg/12 weeks, 2.93 mg/kg/12 weeks, 2.94 mg/kg/12 weeks, 2.95 mg/kg/12 weeks, 2.96 mg/kg/12 weeks, 2.97 mg/kg/12 weeks, 2.98 mg/kg/12 weeks, 2.99 mg/kg/12 weeks, or 3 mg/kg/12 weeks.

When the IL-31 antagonist of the present disclosure is administered at the above-described predetermined dosing interval and predetermined dose (dosage) to a subject with or potentially with uremic pruritus repeatedly at the same dose and the same dosing interval, it can continuously suppress or improve uremic pruritus and possibly further the various symptoms caused by uremic pruritus (e.g., decrease in QOL, decrease in the quality of sleep, decrease in vital prognosis). The dosage and the dosing interval at which the IL-31 antagonist is repeatedly administered are determined, for example, in view of effect and safety.

In one embodiment, oral or parenteral administration can be selected as the method of administering the pharmaceutical composition of present disclosure to a subject. Typically, when the active ingredient is a low-molecular-weight compound, oral or parenteral administration may be selected, and when the active ingredient is a high-molecular-weight compound, parenteral administration is preferred, but not limited thereto. Examples of parenteral administration include injection, nasal, pulmonary, and percutaneous administrations. Additionally, examples of injections include intravenous, intramuscular, intraperitoneal, and subcutaneous injections. Using these methods of administration, the pharmaceutical composition of present disclosure can be systemically or topically administered.

In one embodiment, the pharmaceutical composition of the present disclosure can be prepared by combining the IL-31 antagonist as an active ingredient with pharmaceutically acceptable carriers. For example, the IL-31 antagonist may be combined, as appropriate, with pharmaceutically acceptable carriers or media such as sterilized water or saline solution, vegetable oils, emulsifiers, suspensions, surfactants, stabilizers, flavoring agents, excipients, vehicles, preservatives, and binders, for example, and formulated into a pharmaceutical preparation. Examples of carriers include light anhydrous silicic acid, lactose, crystalline cellulose, mannitol, starch, carmellose calcium, carmellose sodium, hydroxypropylcellulose, hydroxypropylmethylcellulose, polyvinylacetal diethylaminoacetate, polyvinyl pyrrolidone, gelatin, medium chain fatty acid triglycerides, polyoxyethylene hydrogenated castor oil 60, sucrose, carboxymethyl cellulose, corn starch, and inorganic salts. The amount of the active ingredient in these preparations can be set as appropriate within the designated range of doses.

In another embodiment, the present disclosure relates to a method for preventing and/or treating uremic pruritus comprising administering an IL-31 antagonist to a subject with or potentially with uremic pruritus.

In this case, the IL-31 antagonist may be administered to the subject with or potentially with uremic pruritus at 0.1 mg to 1000 mg/body/1 day to 12 weeks, preferably at 0.1 mg to 1000 mg/body/2 weeks, 0.1 mg to 1000 mg/body/4 weeks, or 0.1 mg to 1000 mg/body/8 weeks, repeatedly at the same dose and the same dosing interval. Alternatively, the IL-31 antagonist may be administered to the subject with or potentially with uremic pruritus at 0.01 mg to 10 mg/kg/1 day to 12 weeks, preferably at 0.01 mg to 10 mg/kg/2 weeks, 0.01 mg to 10 mg/kg/4 weeks, or 0.01 mg to 10 mg/kg/8 weeks, repeatedly at the same dose and the same dosing interval. In addition, the IL-31 antagonist may be administered to a subject with or potentially with uremic pruritus having a serum IL-31 concentration equal to or higher than a predetermined value.

In a specific embodiment, the prevention and/or treatment method of the present disclosure may further comprise selecting, before administering an IL-31 antagonist, a subject as a subject for the prevention/treatment if the IL-31 concentration in a serum obtained from the subject is equal to or higher than a predetermined value. Thus, the present disclosure provides a method for preventing and/or treating uremic pruritus, comprising selecting a subject if the IL-31 concentration in a serum obtained from the subject is equal to or higher than a predetermined value, and administering an IL-31 antagonist to the selected subject. This embodiment of the disclosure may further comprise, before the aforementioned selecting, measuring IL-31 concentration in a serum obtained from a subject with or potentially with uremic pruritus. Alternatively, the present disclosure may provide a method for preventing and/or treating uremic pruritus comprising measuring IL-31 concentration in a serum obtained from a subject with or potentially with uremic pruritus; determining the subject to be a responder to prevention and/or treatment with an IL-31 antagonist if the IL-31 concentration is equal to or higher than a predetermined value; and administering an IL-31 antagonist to the subject determined to be a responder. Alternatively, in the above embodiment, a subject with or potentially with uremic pruritus whose serum IL-31 concentration has been measured in advance may be determined to be a responder to prevention and/or treatment with an IL-31 antagonist if the IL-31 concentration is equal to or higher than a predetermined value.

In another embodiment, the present disclosure provides a product comprising at least (i) a container (e.g., an injection); (ii) a pharmaceutical composition comprising an IL-31 antagonist as an active ingredient within the container; and (iii) a document instructing that the IL-31 antagonist be administered at 0.1 mg to 1000 mg/body/1 day to 12 weeks or 0.01 mg to 10 mg/kg/1 day to 12 weeks to a subject with or potentially with uremic pruritus repeatedly at the same dose and the same dosing interval. Additionally, a label, a syringe, an injection needle, a pharmacologically acceptable medium, an alcohol cotton cloth, plaster, and the like may be packaged, as appropriate, with this product. The container may be a bottle, a glass bottle, or a syringe, for example, and may be made of any of various materials such as glass and plastics. The container contains the pharmaceutical composition, and has an outlet sealed with a rubber stopper, for example. The container is provided with, for example, a label indicating that the pharmaceutical composition is for use in preventing or treating a selected pathological condition.

All the technical documents cited herein are incorporated herein by reference in their entirety.

As used herein, the meaning of the term "and/or" is a combination of the terms used before and after the phrase "and/or", and is understood to include all combinations in which "and" and "or" are combined as appropriate.

The terms used herein are employed for illustrating specific embodiments, and should not be understood as limiting the invention. Unless different definitions are expressly described, the terms (including technical and scientific terms) used herein should be interpreted to have the same meanings as broadly understood by those skilled in the art to which the present disclosure pertains, and should not be interpreted in any idealized or excessively formal meaning.

The term "comprise", "comprises", or "comprising" as used herein is intended to mean the presence of the stated matter(s) (e.g., step(s), element(s), and number(s)), and does not exclude the presence of other matter(s) (e.g., step(s), element(s), and number(s)), unless it should be understood in clearly different ways in the context.

The embodiments of the present disclosure, which may be described with reference to schematic diagrams, may be expressed in an exaggerated manner for the sake of clear illustration.

A numerical value recited herein may be understood to have a certain range of variations in light of the common general knowledge in the art, unless it is contradictory in the context. For example, the recitation "1 mg" is understood to be recited as "about 1 mg", and is understood to include a certain range of variations based on the disclosure of the present specification and the common general knowledge in the art. Moreover, the recitation "1 to 5 times", for example, as used herein may be understood to recite "1, 2, 3, 4, or 5 times" as if to specifically and individually recite each value, unless it is contradictory in the context. Furthermore, the recitation "20, ... 25 times", for example, as used herein may be understood to recite "20, 21, 22, 23, 24, or 25 times" as if to specifically and individually recite each value, unless it is contradictory in the context. Moreover, the recitation " 1 to 5000 pg/mL", for example, as used herein may be understood to recite, for example, "1 pg/mL, 2 pg/mL, 3 pg/mL, 4 pg/mL, 5 pg/mL, 6 pg/mL, 7 pg/mL, 8 pg/mL, 9 pg/mL, 10 pg/mL, ... 15 pg/mL, ... 20 pg/mL, ... 25 pg/mL, ... 30 pg/mL, ... 35 pg/mL, ... 40 pg/mL, ... 45 pg/mL, ... 50 pg/mL, ... 55 pg/mL, ... 60 pg/mL, ... 65 pg/mL, ... 70 pg/mL, ... 75 pg/mL, ... 80 pg/mL, ... 85 pg/mL, ... 90 pg/mL, ... 95 pg/mL, ... 100 pg/mL, ... 150 pg/mL, ... 200 pg/mL, ... 250 pg/mL, ... 300 pg/mL, ... 350 pg/mL, ... 400 pg/mL, ... 450 pg/mL, ... 500 pg/mL, ... 600 pg/mL, ... 700 pg/mL, ... 800 pg/mL, ... 900 pg/mL, ... 1000 pg/mL, ... 2000 pg/mL, ... 3000 pg/mL, ... 4000 pg/mL, ... 5000 pg/mL", but not limited thereto, unless it is contradictory in the context. Furthermore, the recitation "10 pg/mL, ... 15 pg/mL", for example, as used herein may be understood to recite "10 pg/mL, 11 pg/mL, 12 pg/mL, 13 pg/mL, 14 pg/mL, or 15 pg/mL" as if it recites specifically and individually each value, unless it is contradictory in the context. Naturally, therefore, a person skilled in the art will directly and unambiguously understand that the recitation "1 to 5000 pg/mL", for example, is meant to specifically and individually recite values such as 100 pg/mL, 224 pg/ml, and 1500 pg/mL, for example. The same interpretation applies, as appropriate, to numerical values recited herein, unless it is contradictory in the context, and likewise, a person skilled in the art may naturally understand directly and unambiguously that each value is specifically and individually recited.

[Example]

The present disclosure will be more specifically described hereinafter with examples; however, the present disclosure is not limited to these examples.

[Example 1]

Preparation of an anti-human IL-31RA antibody

The anti-human IL-31RA antibody CIM331 (nemolizumab) (defined by the amino acid sequences of H chain: SEQ ID NO: 9; L chain: SEQ ID NO: 10) was prepared using a method known to those skilled in the art, in accordance with the disclosure of the aforementioned patent document. As disclosed in WO 2010/064697, CIM331 has neutralizing activity against human IL-31RA and cynomolgus monkey IL-31RA.

[Example 2]

In relation with a nonclinical study preceding the clinical study of CIM331, the following important fact to be noted was found. The antigen-antibody interaction of CIM331 with IL-31RA from each of mouse, rat, and rabbit was evaluated with Biacore (Biacore T100 (GE Healthcare)), using a method known to those skilled in the art. As a result, it was revealed that CIM331 does not exhibit cross-reactivity with IL-31RA from any of mouse, rat, and rabbit (Sakurai T, Esaki K. Cross-reactivity of CH5427227 with NR10 (IL-31RA) from mice, rats and rabbits (Study No. TOX08-0198S). Chugai Pharmaceutical Co., Ltd. In-house report, 2010.).

Thus, to verify the below-described effect concerning the dosage of CIM331 in humans and the dosing interval, even a person skilled in the art needed to actually administer CIM331 to humans, or needed to administer CIM331 to human models (e.g., cynomolgus monkeys) exhibiting cross-reactivity, and then predict the effect by extrapolating the results to humans.

[Example 3A]

Suppressive effect of subcutaneous administration of CIM331 on IL-31-induced pruritus in cynomolgus monkeys

The effect of subcutaneous administration of CIM331 on pruritus induced by intravenous administration of cynomolgus monkey IL-31 to cynomolgus monkeys was studied. The frequency of pruritic behavior was measured as an index of reactivity to pruritus. The frequency of pruritic behavior was measured visually by watching the video recordings (2 hours) of each monkey's behavior, and the movement of scratching a part of the body with a forelimb or hindlimb was counted as one occurrence of pruritic behavior. However, pruritic behaviors that ended in one or two scratching movements were excluded from the frequency of pruritic behavior because they were considered to be coincidental events.

First, before the administration of CIM331, the behavior of each animal without the administration of cynomolgus monkey IL-31 was recorded with a video camera (2 hours). Subsequently, the behavior of each animal was observed by playing the video, and the frequency of pruritic behavior without the administration of cynomolgus monkey IL-31 was measured using the above-described method.

A single subcutaneous dose of 0.2 or 1 mg/kg of CIM331 was administered to cynomolgus monkeys, and the frequency of pruritic behavior after the administration of cynomolgus monkey IL-31 was measured as follows, to evaluate the effect of subcutaneous administration of CIM331. A single subcutaneous dose of 0.2 mg/kg of CIM331 was administered to cynomolgus monkeys, and 1 $\mu$g/kg of cynomolgus monkey IL-31 was intravenously administered before the subcutaneous administration of CIM331 and on days 3, 15, 28, 42, 56, and 93 after the subcutaneous administration of CIM331. After the administration of cynomolgus monkey IL-31, the individual behavior was recorded with a video camera (2 hours). Likewise, a single subcutaneous dose of 1 mg/kg of CIM331 was administered to cynomolgus monkeys, and 1 $\mu$g/kg of cynomolgus monkey IL-31 was intravenously administered before the subcutaneous administration of CIM331 and on days 28, 42, 56, 77, 79, 81, 84, and 93 after the subcutaneous administration of CIM331. After the administration of cynomolgus monkey IL-31, the individual behavior was recorded with a video camera (2 hours). The individual behavior was subsequently observed by playing the video, and the frequency of pruritic behavior after the administration of cynomolgus monkey IL-31 was measured using the above-described method.

It was verified that the administration of cynomolgus monkey IL-31 before the administration of CIM331 induced pruritus in that it increased the frequency of pruritic behavior, compared to that before the administration of cynomolgus monkey IL-31. It was also verified that the administration of a single subcutaneous dose of CIM331 to cynomolgus monkeys reduced the frequency of pruritic behavior after the administration of cynomolgus monkey IL-31.

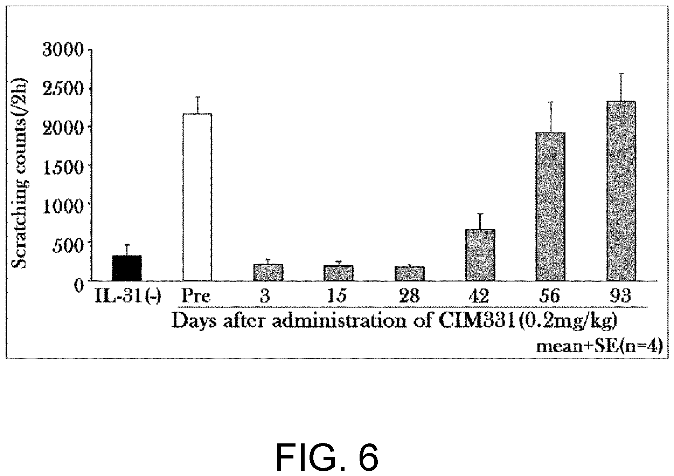

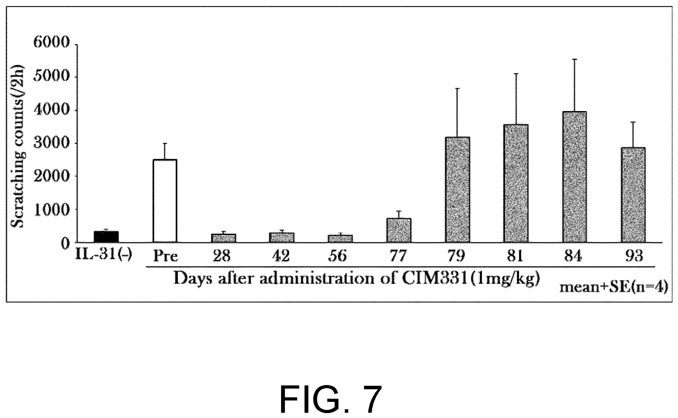

The administration of a single subcutaneous dose of 0.2 mg/kg of CIM331 to cynomolgus monkeys was shown to reduce the mean value of the frequency of pruritic behavior after the administration of cynomolgus monkey IL-31 in the evaluation on day 3 after the CIM331 administration, compared to that before the CIM331 administration; and was shown to reduce the mean value of the frequency of pruritic behavior after the administration of cynomolgus monkey IL-31 even on day 42 after the CIM331 administration (Figure 6). Furthermore, the administration of a single subcutaneous dose of 1 mg/kg of CIM331 was shown to reduce the mean value of the frequency of pruritic behavior after the administration of cynomolgus monkey IL-31, even on day 77 after the CIM331 administration (Figure 7).

For setting a dosage for humans, the effective plasma concentration of CIM331 was determined from the outcome of a study using an *in vivo* cynomolgus monkey IL-31-induced pruritus model in which systemic pruritus was induced by the administration of cynomolgus monkey IL-31 to a cynomolgus monkey. In this study, CIM331 was intravenously administered to the same cynomolgus monkey individual while gradually increasing the dosage from 3 to 100 $\mu$g/kg (3, 10, 40, 60, and 100 $\mu$g/kg) to increase the plasma concentration. On the day following the CIM331 administration in each stage, blood was collected to measure the plasma concentration of CIM331. Additionally, pruritic behavior induced by intravenous administration of 1 $\mu$g/kg of cynomolgus monkey IL-31 was recorded with a video camera for 2 hours after the administration, and the frequency of pruritic behavior was measured. To measure the frequency of pruritic behavior, the behavior of the monkey recorded with a video camera (2 hours) was visually observed, and the movement of scratching a part of the body with a forelimb or hindlimb was counted as one occurrence of pruritic behavior. However, pruritic behaviors that ended in one or two scratching movements were excluded from the frequency of pruritic behavior because they were considered to be coincidental events. By intravenously administering CIM331 while gradually increasing the dosage, the mean plasma concentration of CIM331 on the day following the CIM331 administration was gradually increased, depending on the dosage. CIM331 demonstrated an evident suppressive effect on cynomolgus monkey IL-31-induced pruritus subsequent to the administration of 40 $\mu$g/kg of CIM331 (the mean plasma concentration on the day following the administration was 670 ng/mL). A mean plasma concentration of 670 ng/mL was defined as the estimated effective serum concentration of CIM331 in humans. It has been reported that the *in vivo* pharmacokinetics of antibodies is similar between human and cynomolgus monkey (Jennifer Q. Dong et al., Quantitative Prediction of Human Pharmacokinetics for Monoclonal Antibodies. Clin Pharmacokinet 2011; 50(2):131-142; Jie Ling et al., Interspecies Scaling of Therapeutic Monoclonal Antibodies: Initial Look. J Clin Pharmacol 2009:49(12):1382-1402; Rong Deng et al., Projecting human pharmacokinetics of therapeutic antibodies from nonclinical data. mAbs 2011:3(1):61-66). Thus, PK parameters obtained by nonlinear analysis of data on changes in the plasma concentration of CIM331 in a cynomolgus monkey PK study were used as predicted values of PK parameters in humans. For the nonlinear analysis, a nonlinear analytical model adopting the Michaelis-Menten equation as shown in Figure 8 was used.

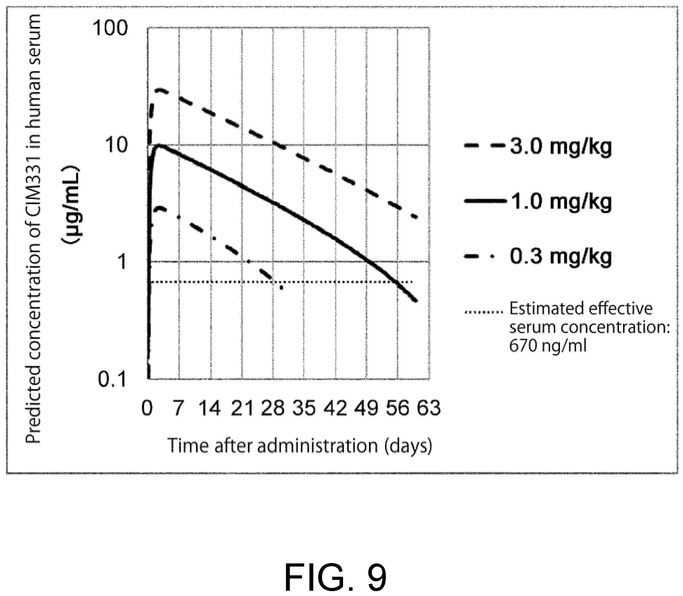

After intravenous and subcutaneous administration of CIM331 at 0.04 mg/kg, 0.2 mg/kg, and 1.0 mg/kg to cynomolgus monkeys, the mean values of changes in the plasma concentration of CIM331 for the respective groups were applied to the above-described model simultaneously, and optimal parameters were calculated. Using the obtained parameters, changes in the serum concentration that would result from the administration of CIM331 to humans were predicted. It was predicted that when 1 mg/kg of CIM331 was administered to humans, a serum concentration of CIM331 not lower than 670 ng/mL, i.e., the estimated effective serum concentration of CIM331 in humans, would be maintained for 56 days (Figure 9). A dose of 1 mg/kg, at which it is expected that CIM331 can reliably maintain the effect of inhibiting IL-31 signaling for a period of 1 month or longer, was defined as the expected clinical optimal dose.

[Example 3B]

Single subcutaneous administration to patients with atopic dermatitis

In test drug groups in a phase I single dose study, one of CIM331 dosages of 0.3 mg/kg, 1 mg/kg, and 3 mg/kg per body weight, and placebo was subcutaneously administered in a single dose into the abdomen of each of 36 patients with atopic dermatitis who met the following criteria, each group including 9 patients.

As the patients administered with CIM331, patients with atopic dermatitis were selected who met the following criteria although they underwent treatment with a topical steroid for a duration of 12 weeks or longer:

· An Eczema Area Severity Index score of 10 or more, and rash with intense inflammation affecting 5% or more of the body surface area.
· A total score of 4 or more in the evaluation of the degree of itchiness in the daytime and nighttime based on Shiratori's severity classification.
· A pruritus VAS mean value ≥ 50 mm.

For use as an investigational drug, a preparation was obtained by filling a vial with 1 mL of a solution containing 100 mg of the CIM331 antibody per milliliter, or by diluting the solution to an intended concentration for administration. Saline solution was used as the placebo.

(3-1) Endpoint: pruritus

The intensity of pruritus was evaluated using the Visual Analog Scale (VAS). The VAS consists of a 100-mm straight line, on which the patients themselves indicate the intensity of itchiness when awakening and when going to bed by drawing a line between 0 to 100 mm, where 0 mm represents no itchiness and 100 mm represents the severest itchiness that patients with atopic dermatitis experienced in the past. The patients kept records every day during the period of the study.

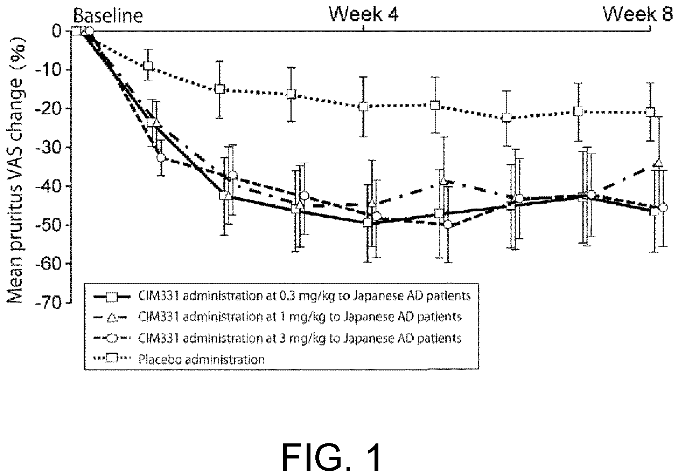

As a result, the placebo group showed a change in VAS that is approximately a 20% decrease, whereas all dose groups of the CIM331-administered groups showed a decrease in VAS from week 1 after the administration, and maintained approximately a 50% decrease even at week 4 after the administration and thereafter (Fig. 1).

(3-2) Endpoint: dermatitis

The Eczema Area Severity Index (EASI) score is a tool for assessing the severity and the range of atopic dermatitis. The extent and the proportion of eczema in representative affected areas were evaluated for each of the four areas, i.e., the head and neck, the upper limb, the trunk, and the lower limb, and the degrees of redness (erythema), thickness (induration, papules, and edema), excoriations (scratch marks), and lichenification were evaluated on a scale of (0) none, (1) mild, (2) moderate, and (3) severe. During the clinical study period, a doctor made assessments at a frequency of once in 1 or 2 weeks. Mean variations in the EASI score at week 4 after the administration from baseline were analyzed according to the percent decrease in the pruritus VAS score at week 4 after the administration (i.e., a group showing a percent decrease of less than 50% and a group showing a percent decrease of 50% or more).

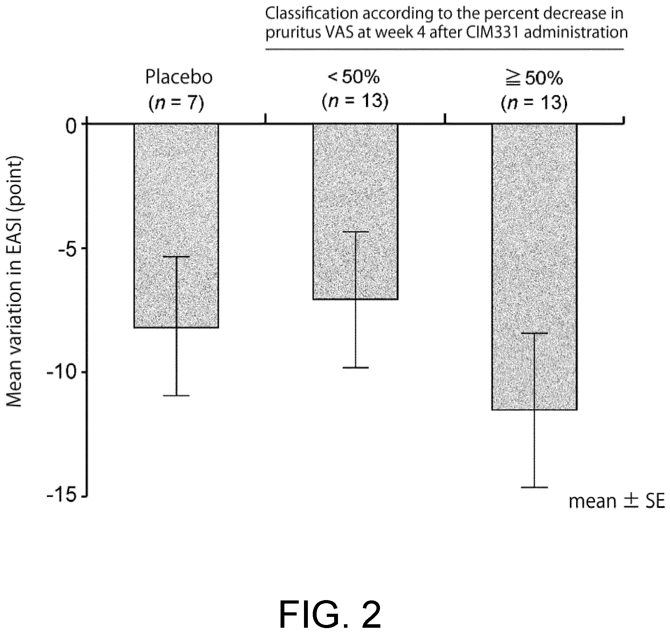

As a result, in the group showing a percent decrease of 50% or more in the pruritus VAS score, the mean variation in the EASI score was -11.5 points, and the decrease in the EASI score was greater than that in the placebo group or the group showing a percent decrease of less than 50% in the pruritus VAS score (Fig. 2).

(3-3) Endpoint: quality of life (OOL)

(3-3-1) Sleep

Actiwatch (registered trademark) is a noninvasive measurement device designed to be worn around a wrist, and capture, record, and store movements of the wrist that serve as an index of systemic movement while the user can behave freely. The subjects wore this device until week 4 after the administration. Other parameters including the actual time from falling asleep to awakening, sleep latency, and sleep efficiency were measured using an objective method. Sleep efficiency was calculated based on the following equation:

[Expression 1]

$$\text{Sleep Efficiency} = \frac{\text{Actual Sleep Time}}{\text{Time Lying in Bed}}$$

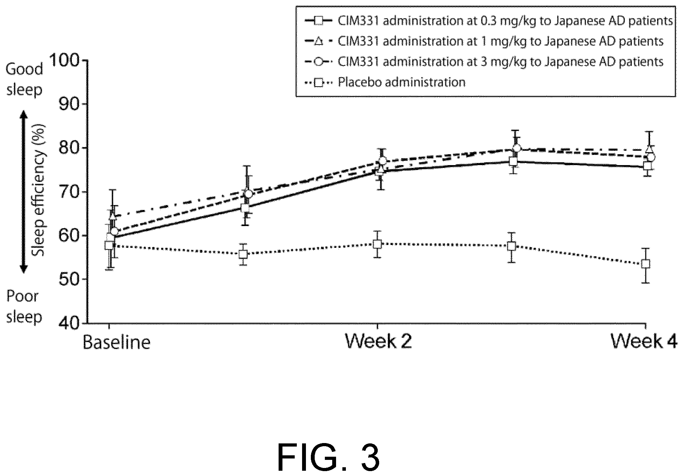

As a result, although the sleep efficiency was approximately 60% in all the groups before the administration, all dose groups of the CIM331-administered groups showed an improvement in sleep efficiency from week 1 after the administration, and showed an improvement up to approximately 80% at week 4 after the administration (Fig. 3).

(3-3-2) DLQI

The Dermatology Life Quality Index is a dermatologic tool DLQI for evaluating the QOL (Finlay et al. 1994), and consists of 10 questions. The DLQI questions can be grouped under the following six items: symptoms and feelings, daily activities, leisure, work and school, personal relationships, and treatment. The DLQI is determined by adding the scores for all the items of the questionnaire. The maximum score is 30, and the minimum score is 0. A higher score indicates lower QOL.

The patients kept records every 2 or 4 weeks. As a result, at week 4 after the administration, the placebo group showed a 0.7-point decrease on average, whereas the CIM331 groups showed a 5.4- to 6.3-point decrease on average.

(3-4) Endpoint: amount of topical steroid used

A topical steroid (Locoid (registered trademark); hydrocortisone butyrate) was used in combination in all the patients. The amount of the topical steroid used could be varied as appropriate, depending on the condition of the patient.

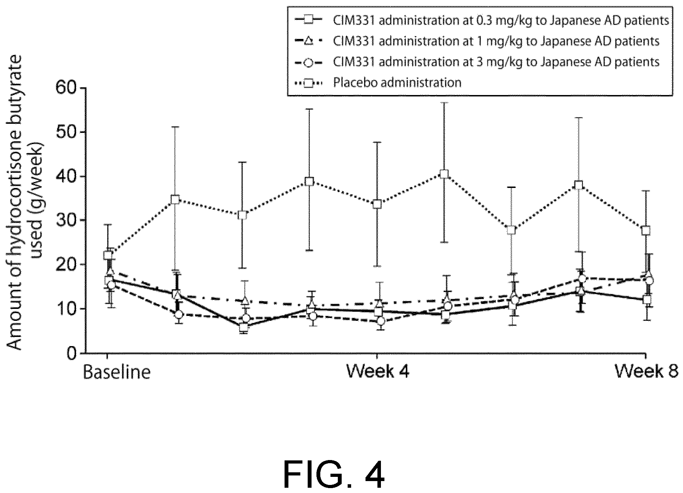

As a result, the amount of Locoid used tended to increase in the placebo-treated group, whereas the amount of Locoid used tended to decrease from week 1 after the administration in all dose groups of the CIM331-administered groups (Fig. 4).

(3-5) Endpoint: serum concentration time course of CIM331 and pharmacokinetic parameters of CIM331

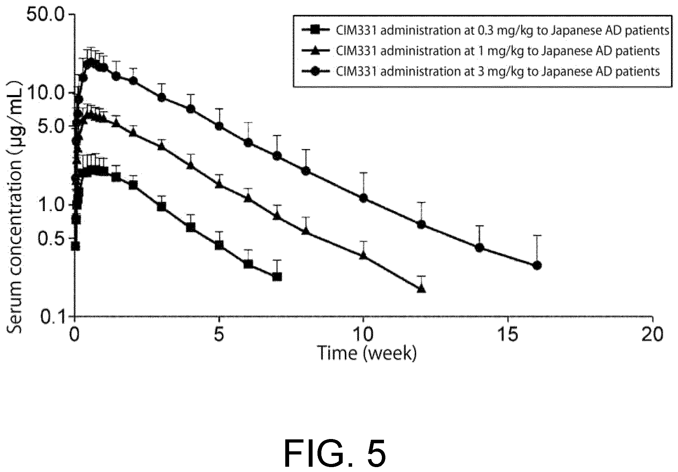

Fig. 5 shows serum concentration_time course of CIM331 in Japanese patients with atopic dermatitis, and Table 1 shows pharmacokinetic parameters.

[Table 1]

| Step | $\text{AUC}_{inf}$ (day*μg/mL) | $\text{AUC}_{last}$ (day*μg/mL) | CL/F (mL/day) | $\text{C}_{max}$ (μg/mL) | MRT (day) | $t_{1/2}$ (day) | $\text{T}_{max}$ (day) |
|---|---|---|---|---|---|---|---|
| C-1 (Japanese, 0.3 mg/kg) | 49.2 | 45.7 | 408 | 2.20 | 19.9 | 12.6 | 5.66 |
| C-2 (Japanese, 1.0 mg/kg) | 161 | 158 | 368 | 6.50 | 22.1 | 13.2 | 4.87 |
| C-3 (Japanese, 3.0 mg/kg) | 489 | 484 | 459 | 19.4 | 23.7 | 14.6 | 4.46 |

As a result, on days 4.46 to 5.66 (mean value; the same applies below) after the administration of CIM331, CIM331 reached its maximum serum concentration, and thereafter showed mild elimination with serum elimination half-lives ($t_{1/2}$) of days 12.6 to 14.6. The $C_{max}$ for the 0.3 mg/kg group, 1 mg/kg group, and 3 mg/kg group were 2.20, 6.50, and 19.4 $\mu$g/mL, respectively, and the $AUC_{inf}$ were 49.2, 161, and 489 day*$\mu$g/mL, respectively. Moreover, the $AUC_{inf}$, $AUC_{last}$, and $C_{max}$ upon administration of single subcutaneous doses of CIM331 increased dose-proportionally. The serum concentration of CIM331 dose-dependently showed a tendency to prolong the period during which the concentration was maintained at a certain level or higher. Meanwhile, the relation between the pruritus-suppressing effect of the CIM331 administration and exposure was not clear in this study.

The terms used in the table refer to the following:

$AUC_{inf}$: AUC from time zero extrapolated to infinite time
$AUC_{last}$: AUC from time zero until the last measurable plasma concentration
CL/F: apparent clearance
$C_{max}$: maximum blood concentration
MRT: mean residence time
$t_{1/2}$: elimination half-life
$T_{max}$: time to achieve maximum blood concentration

(3-6) Exploratory endpoint: efficacy

It was found from these results that CIM331 improved the pruritus, dermatitis, and QOL of the patients with atopic dermatitis. This study is the first clinical study outcome report showing that the IL-31 antagonist is effective against pruritus which occurs due to atopic dermatitis. CIM331 can thus be expected to provide improvements not only in pruritus which occurs due to atopic dermatitis, but also in dermatitis and QOL, based on the novel mechanism of action that blocks the itch-scratch cycle. It is known that scratching caused by pruritus is an exacerbating factor that aggravates rash. Scratching mechanically damages the skin and reduces the barrier function. Foreign antigens that have invaded through the epidermis increase inflammatory responses. This leads to aggravation of dermatitis and an exacerbation of pruritus. This vicious circle of scratching-dermatitis aggravation-pruritus aggravation is known as the itch-scratch cycle (e.g., Wahlgren CF. et al. J Allergy Clin Immunol 2006; 118: 178-89).

[Example 4]

Repeated subcutaneous administration to patients with atopic dermatitis

(4-1) Phase II repeated dose study

In test drug groups in a phase II repeated dose study, about 250 patients with moderate or severe atopic dermatitis for which topical therapy was not sufficiently effective or was intolerable are subcutaneously administered in the abdomen with either CIM331 or placebo, as outlined below. The dosages of CIM331 per body weight and the concentrations of the CIM331 solution for administration are as shown below. The CIM331 solution for administration is slowly administered in a volume of 20 $\mu$L/kg per body weight. When the body weight of a subject exceeds 120 kg, an investigational drug is prepared on the assumption that the body weight is 120 kg. The investigational drug is prepared as follows: A preparation obtained by filling each vial with 1.53 mL of a solution containing 100 mg of the CIM331 antibody per mL, and freeze-drying the solution, is dissolved in water for injection to provide a solution for administration. This solution for administration is further diluted with a separately dissolved placebo solution to an intended concentration for administration.

[Table 2]

| CIM331 Dose (mg/kg) | Concentration of CIM331 Solution for Administration (mg/mL) |
|---|---|
| 0.1 | 5 |
| 0.5 | 25 |
| 2.0 | 100 |

As the patients to be administered with CIM331, patients with atopic dermatitis were selected for which the administration of a topical steroid or a topical calcineurin inhibitor at a fixed dosage for a duration of 4 weeks or longer was not sufficiently effective, or for which standard topical therapy was intolerable, or for which standard topical therapy could not be carried out (due to contraindications and the like), and who met the following criteria:

- An Eczema Area Severity Index score of 10 or more
- An sIGA score of 3 or more
- Pruritus VAS score ≥ 50 mm

This clinical study consisted of two parts. Part A was a randomized, double-blind, placebo-controlled, parallel-group comparison study (weeks 0 to 12). Part B was a double-blind administration extension period, during which the CIM331 administration to the subjects was continued for additional 52 weeks (weeks 12 to 64). About 250 subjects in Part A were randomly allocated to one of four test drug groups (about 50 subjects per group) and a placebo group (about 50 subjects) at a ratio of 1:1:1:1:1.

Part A

· CIM331 (0.1 mg/kg) was subcutaneously administered every 4 weeks (administered at day 1, week 4, and week 8).
· CIM331 (0.5 mg/kg) was subcutaneously administered every 4 weeks (administered at day 1, week 4, and week 8).
· CIM331 (2.0 mg/kg) was subcutaneously administered every 4 weeks (administered at day 1, week 4, and week 8).
· CIM331 (2.0 mg/kg) was subcutaneously administered every 8 weeks (CIM331 administered at day 1 and week 8, and placebo administered at week 4).
· The placebo was subcutaneously administered every 4 weeks (administered at day 1, week 4, and week 8).

More specifically, there were 264 patients who received the administration of the investigational drug or placebo once or more in Part A, and there were 53, 53, 54, 52, and 52 patients, respectively, in the placebo group, the groups to which CIM331 was subcutaneously administered every 4 weeks at 0.1 mg/kg, 0.5 mg/kg, and 2.0 mg/kg, and the group to which CIM331 was subcutaneously administered every 8 weeks at 2.0 mg/kg.

Part B

The subjects who were allocated to the placebo group in Part A were randomly reallocated to groups to which CIM331 (0.1 mg/kg, 0.5 mg/kg, and 2.0 mg/kg) was subcutaneously administered every 4 weeks in Part B.
The subjects who were randomly allocated to the test drug groups in Part A were reallocated to the same dose groups as in Part A, and continued to receive the same treatment from week 12 and thereafter.

- CIM331 (0.1 mg/kg) was subcutaneously administered every 4 weeks for a total period of 52 weeks.
- CIM331 (0.5 mg/kg) was subcutaneously administered every 4 weeks for a total period of 52 weeks.
- CIM331 (2.0 mg/kg) was subcutaneously administered every 4 weeks for a total period of 52 weeks.
- CIM331 (2.0 mg/kg) was subcutaneously administered every 8 weeks for a total period of 52 weeks (CIM331 and the placebo were alternately administered every 4 weeks to the subjects in this group).

(4-2) Rescue therapy

For subjects who did not demonstrate an improvement in pruritus VAS or skin condition, the use of a topical drug is allowed as rescue therapy based on a doctor's judgement from week 4 after the initial administration and thereafter. The definition of "did not demonstrate an improvement" means cases where all of the following conditions are met:

(1) no improvement in the sIGA score from baseline is demonstrated;
(2) the sIGA score is 3 or more; and
(3) the percent improvement in pruritus VAS from baseline is less than 10%, and the latest pruritus VAS score is 50 mm or more.

(4-3) Endpoints:

· The intensity of pruritus is evaluated using the Visual Analog Scale (VAS) (Furue et al. 2013). The VAS consists of a 100-mm straight line, on which the patients themselves indicate the intensity of itchiness in the past 24 hours by drawing a line between 0 to 100 mm, wherein 0 mm represents no itchiness, and 100 mm represents the worst imaginable itchiness.

· The verbal rating scale (VRS) for pruritus is a VRS on which the subjects evaluate the degree of pruritus in the past 24 hours on a scale of (0) no itchiness, (1) mild itchiness, (2) moderate itchiness, (3) severe itchiness, and (4) very severe itchiness (Reich et al. 2012).

· The Eczema Area Severity Index (EASI) score is a tool for assessing the severity and the range of atopic dermatitis. The extent and the proportion of eczema in representative affected areas is evaluated for each of the four areas, i.e., the head and neck, the upper limb, the trunk, and the lower limb, and the degrees of redness (erythema), thickness (induration, papules, and edema), excoriations (scratch marks), and lichenification were evaluated on a scale of (0) none, (1) mild, (2) moderate, and (3) severe.

· SCORing Atopic dermatitis (SCORAD) is a clinical tool for assessing the range and severity of eczema (European Task Force on Atopic Dermatitis 1993).

· static Investigator's Global Assessment (sIGA) comprehensively assesses severity at the time of the evaluation using the clinical characteristics, i.e., erythema, infiltration, papules, exudation, and crusts, on a six scale from clear to very severe disease (0 = clear, 1 = almost clear, 2 = mild disease, 3 = moderate disease, 4 = severe disease, 5 = very severe disease).

· The body surface area (BSA) of atopic dermatitis lesions represents the proportion of the lesions in the entire body.

· The VAS for sleep disturbance is a VAS on which the subjects evaluate the extent of sleep disturbance in the past 24 hours from a scale of (0) "no sleep problems" to (10) "no sleep at all" (Furue et al. 2013).

· The Dermatology Life Quality Index is a dermatologic tool for evaluating the QOL (Finlay et al. 1994), and consists of 10 questions. The DLQI questions can be grouped under the following six items: symptoms and feelings, daily activities, leisure, work and school, personal relationships, and treatments. The DLQI is determined by adding the scores for all the items of the questionnaire. The maximum score is 30, and the minimum score is 0. A higher score indicates lower QOL.

· Actigraphy

Actiwatch is a noninvasive measurement device designed to be worn around a wrist, and capture, record, and store movements of the wrist that serve as an index of systemic movement while the user can behave freely. The subjects wear this device from the start of the preobservation period until week 4. Other parameters including the actual time from falling asleep to awakening, sleep latency, and sleep efficiency are measured using an objective method.

(4-4) Analysis means, analysis method, and the like:

In the groups to which CIM331 is subcutaneously administered every 4 weeks, the percent improvement in pruritus VAS at week 12 after the start of administration compared to that at the start of administration is used as the primary endpoint to verify the superiority and efficacy of each dose group to which CIM331 is administered once every 4 weeks, compared to the placebo group. Analysis of covariance (ANCOVA) is used as the primary analysis method. Specifically, a model is fitted in which the percent improvement in pruritus VAS at week 12 after the start of administration compared to that at the start of administration is used as a response variable, the treated groups are used as fixed effects, and pruritus VAS at the start of administration and the regions (Japan, Europe, and the United States) are used as covariates. In the primary analysis, using a one-sided significance level of 0.025 as the significance level of the test, multiplicity due to the repetition of tests is considered by performing comparisons between two groups successively from a high dose, based on the principle of the closed testing procedure. As the primary analysis set, the per-protocol (PP) set is used which excludes, for example, some subjects who demonstrated a serious deviation from the protocol, subjects who withdrew from the clinical study in an early stage, and subjects who were administered with an investigational drug different from those allocated. Data measured after the receipt of rescue therapy are all excluded, and missing values are complemented using LOCF (Last Observation value Carrying Forward after baseline).

Of the patients who received the administration of the investigational drug or placebo in Part A, the primary analysis set included 46, 46, 45, 47, or 45 patients, respectively, in the placebo group, the groups to which CIM331 was subcutaneously administered every 4 weeks at 0.1 mg/kg, 0.5 mg/kg, or 2.0 mg/kg, or the group to which CIM331 was subcutaneously administered every 8 weeks at 2.0 mg/kg.

Between each of the groups to which CIM331 was administered every 4 weeks and the placebo group, the difference (least square mean) in the percent improvement in pruritus VAS at week 12 after the start of administration compared to that at the start of administration, which was used as the primary endpoint, was -21.39% (p = 0.0027), -41.16% (p < 0.0001), or -40.39% (p < 0.0001) in the 0.1 mg/kg, 0.5 mg/kg, or 2.0 mg/kg group, respectively.

Moreover, since this study is an exploratory dose-finding study, secondary analysis besides the primary analysis can be performed to make a comprehensive investigation of dosages. In this case, although the study is exploratory, a one-sided significance level of 0.025 as the significance level of the test is used as a guide. While not intended to be limiting, specifically, besides ANCOVA, the use of a mixed-effects model repeated measures approach (MMRM), the aggregation of summary statistics independent of a model, the use of the Intent-to-Treat (ITT) analysis set using all the available data obtained at a prescribed observation point after the administration of the investigational drug, and the use of data measured after the receipt of rescue therapy may be contemplated. Alternatively, the results of analysis without compensation of missing values may be checked as appropriate, and comprehensively studied. Furthermore, using pruritus VAS at a time point other than week 12 after the start of administration, or using a variation corresponding to a percent improvement in pruritus VAS compared to that at the start of administration or values at various time points, analysis of a model using the proportion of improved cases based on their continuous quantity or a certain threshold as a response variable may be evaluated. Important subpopulations may be considered for this analysis. A secondary efficacy endpoint besides the pruritus VAS may be similarly analyzed.

A similar exploratory comparison can also be performed on the group to which CIM331 was subcutaneously administered every 8 weeks.

· Proportion of improved subjects: pruritus VAS, EASI, and SCORAD

For each endpoint, the proportion of subjects who demonstrated an improvement of 25%, 50%, or 75% from baseline until each time point was calculated.

With respect to the results for the groups to which CIM331 was administered every 4 weeks in Part A, data measured after the receipt of rescue therapy in the PP set were all excluded, and missing values were complemented using LOCF. In this case, the proportion of subjects who demonstrated an improvement of 50% in pruritus VAS at week 12 after the start of administration was 41%, 67%, or 59% in the 0.1 mg/kg group, 0.5 mg/kg group, or 2.0 mg/kg group, respectively, compared to 21% in the placebo group. The proportion of subjects who demonstrated an improvement of 75% was 14%, 49%, or 44% in the 0.1 mg/kg group, 0.5 mg/kg group, or 2.0 mg/kg group, respectively, compared to 12% in the placebo group.

Likewise, the proportion of subjects who demonstrated an improvement of 50% in EASI at week 12 after the start of administration was 43%, 51%, or 41% in the 0.1 mg/kg group, 0.5 mg/kg group, or 2.0 mg/kg group, respectively, compared to 33% in the placebo group. The proportion of subjects who demonstrated an improvement of 75% was 23%, 37%, or 22% in the 0.1 mg/kg group, 0.5 mg/kg group, or 2.0 mg/kg group, respectively, compared to 14% in the placebo group.

The proportion of subjects who demonstrated an improvement of 50% in SCORAD at week 12 after the start of administration was 18%, 39%, or 31% in the 0.1 mg/kg group, 0.5 mg/kg group, or 2.0 mg/kg group, respectively, compared to 15% in the placebo group. The proportion of subjects who demonstrated an improvement of 75% was 0%, 15%, or 17% in the 0.1 mg/kg group, 0.5 mg/kg group, or 2.0 mg/kg group, respectively, compared to 3% in the placebo group.

· Proportion of subjects who demonstrated an improvement by 2 or more points: sIGA and pruritus VRS

For each endpoint, the proportion of subjects who demonstrated an improvement by 2 or more points from baseline until each time point was calculated.

With respect to the results for the groups to which CIM331 was administered every 4 weeks in Part A, the proportion of subjects who demonstrated an improvement by 2 points or more in sIGA at week 12 after the start of administration was 21%, 30%, or 22% in the 0.1 mg/kg group, 0.5 mg/kg group, or 2.0 mg/kg group, respectively, compared to 12% in the placebo group.

The proportion of subjects who demonstrated an improvement by 2 points or more in pruritus VRS at week 12 after the start of administration was 14%, 47%, or 30% in the 0.1 mg/kg group, 0.5 mg/kg group, or 2.0 mg/kg group, respectively, compared to 5% in the placebo group.

· Degree of improvement: pruritus VAS, EASI, SCORAD, sIGA, BSA of atopic dermatitis lesions, pruritus VRS, and sleep disturbance VAS

For each endpoint, degrees of improvements from baseline until each time point were summarized using descriptive statistics.

With respect to the results for the groups to which CIM331 was administered every 4 weeks in Part A, data measured after the receipt of rescue therapy in the PP set were all excluded. In this case, the percent improvement in pruritus VAS at week 4 after the administration was 39%, 55%, or 46% in the 0.1 mg/kg group, 0.5 mg/kg group, or 2.0 mg/kg group, respectively, compared to 12% in the placebo group. The percent improvement at week 12 after the administration was 47%, 68%, or 67% in the 0.1 mg/kg group, 0.5 mg/kg group, or 2.0 mg/kg group, respectively, compared to 24% in the placebo group.

Likewise, the percent improvement in EASI at week 12 after the administration was 34%, 54%, or 48% in the 0.1 mg/kg, 0.5 mg/kg, or 2.0 mg/kg group, respectively, compared to 38% in the placebo group. The percent improvement in SCORAD at week 12 after the administration was 37%, 45%, or 47% in the 0.1 mg/kg, 0.5 mg/kg, or 2.0 mg/kg group, respectively, compared to 22% in the placebo group. The percent improvement in sIGA at week 12 after the administration was 25%, 34%, or 28% in the 0.1 mg/kg, 0.5 mg/kg, or 2.0 mg/kg group, respectively, compared to 13% in the placebo group. The percent improvement in BSA at week 12 after the administration was 25%, 26%, or 33% in the 0.1 mg/kg, 0.5 mg/kg, or 2.0 mg/kg group, respectively, compared to 31% in the placebo group. The percent improvement in pruritus VRS at week 12 after the administration was 42%, 58%, or 58% in the 0.1 mg/kg, 0.5 mg/kg, or 2.0 mg/kg group, respectively, compared to 18% in the placebo group. The percent improvement in sleep disturbance VAS at week 12 after the administration was 57%, 65%, or 67% in the 0.1 mg/kg, 0.5 mg/kg, or 2.0 mg/kg group, respectively, compared to 31% in the placebo group.

· Time to response: pruritus VAS, EASI, SCORAD, and sIGA

Times from baseline until an improvement of 25%, 50%, or 75% was achieved in pruritus VAS, EASI, and SCORAD and times from baseline until a 2-point improvement was achieved in sIGA were summarized as cumulative incidences over time, using Kaplan-Meier estimates.

With respect to the results for the groups to which CIM331 was administered every 4 weeks in Part A, the time from baseline until 50% of patients achieved an improvement of 25%, 50%, or 75% in pruritus VAS was as follows: 2 weeks, 4 weeks, or not achieved, respectively, in the 0.1 mg/kg group; 2 weeks, 2 weeks, or 5 weeks, respectively, in the 0.5 mg/kg group; and 2 weeks, 4 weeks, or not achieved, respectively, in the 2.0 mg/kg group; compared to 11 weeks, not achieved, or not achieved, respectively, in the placebo group. Moreover, the percent achievement in the improvement of 25%, 50%, or 75% from baseline at week 12 after the start of administration as determined using Kaplan-Meier estimates was as follows: 84%, 66%, or 38%, respectively, in the 0.1 mg/kg group; 95%, 80%, or 68%, respectively, in the 0.5 mg/kg group; and 94%, 71%, or 48%, respectively, in the 2.0 mg/kg group; compared to 52%, 38%, or 22%, respectively, in the placebo group.

Likewise, the time from baseline until 50% of patients achieved an improvement of 25%, 50%, or 75% in EASI was as follows: 2 weeks, 4 weeks, or not achieved, respectively, in the 0.1 mg/kg group; 2 weeks, 4 weeks, or 12 weeks, respectively, in the 0.5 mg/kg group; and 2 weeks, 6 weeks, or not achieved, respectively, in the 2.0 mg/kg group; compared to 6 weeks, 12 weeks, or not achieved, respectively, in the placebo group. Moreover, the percent achievement in the improvement of 25%, 50%, or 75% from baseline at week 12 after the start of administration as determined using Kaplan-Meier estimates was as follows: 71%, 66%, or 37%, respectively, in the 0.1 mg/kg group; 84%, 73%, or 53%, respectively, in the 0.5 mg/kg group; and 93%, 67%, or 28%, respectively, in the 2.0 mg/kg group; compared to 68%, 51%, or 23%, respectively, in the placebo group.

The time from baseline until 50% of patients achieved an improvement of 25%, 50%, or 75% in SCORAD was as follows: 2 weeks, not achieved, or not achieved, respectively, in the 0.1 mg/kg group; 3 weeks, 10 weeks, or not achieved, respectively, in the 0.5 mg/kg group; and 2 weeks, not achieved, or not achieved, respectively, in the 2.0 mg/kg group; compared to 6 weeks, not achieved, or not achieved, respectively, in the placebo group. Moreover, the percent achievement in the improvement of 25%, 50%, or 75% from baseline at week 12 after the start of administration as determined using Kaplan-Meier estimates was as follows: 78%, 46%, or 9%, respectively, in the 0.1 mg/kg group; 78%, 55%, or 30%, respectively, in the 0.5 mg/kg group; and NE (not evaluable), 46%, or 25%, respectively, in the 2.0 mg/kg group; compared to 57%, 40%, or 6%, respectively, in the placebo group.

With respect to the time from baseline until 50% of patients achieved a 2-point improvement in sIGA, such an improvement was not achieved by week 12 in any of the placebo group, 0.1 mg/kg group, 0.5 mg/kg group, and 2.0 mg/kg group. Moreover, the percent achievement in the 2-point improvement from baseline at week 12 after the start of administration as determined using Kaplan-Meier estimates was 36% in the 0.1 mg/kg group, 47% in the 0.5 mg/kg group, or 38% in the 2.0 mg/kg group, compared to 30% in the placebo group.

· Period until the receipt of rescue therapy

Periods until the receipt of rescue therapy were summarized as cumulative incidences over time, using Kaplan-Meier estimates. Subjects who did not receive rescue therapy were censored at the earlier of the hospital visit at week 12 in Part A (or the hospital visit at week 64 in Part B) and the early withdrawal from the clinical study.

With respect to the results for the groups to which CIM331 was administered every 4 weeks in Part A, the time from baseline until 50% of patients received rescue therapy was not achieved by week 12 in any of the placebo group, 0.1 mg/kg group, 0.5 mg/kg group, and 2.0 mg/kg group. Moreover, the time from baseline until 25% of patients received rescue therapy was 5 weeks, 9 weeks, not achieved, or 9 weeks, respectively, in the placebo group, 0.1 mg/kg group, 0.5 mg/kg group, or 2.0 mg/kg group.

· Proportion of subjects who received rescue therapy

The proportion of subjects who had received rescue therapy at each time point was calculated.
With respect to the results for the groups to which CIM331 was administered every 4 weeks in Part A, the proportion of subjects who received rescue therapy was 26.1% in the 0.1 mg/kg group, 24.4% in the 0.5 mg/kg group, or 29.8% in the 2.0 mg/kg group, compared to 39.1% in the placebo group.

· Actigraphy

The results of actigraphy in the groups to which CIM331 was administered every 4 weeks in Part A showed that the actual time from falling asleep to awakening at week 4 after the administration was increased by 49.5 minutes in the 0.1 mg/kg group, increased by 53.1 minutes in the 0.5 mg/kg group, or increased by 48.2 minutes in the 2.0 mg/kg group, compared to an increase by 7.3 minutes in the placebo group. The sleep latency (the time from going to bed to falling asleep) at week 4 after the administration was decreased by 17.6 minutes in the 0.1 mg/kg group, decreased by 14.8 minutes in the 0.5 mg/kg group, or decreased by 12.7 minutes in the 2.0 mg/kg group, compared to a decrease by 4.3 minutes in the placebo group.
Furthermore, in the group to which CIM331 was administered once every 8 weeks in Part A, the mean value of percent improvements in pruritus VAS at week 12 after start of administration from which all the data measured after the receipt of rescue therapy were excluded, was 70%.
From the results of pruritus VAS at week 12 after the administration as the primary endpoint, as well as the results of dermatitis scores such as EASI and sIGA in PartA, it was thought that the effects against pruritus and dermatitis reached maximum in the group to which CIM331 was administered at 0.5 mg/kg/4 weeks.

· Optimal dosage simulations

With respect to dosages, from the viewpoint of further improving the convenience, optimal dosage and administration at a fixed dose were evaluated from dosages per body weight, by performing modeling and simulation.
Initially, exposure was compared between doses per body weight and fixed doses, and optimal dosage and administration were studied from the viewpoint of pharmacokinetics. The serum drug concentration of CIM331 fitted well to a one-compartment model with first order absorption. Moreover, body weight as a covariate was integrated into model parameters, using the allometry equation. The model parameters are shown below.

[Table 3]

| Parameter | Unit | Estimate | Bootstrapped 90% interval |
|---|---|---|---|
| CL/F | L/day | 0.327 | 0.312 - 0.343 |
| Covariate effect of ALB | | -1.72 | -2.00 - -1.38 |
| V/F | L | 7.46 | 7.12 - 7.83 |
| ka | 1/day | 0.514 | 0.442 - 0.609 |
| Inter-individual variability | | | |
| Variance for CL/F | | 0.186 | 0.142 - 0.239 |
| Variance for V/F | | 0.179 | 0.123 - 0.244 |
| Variance for ka | | 0.276 | 0.182 - 0.377 |
| Covariance for CL/F and V/F | | 0.134 | 0.0871 - 0.185 |
| Residucal variability | | | |
| Log normal error (CV) | % | 15.5 | 14.0 -17.1 |

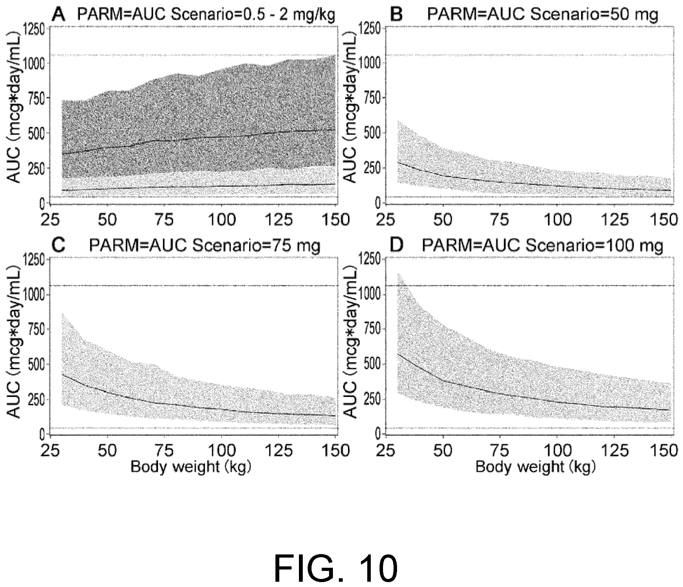

Simulations were performed using the above-described one-compartment model. Fig. 10 shows relationships between the body weight and exposure. In Fig. 10, the graph A shows estimated exposure by administration at 0.5 mg/kg or 2 mg/kg, and the graphs B, C, and D show estimated exposure by administration at 50, 75, and 100 mg/body, respectively. The reference curves in each graph indicate an estimated upper limit of exposure (1060 $\mu$g*day/mL) at 2 mg/kg and an estimated lower limit of exposure (44 $\mu$g*day/mL) at 0.5 mg/kg.
When the dosage was fixed at 50 mg, the lower limit of exposure at approximately 0.5 mg/kg was estimated to be exceeded at a body weight below 100 kg, and when the dosage was fixed at 100 mg, the upper limit of exposure at 2 mg/kg was estimated to be exceeded at low body weights. Moreover, when the dosage was fixed at 75 mg, the exposure was estimated to fall within the range of exposure obtained at 0.5 mg/kg or 2 mg/kg. From these results, it was thought that the administration of CIM331 once every 4 weeks at a fixed dosage of 50 mg (or 100 mg for a body weight over 100 kg) or 75 mg would result in exposure similar to that obtained in the phase II study.

Subsequently, modeling and simulation was performed on pruritus VAS, using PK/PD analysis. An indirect turnover model was used for the part of pruritus VAS, and scale conversion was performed. Model predicted values were confirmed to imitate actual measurements well. The calculated model parameters are shown below.

[Table 4]

| Parameter | Unit | Estimate | Bootstrapped 90% interval |
| --- | --- | --- | --- |
| Kout | 1/day | 0.0710 | 0.0578 - 0.0839 |
| Placebo effect | - | 0.554 | 0.400 - 0.769 |
| Imax | - | 0.893 | 0.490 - 1.50 |
| IC50 | $\mu$g/mL | 3.21 | 0.956 - 9.43 |
| Inter-individual variability | | | |
| Variance for kout | - | 0.581 | 0.396 - 0.809 |
| Variance for Placebo effect | - | 0.983 | 0.737 -1.46 |
| Variance for Imax | - | 1.81 | 0.712 - 3.01 |
| Residucal variability | | | |
| Additive error | - | 0.0276 | 0.0193 - 0.0354 |
| Proportional error (CV) | % | 25.9 | 23.3 - 28.7 |

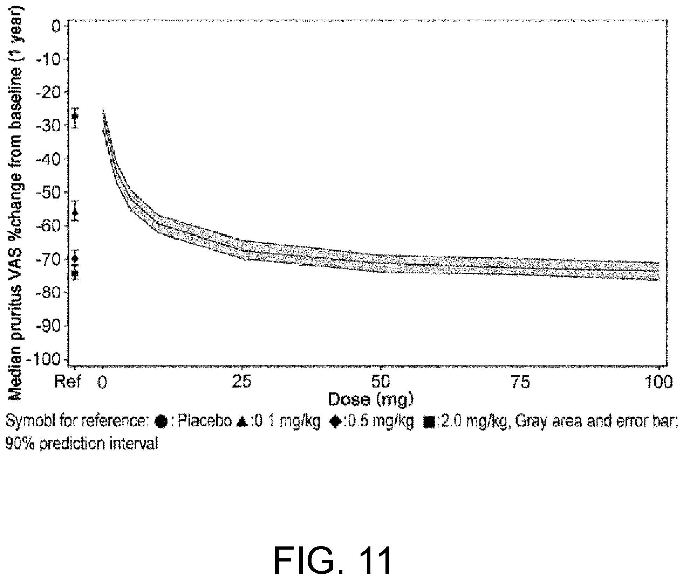

Simulations were performed using the model. Figure 11 shows estimated pruritus VAS at 1 year after the administration of CIM331.

When the fixed dosage per 4 weeks was 25 mg/body or more, and preferably 50 mg/body or more, pruritus VAS was estimated to show values similar to those at 0.5 mg/kg or 2 mg/kg.

(4-5) Expected results and advantageous effects

Repeated administration of CIM331 every 4 or 8 weeks achieves a steady-state serum concentration of CIM331, and hence, can demonstrate a sustained effect against the pruritus in patients with atopic dermatitis. Moreover, the maintenance of the pruritus-improving effect, i.e., the blockage of the itch-scratch cycle, can improve dermatitis, and can improve the QOL. A long-term efficacy profile over a total period of 64 weeks can be confirmed.

Furthermore, in view of the fact that the currently existing systemic therapeutic methods for atopic dermatitis require taking a medicine or applying a medicine to an affected area several times a day, or ultraviolet therapy may require a visit to the hospital as many as once or twice a week, a therapeutic embodiment involving repeatedly administering CIM331 every 4 or 8 weeks, for example, can be expected to markedly alleviate the patient's burden of taking the medicine or visiting the hospital, for example, and can further contribute to improving the patient's QOL.

[Example 5]

Single subcutaneous administration targeting hemodialysis patients with pruritus

(5-2) Phase II clinical study

Regarding the test drug groups in the phase II clinical study, about 60 hemodialysis patients (more specifically, 69 patients) for whom a topical therapy or systemic therapy other than nalfurafine hydrochloride did not work sufficiently well were selected as subjects and, according to the procedures specified below, a single dose of CIM331 or placebo was subcutaneously administered to the abdomen, or to the reference group, nalfurafine hydrochloride capsule was orally administered every day for 12 weeks at one capsule (2.5 $\mu$g) per time per day after an evening meal or before going to bed. The dose per body weight and the concentration of administration solution for CIM331 were adjusted as specified below and a volume of 20 $\mu$L per body weight was slowly administered. The preparation of the investigational drug, prepared by filling a vial with 1.53 mL of a solution containing the CIM331 antibody at 100 mg per 1 mL and freeze-drying it, was dissolved in injection water to prepare an administration solution and it was further diluted using a solution of placebo dissolved separately to give the desired concentration for administration.

[Table 5]

| Dose of CIM331 (mg/kg) | Concentration of CIM331 administration solution (mg/mL) |
| --- | --- |
| 0.125 | 6.25 |
| 0.5 | 25 |
| 2.0 | 100 |

Uremic pruritus patients selected for CIM331 administration were those who had been receiving an antihistamine agent or antiallergic agent, excluding nalfurafine hydrochloride, for two or more weeks without sufficient effect, or had received anti-pruritus treatment with nalfurafine hydrochloride during the last one year (irrespective of the duration of the treatment), and who satisfied the following criteria:

- Patients who were measured for pruritus VAS on five or more days of the one-week preobservation period, and satisfied both of the following conditions:

  (1) the measured value was 20 mm or greater on five or more days; and
  (2) the average measured value was 50 mm or greater.

This investigation was a randomized, double-blind, placebo-controlled, parallel-group comparative study with an open-label reference group (nalfurafine hydrochloride group).

About 60, or more specifically 69, test subjects were assigned randomly to any one of the following five groups at 1:1:1:1:1.

- Placebo group: A single dose of a placebo for CIM331 was subcutaneously administered.
- CIM331 0.125 mg/kg group: A single dose of CIM331 (0.125 mg/kg) was subcutaneously administered.
- CIM331 0.5 mg/kg group: A single dose of CIM331 (0.5 mg/kg) was subcutaneously administered.
- CIM331 2.0 mg/kg group: A single dose of CIM331 (2.0 mg/kg) was subcutaneously administered.
- Nalfurafine hydrochloride group: One nalfurafine hydrochloride capsule (2.5 μg) was orally administered once every day for 12 weeks. The dose could be increased depending on the symptoms but up to two capsules (5 μg) a day.

(5-2) Rescue therapy

After all observations and inspections on day 29 were completed, permission was given to change the type, dose regimen, and dosage of treatment against pruritus for patients who experienced no improvement of pruritus or who had weakened efficacy (the reduction of pruritus VAS from the baseline being below 10 mm), on the condition that the principal investigator or a subinvestigator of the clinical trial recognized the changes as being necessary.

(5-3) Endpoint:

- The intensity of pruritus was evaluated using the Visual Analog Scale (VAS) (Furue et al. 2013). The VAS consists of a 100-mm straight line, on which the patients themselves indicate the intensity of itchiness in the past 24 hours by drawing a line between 0 to 100 mm, wherein 0 mm represents no itchiness, and 100 mm represents the worst imaginable itchiness. The assessment was carried out as close to the same time of day as possible.
- Shiratori severity scores (Shiratori, et al., 1983): The extent of the symptoms in the daytime and nighttime within the past 24 hours was evaluated based on the severity scores of pruritus from 0 (no symptom) to 4 (severe itch). The principal investigator or a subinvestigator participated in making this assessment, in addition to the test subject. The assessment for the test subject was carried out as close to the same time of day as possible.
- 5-D itch scale (Ebata, et al., 2015): Duration of having pruritus, degree, direction of progress, disability, and distribution of pruritus within the past two weeks were assessed and scored.
- VAS for sleep disturbance: VAS on which the subjects evaluated the extent of sleep disturbance in the past 24 hours from a scale of (0) "no sleep problems" to (10) "no sleep at all" (Furue et al. 2013).

- Insomnia Severity Index (Murosawa, et al., 2009): The condition of insomnia was scored based on the insomnia severity questionnaire having five questions.
- EQ-5D-5L (Ikeda, et al., 2015): Health states for the five points ("mobility", "self-care", "usual activities", "pain/discomfort", and "anxiety/depression") were investigated by five levels.
- Actigraphy

Actiwatch is a noninvasive measurement device designed to be worn around a wrist, and capture, record, and store movements of the wrist that serve as an index of systemic movement while the user can behave freely. Other parameters including the actual time from falling asleep to awakening, sleep latency, and sleep efficiency were measured using an objective method.

- Assessment of skin symptoms (assessment by physician): Assessment of skin symptoms by four levels from mild to the severest was made by the principal investigator or a subinvestigator, with referring to the "standards for severity" provided in the "Guidelines for Treatment of Atopic Dermatitis 2008, Health, Health and Labor Sciences Research Group".
- Assessment of skin symptoms (assessment using photographs): Photographs were taken by the principal investigator or a subinvestigator for skin rash including those identified in the "standards for severity" provided in the "Guidelines for Treatment of Atopic Dermatitis 2008, Health and Labor Sciences Research Group" (i.e., erythema, drying, desquamation, papule, erosion, infiltration, and lichenification) as well as scars by scratching, and a representative symptom (the part of highest severity) was selected for each subject. The assessment of skin symptoms was made by an evaluator independently of medical experts based on the criteria for evaluation defined as the Japanese Dermatological Association's atopic dermatitis severity classification (simplified version) in the range of five levels from 0 (no symptom) to 4 (the severest).

(5-4) Analysis means, analysis method, and the like:

Paired comparison between each CIM331 administration group and the placebo administration group for the change in pruritus VAS at four weeks after the administration from the baseline was carried out as the primary analysis. Hypothesis was tested with reference to 20% level of significance for the two-tailed test. As this is an exploratory study, adjustment for multiplicity was not made.

For the primary assessment, the placebo group and each dosage group of CIM331 were compared by analysis of covariance (ANCOVA), selecting the change in pruritus VAS at four weeks after the administration from the baseline as an objective variable and the pruritus VAS at the baseline as a covariate. The 95% confidence interval of the difference in average values was calculated by ANCOVA. Missing values were complemented using LOCF (Last Observation Carried Forward after baseline).

The nalfurafine hydrochloride group was exploratorily compared with each CIM331 administration group and the placebo group. Similar analysis was carried out as the primary analysis for each CIM331 administration group and the placebo group. Other exploratory analyses were as described in the SAP (statistical analysis plan).

In the phase II clinical study, the primary analysis set from the patients who received administration of placebo, CIM331, or nalfurafine hydrochloride capsule (reference group) was the placebo group, CIM331 0.125 mg/kg group, CIM331 0.5 mg/kg group, CIM331 2.0 mg/kg group, and nalfurafine hydrochloride group which consisted of 14 cases, 14 cases, 13 cases, 14 cases, and 12 cases, respectively.

Difference on the primary endpoint, i.e., the change in pruritus VAS at four weeks after the administration from the start (baseline) (least mean square), as compared to the placebo was - 2.4 mm (p=0.7806), -8.7 mm (p=0.3317), and 0.4 mm (p=0.9678) in the CIM331 0.125 mg/kg group, CIM331 0.5 mg/kg group, and CIM331 2.0 mg/kg group, respectively. The difference from the placebo was 5.7 mm (p=0.5154) in the nalfurafine hydrochloride group (reference group).

- Changes in pruritus VAS at each evaluation time point

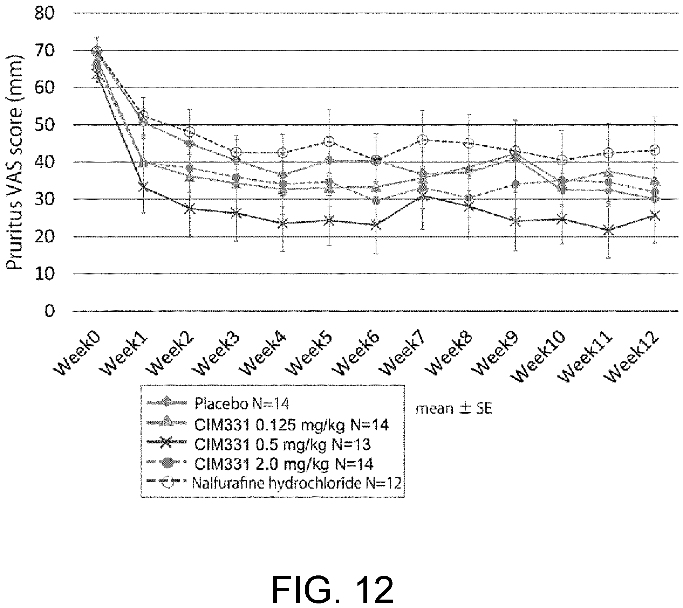

The change in pruritus VAS at one week after the start of administration from the baseline was -18.6 mm in the placebo group and -17.4 mm in the nalfurafine hydrochloride group, and, in contrast thereto, was -27.4 mm in the CIM331 0.125 mg/kg group, -30.3 mm in the CIM331 0.5 mg/kg group, and -25.9 mm in the CIM331 2.0 mg/kg group, showing quick improvement effect on pruritus in every CIM331 groups. The change in pruritus VAS at four weeks after the start of administration was -32.8 mm in the placebo group and -27.3 mm in the nalfurafine hydrochloride group, and, in contrast thereto, was -34.7 mm in the CIM331 0.125 mg/kg group, - 40.1 mm in the CIM331 0.5 mg/kg group, and -31.5 mm in the CIM331 2.0 mg/kg group, with the largest change being observed in the CIM331 0.5 mg/kg group (Fig. 12).

- Percentage of test subjects found with improvement reducing pruritus VAS to below 30 mm

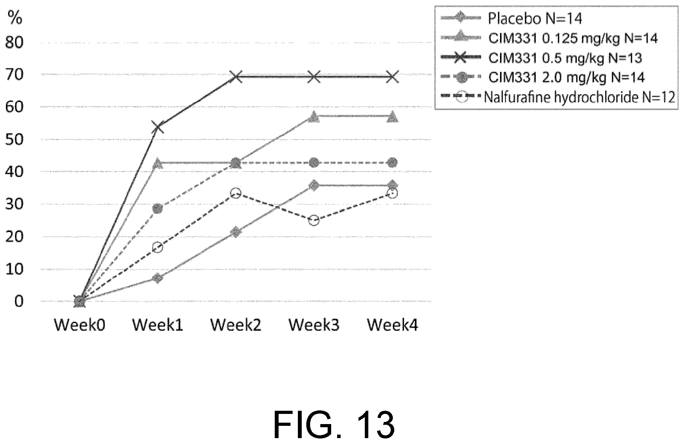

The percentage of test subjects found with the improvement to reduce pruritus VAS to less than 30 mm after four weeks from the start of administration, i.e., at the time to evaluate the primary endpoint, was 35.7% in the placebo group and 33.3% in the nalfurafine hydrochloride group, and, in contrast thereto, was 57.1% in the CIM331 0.125 mg/kg group, 69.2% in the CIM331 0.5 mg/kg group, and 42.9% in the CIM331 2.0 mg/kg group, with higher proportion of test subjects showing improvement of VAS to less than 30 mm, which is indicative of mild pruritus, in the CIM331 groups than in the placebo group and the nalfurafine hydrochloride group (Fig. 13).

- Scores based on Shiratori severity scores

The change in Shiratori score (daytime) at four weeks after the start of administration was -0.89 in the placebo group and -0.67 in the nalfurafine hydrochloride group, and, in contrast thereto, was -1.00 in the CIM331 0.125 mg/kg group, -1.30 in the CIM331 0.5 mg/kg group, and -0.79 in the CIM331 2.0 mg/kg group, with the largest improvement being observed in the CIM331 0.5 mg/kg group.
The change in Shiratori score (nighttime) at four weeks after the start of administration was -0.81 in the placebo group and -0.51 in the nalfurafine hydrochloride group, and, in contrast thereto, was -0.64 in the CIM331 0.125 mg/kg group, -1.16 in the CIM331 0.5 mg/kg group, and -0.79 in the CIM331 2.0 mg/kg group, with the largest improvement being observed in the CIM331 0.5 mg/kg group.

- Scores based on 5-D itch scale

The change in 5-D itch scale score at four weeks after the start of administration was -5.4 in the placebo group and -3.7 in the nalfurafine hydrochloride group, and, in contrast thereto, was -5.6 in the CIM331 0.125 mg/kg group, -6.5 in the CIM331 0.5 mg/kg group and -3.1 in the CIM331 2.0 mg/kg group, with the largest improvement being observed in the CIM331 0.5 mg/kg group.

[Example 6]

Biomarker analysis in hemodialysis patients with pruritus

(6-1) Biomarker evaluation

Biomarker evaluation was performed to evaluate correlation between the serum IL-31 concentration before CIM331 administration and the clinical trial results after CIM331 administration.

(6-2) Analysis means, analysis method, and the like:

Serum samples were obtained from all test patients who agreed to storage of their sera for use in biomarker analysis (n = 68). The serum samples cryopreserved at or below -70°C were thawed at room temperature, and serum IL-31 concentration was measured using ultrasensitive enzyme-linked immunosorbent assay (ELISA; SiMoA™, Quanterix, Billerica, MA, USA). Data obtained from the samples of these 68 patients were used to derive a cutoff median value, 0.86 pg/mL, for the serum IL-31 concentration at the screening stage (before CIM331 administration). This IL-31 cutoff value was used to evaluate correspondence between IL-31 and the clinical trial results for the patients who received the trial administration as described in Example 5 (n = 48). The samples from the test patients were also compared with commercially available healthy volunteer-derived samples by post hoc analysis.

(6-3) Analysis results:

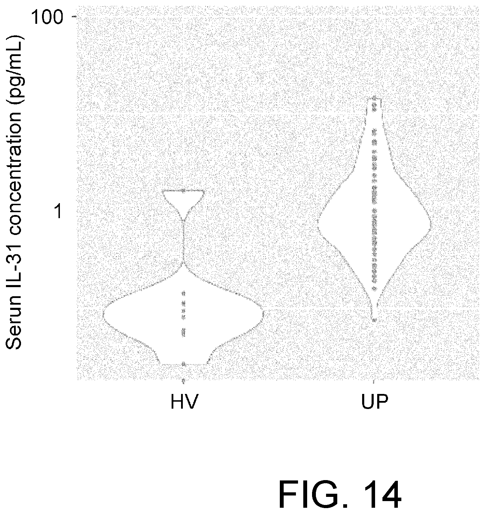

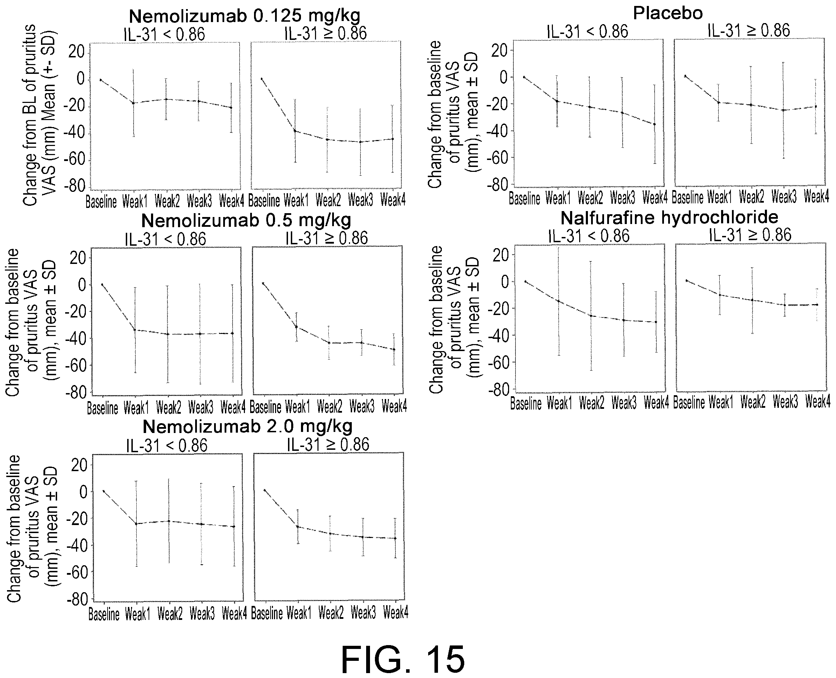

The result of the biomarker analysis of IL-31 distribution by post hoc analysis is shown in Fig. 14. Compared to healthy volunteers (HV; n = 20), uremic pruritus (UP; n = 68) patients showed clearly high serum IL-31 levels (Fig. 14). Among the 48 patients who underwent serum sampling and trial administration, those with a serum IL-31 level of 0.86 pg/mL or higher showed significant reduction in pruritus VAS as a result of CIM331 administration, compared to those with a serum IL-31 level lower than 0.86 pg/mL (Fig. 15). This trend was not observed in the placebo-administered group or the nalfurafine hydrochloride-administered group.
It has been reported that among patients under maintenance dialysis, those with pruritus have higher serum IL-31 concentrations than those without pruritus. This suggests the possibility that the serum IL-31 concentration might influence the effect of CIM331 administration. As shown in Fig. 14, the serum IL-31 levels of the UP patients were clearly higher than those of the healthy volunteers in post hoc analysis. However, there was no evident correlation between the serum IL-31 levels at the screening stage and the pruritus VAS values at the baseline. Nevertheless, patients who had high serum IL-31 levels at the screening stage tended to show significant reduction in pruritus VAS after CIM331 administration. On the other hand, such results were not observed in the placebo-administered group or the nalfurafine hydrochloride-administered group. These results support the hypothesis that IL-31 is one of the factors contributing to the development of uremic pruritus.

(6-4) Expected results and beneficial effect

By single subcutaneous administration of CIM331, the serum CIM331 concentration increases and effects against dialysis patient's pruritus can be persistently exerted. With sustained pruritus improving effect, or in other words the blockade of the Itch-Scratch Cycle, dermatitis can become improvable, allowing for betterment of QOL.

Furthermore, in view of the fact that the currently existing systemic therapeutic methods for uremic pruritus require taking a medicine or applying a medicine to an affected area several times a day, or ultraviolet therapy may require a visit to the hospital as many as once or twice a week, a therapeutic embodiment that can suppress pruritus over four weeks or more by single subcutaneous administration of CIM331, for example, can be expected to markedly alleviate the patient's burden of taking the medicine or visiting the hospital, for example, and can further contribute to improving the patient's QOL.

Moreover, in uremic pruritus patients who have IL-31 concentrations equal to or higher than a predetermined value before starting CIM331 administration, the administration of CIM331 can be expected to exert higher pruritus-improving effect. Such a predetermined serum IL-31 concentration that may serve as a baseline at which the exertion of high pruritus-improving effect can be expected is, for example, 0.86 pg/mL. Administering CIM331 to uremic pruritus patients with serum IL-31 concentration equal to or higher than such a predetermined value may further contribute to improvement of patients' QOL.

(6-5) An embodiment of practice for CIM331 after approval

CIM331 may be administered to dialysis patients for whom existing treatment against pruritus other than nalfurafine hydrochloride does not work sufficiently well, although it is not limited to such embodiments.

CIM331 may be subcutaneously administered repeatedly in equal dosages at the same dosing interval, for example, once every 2 to 12 weeks, and specifically, for example, every 2, 3, 4, 5, 6, 7, 8, 9, 10, 11, or 12 weeks, or once every 1, 2, or 3 months.

The dosage of CIM331 per body weight and the concentration of the CIM331 solution for administration may be determined as appropriate, based on the results of the phase II clinical trial or the results of other studies, for example. For example, when the body weight of a patient with uremic pruritus exceeds 120 kg, an investigational drug may be prepared on the assumption that the body weight is 120 kg. Furthermore, when it is intended that CIM331 be administered in mg/body to a patient with uremic pruritus, dosages in mg/kg of CIM331 may be converted to dosages in mg/body, based on the results of the phase II clinical trial, for example, and an appropriate dosage (mg/body) may be selected and administered. In this case, although the logic for converting mg/kg to mg/body is not limited, it will be understood that a person skilled in the art can determine a dosage in mg/body, as appropriate, by using the following logic.

Assuming that there are the minimum effective serum concentration and the maximum tolerable (empirical) serum concentration of CIM331, changing of a dosage in mg/kg into a dosage in mg/body is considered based on the results of the phase II study, such that a serum concentration of CIM331 within this range of concentrations is achieved, regardless of body weight. Moreover, because a dosage in mg/body for a child with a low body weight may markedly increase the exposure, a dosage in mg/kg is considered in such a case. Conversion to mg/body can be accomplished by determining the minimum effective serum concentration and the maximum tolerable serum concentration from the results of the phase II study that is currently being performed, and adjusting the exposure as described above.

Alternatively, based on the body weight of a typical uremic pruritus patient estimated based on statistics, the dosage per body weight (mg/kg) may be converted into a fixed dose (mg/body) using the following calculation formula: [dosage per body weight] x [estimated body weight] = fixed dose.

In a non-limiting embodiment, for an adult or pediatric patient with uremic pruritus, CIM331 may be subcutaneously administered at one dosage selected from 0.1 mg to 1000 mg/body, for example, 0.2 mg to 360 mg/body, and preferably 10 mg to 200 mg/body, 10 mg to 100 mg/body, 25 mg to 100 mg/body, 50 mg to 100 mg/body, or 50 mg to 75 mg/body, and at a dosing interval described above, repeatedly at the same dosage and the same dosing interval.

Alternatively, in another non-limiting embodiment, for a pediatric patient with uremic pruritus, CIM331 may be subcutaneously administered at one dosage selected from 0.01 mg to 10 mg/kg, for example, 0.1 mg to 3 mg/kg, preferably 0.2 mg to 2 mg/kg, and more preferably 0.5 mg to 1.5 mg/kg, and at a dosing interval described above, repeatedly at the same dosage and the same dosing interval.

[Reference Example 1]

Expression and Purification of IgG antibodies

The expression of antibodies was performed using the following method. Human fetal renal cancer cell-derived HEK293H cell line (Invitrogen) was suspended in DMEM medium (Invitrogen) supplemented with 10% Fetal Bovine Serum (Invitrogen). The cells were plated at 10 mL per dish for adherent cells (10 cm in diameter; CORNING) at a cell density of 5 to 6 x $10^5$ cells/mL, and cultured in a $CO_2$ incubator (37°C, 5% $CO_2$) for one whole day and night. The medium was then removed by aspiration, and 6.9 mL of CHO-S-SFM-II (Invitrogen) medium was added. The prepared plasmid was introduced into the cells by the lipofection method. The resulting culture supernatants were collected and centrifuged (about 2000 g, 5 min, room temperature) to remove the cells, and sterilized by filtering through 0.22-$\mu$m filter MILLEX (R)-GV (Millipore) to obtain culture supernatants. Antibodies were purified from the obtained culture supernatants by a method known to those skilled in the art using rProtein A SepharoseTM Fast Flow (Amersham Biosciences). To determine concentrations of the purified antibodies, absorbance was measured at 280 nm using a spectrophotometer. The antibody concentrations were calculated from the determined value, using an absorbance coefficient calculated by the method described in Protein Science 1995; 4: 2411-2423.

**Claims**

1. A pharmaceutical composition for prevention and/or treatment of uremic pruritus comprising an IL-31 antagonist as an active ingredient.

2. The pharmaceutical composition of claim 1, wherein the IL-31 antagonist is administered to a subject with or potentially with uremic pruritus at 0.1 mg to 1000 mg/body/2 weeks, 0.1 mg to 1000 mg/body/4 weeks, or 0.1 mg to 1000 mg/body/8 weeks, repeatedly at the same dose and the same dosing interval.

3. The pharmaceutical composition of claim 2, wherein the IL-31 antagonist is administered at 25 mg to 100 mg/body/4 weeks.

4. The pharmaceutical composition of claim 3, wherein the IL-31 antagonist is administered at 50 mg to 100 mg/body/4 weeks.

5. The pharmaceutical composition of claim 1, wherein the IL-31 antagonist is administered to a subject with or potentially with uremic pruritus at 0.01 mg to 10 mg/kg/2 weeks, 0.01 mg to 10 mg/kg/4 weeks, or 0.01 mg to 10 mg/kg/8 weeks, repeatedly at the same dose and the same dosing interval.

6. The pharmaceutical composition of claim 5, wherein the IL-31 antagonist is administered at 0.2 mg to 2 mg/kg/4 weeks.

7. The pharmaceutical composition of any one of claims 1 to 6, wherein the IL-31 antagonist is administered to a subject with or potentially with uremic pruritus having a serum IL-31 concentration equal to or higher than a predetermined value.

8. The pharmaceutical composition of any one of claims 1 to 7, which is for use in improvement of sleep disturbance caused by uremic pruritus.

9. The pharmaceutical composition of claim 8, wherein the improvement of sleep disturbance is for increasing time from falling asleep to awakening, and/or for decreasing sleep latency (time from going to bed to falling asleep).

10. The pharmaceutical composition of any one of claims 1 to 9, wherein the IL-31 antagonist is an antibody that inhibits IL-31 signaling.

11. The pharmaceutical composition of claim 10, wherein the antibody does not exhibit cross-reactivity with IL-3 IRA from any of mouse, rat, and rabbit.

12. The pharmaceutical composition of claim 10 or 11, wherein the antibody is an anti-IL-31 neutralizing antibody or an anti-IL-31RA neutralizing antibody.

**13.** The pharmaceutical composition of claim 12, wherein the anti-IL-31RA neutralizing antibody is any of

(1) an anti-IL-31RA antibody comprising an H chain variable region comprising CDR1 as set forth in SEQ ID NO: 1, CDR2 as set forth in SEQ ID NO: 2, and CDR3 as set forth in SEQ ID NO: 3, and an L chain variable region comprising CDR1 as set forth in SEQ ID NO: 4, CDR2 as set forth in SEQ ID NO: 5, and CDR3 as set forth in SEQ ID NO: 6;
(2) an anti-IL-31RA antibody comprising an H chain variable region as set forth in SEQ ID NO: 7 and an L chain variable region as set forth in SEQ ID NO: 8; and
(3) an anti-IL-31RA antibody comprising an H chain as set forth in SEQ ID NO: 9 and an L chain as set forth in SEQ ID NO: 10.

FIG. 1

FIG. 2

FIG. 3

FIG. 4

FIG. 5

FIG. 6

FIG. 7

FIG. 8

FIG. 9

FIG. 10

Symobl for reference: ●: Placebo ▲:0.1 mg/kg ◆:0.5 mg/kg ■:2.0 mg/kg, Gray area and error bar: 90% prediction interval

## FIG. 11

FIG. 12

FIG. 13

FIG. 14

FIG. 15

EP 4 209 227 A1

## INTERNATIONAL SEARCH REPORT

| | International application No. |
|---|---|
| | PCT/JP2020/032987 |

**A. CLASSIFICATION OF SUBJECT MATTER**
Int.Cl. A61K39/395(2006.01)i, A61K45/00(2006.01)i, A61P25/20(2006.01)i, A61P17/04(2006.01)i
FI: A61K45/00ZNA, A61P17/04, A61P25/20, A61K39/395D
According to International Patent Classification (IPC) or to both national classification and IPC

**B. FIELDS SEARCHED**

Minimum documentation searched (classification system followed by classification symbols)
Int.Cl. A61K39/395, A61K45/00, A61P25/20, A61P17/04

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS/WPIDS(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | WO 2014/208645 A1 (CHUGAI PHARMACEUTICAL CO., LTD.) 31 December 2014 (2014-12-31), claims 7-11, 13, paragraphs [0002], [0005], [0048] | 1-7, 10-13<br>8-13 |
| X<br>Y | JP 2009-528264 A (ZYMOGENETICS INC.) 06 August 2009 (2009-08-06), example 2, paragraph [0074] | 1-7, 10<br>8, 9, 11-13 |
| X<br>Y | KR 10-2019-0059860 A (VIROMED CO., LTD.) 31 May 2019 (2019-05-31), Claims 1, 3, 5, paragraph [0002], examples 2, 4, 5, abstract | 1-7<br>8-13 |

☒ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 October 2020 | 20 October 2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2020/032987

C (Continuation). DOCUMENTS CONSIDERED TO BE RELEVANT

| Category* | Citation of document, with indication, where appropriate, of the relevant passages | Relevant to claim No. |
|---|---|---|
| Y | 飯野 則昭、ほか１名, 透析皮膚そう痒症, 日本臨床(別冊), 22 July 1997, no. 18, pp. 259-263, p. 259, left column, third paragraph, p. 259, right column, first paragraph, (IINO, Noriaki and 1 other), non-official translation (Dialysis skin pruritus, Japanese Clinical Practice (separate volume)) | 8, 9 |
| Y | WO 2015/025767 A1 (HISAMITSU PHARMACEUTICAL CO INC.) 26 February 2015 (2015-02-26), paragraph [0002] | 8, 9 |
| Y | JP 2002-338476 A (HEALTH FACTOR KENKYUSHO KK) 27 November 2002 (2002-11-27), abstract, paragraph [0002] | 8, 9 |
| A | US 2019/0135804 A1 (THE UNITED STATES OF AMERICA, AS REPRESENTED BY THE SECRETARY, DEPARTMENT OF HEALTH AND HUMAN SERV) 09 May 2019 (2019-05-09), claim 10, paragraph [0071] | 1-13 |
| A | 笠倉 新平, アトピー性皮膚炎の発症に関与する新規サイトカイン： IL-31, 医学のあゆみ, 21 October 2006, vol. 219, no. 3, pp. 229, 230, p. 230, left column, (KASAKURA, Shimpei, Journal of Clinical and Experimental Medicine), non-official translation (A novel cytokine involved in the development of atopic dermatitis: IL-31) | 1-13 |

Form PCT/ISA/210 (second sheet) (January 2015)

**INTERNATIONAL SEARCH REPORT**
Information on patent family members

| International application No. |
| --- |
| PCT/JP2020/032987 |

| | | |
| --- | --- | --- |
| WO 2014/208645 A1 | 31 December 2014 | TW 201537175 A<br>claims 7-11, 13, paragraphs<br>[0002], [0005], [0048] |
| JP 2009-528264 A | 06 August 2009 | US 2007/0160610 A1<br>example 2, paragraph [0075]<br>WO 2007/143231 A2<br>EP 1991581 A2<br>KR 10-2008-0108413 A<br>CN 101589060 A |
| KR 10-2019-0059860 A | 31 May 2019 | WO 2019/103497 A1<br>claims 1, 3, 5,<br>paragraph [0002],<br>examples 2, 4, 5, abstract |
| WO 2015/025767 A1 | 26 February 2015 | TW 201542247 A<br>paragraph [0002] |
| JP 2002-338476 A | 27 November 2002 | (Family: none) |
| US 2019/0135804 A1 | 09 May 2019 | WO 2019/090039 A1<br>claim 9, paragraph [0062] |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004003140 A
- WO 2005079566 A
- WO 2006063864 A
- WO 2006063865 A
- WO 2009071696 A
- WO 2006088855 A
- WO 2006088955 A
- WO 2006088956 A
- WO 2007133816 A
- WO 2007142325 A
- WO 2009072598 A
- WO 2006122079 A

- WO 2007143231 A
- WO 2008028192 A
- WO 2009072604 A
- WO 2010064697 A
- WO 00075314 A
- WO 9954342 A
- WO 0061739 A
- WO 2002020565 A
- WO 1995001937 A
- WO 2004044011 A
- WO 2002032925 A

**Non-patent literature cited in the description**

- **NAKAI S et al.** Current situation on chronic dialysis therapy in Japan (as of December 31, 1999. *Journal of the Japanese Society for Dialysis Therapy,* 2001, vol. 34, 1-31
- **DANNO K.** Friends of itchiness, The perfect guide for treating itchiness you want to leave in the dialysis room. *Kinpodo,* 2008, 1-16
- **OMORI K et al.** Situation on dialysis dermal pruritus - Search report on 2474 patients at 41 institutions in the Niigata prefecture. *Journal of the Japanese Society for Dialysis Therapy,* 2001, vol. 34, 1469-77
- **NARITA I et al.** Etiology and prognostic significance of severe uremic pruritus in chronic hemodialysis patients. *Kidney Int.,* 2006, vol. 69, 1626-32
- **PISONI RL et al.** Pruritus in haemodialysis patients: International results from the Dialysis Outcomes and Practice Patterns Study (DOPPS). *Nephrol Dial Transplant.,* 2006, vol. 21, 3495-505
- **KIMATA N et al.** Pruritus in hemodialysis patients: Results from the Japanese Dialysis Outcomes and Practice Patterns Study (JDOPPS). *Hemodial Int,* 2014, vol. 18, 657-67
- **GANGEMI S ; QUARTUCCIO S ; CASCIARO M ; TRAPANI G ; MINCIULLO PL ; IMBALZANO E.** Interleukin 31 and skin diseases: A systematic review. *Allergy Asthma Proc.,* 2017, vol. 38 (6), 401-8
- **KO MJ et al.** Interleukin-31 is associated with uremic pruritus in patients receiving hemodialysis. *J Am Acad Dermatol.,* 2014, vol. 71, 1151-9
- *Nat Immunol,* 2004, vol. 5, 752-760
- *Nat Immunol,* 2004, vol. 5, 752-60

- **DILLON et al.** *Nat Immunol,* 2004, vol. 5, 752-760
- *Curr Opin Biotechnol,* 2006, vol. 17, 653-658
- *Curr Opin Struct Biol,* 1997, vol. 7, 463-469
- *Protein Sci,* 2006, vol. 15, 14-27
- *Nature,* 1975, vol. 256, 495
- *Nature,* 1991, vol. 352, 624-628
- *J Mol Biol,* 1991, vol. 222, 581-597
- **KABAT et al.** Sequences of Proteins of Immunological Interest. 1991
- **CHOTHIA et al.** *Science,* 1986, vol. 233, 755-758
- *J Mol Biol,* 1996, vol. 262, 732-745
- *J Biol Chem,* 1995, vol. 270, 21619-21625
- *J Biol Chem,* 2006, vol. 281, 20464-20473
- Using Antibodies: A Laboratory Manual. Cold Spring Harbor Laboratory Press, 1999
- **SHIGEKI YAMADA et al.** Investigation of the status of uremic pruritus in hemodialysis patients and the efficacy of nalfurafine hydrochloride - Questionnaire administered to 1,936 patients from 17 institutions in Tokai area of Japan. *Journal of the Japanese Society for Dialysis Therapy,* 2012, vol. 45, 1133-40
- **JENNIFER Q. DONG et al.** Quantitative Prediction of Human Pharmacokinetics for Monoclonal Antibodies. *Clin Pharmacokinet,* 2011, vol. 50 (2), 131-142
- **JIE LING et al.** Interspecies Scaling of Therapeutic Monoclonal Antibodies: Initial Look. *J Clin Pharmacol,* 2009, vol. 49 (12), 1382-1402
- **RONG DENG et al.** Projecting human pharmacokinetics of therapeutic antibodies from nonclinical data. *mAbs,* 2011, vol. 3 (1), 61-66

- **WAHLGREN CF. et al.** *J Allergy Clin Immunol,* 2006, vol. 118, 178-89

- *Guidelines for Treatment of Atopic Dermatitis,* 2008
- *Protein Science,* 1995, vol. 4, 2411-2423